# EUROPEAN PATENT APPLICATION

(11) **EP 4 445 860 A1**
(43) Date of publication of application: **16.10.2024**
(21) Application number: 22903194.3
(22) Date of filing: 23.11.2022
(51) Int. Cl.: A61B 90/00, A61B 17/34

(54) **MEDICAL OPTICAL TRACING SYSTEM**

(30) Priority: 10.12.2021 CN 202111506551
(71) Applicant: Zhou, Xing, Guangzhou, Guangdong 510663 (CN)
(72) Inventor: Zhou, Xing, Guangzhou, Guangdong 510663 (CN)
(74) Representative: Boult Wade Tennant LLP
(86) International application number: PCT/CN2022/133680
(87) International publication number: WO 2023/103771

(57) **Abstract**

A medical optical tracking system of the present invention includes a light source and an optical tracking carrier. The optical tracking carrier includes a light guide material, for example, a light-emitting optical fiber. Light emitted by the light source is conducted through the optical tracking carrier, and optical tracking is performed on the optical tracking carrier. The light source is a medical cold light source, or may be an LED light source. Particularly, light-emitting ends of a micro LED light source and the optical tracking carrier may be directly integrated and placed in vivo to perform optical tracking. The medical optical tracking system of the present invention may be manufactured as a visible-light tracking medical catheter, or a visible-light tracking solid tumor tracking apparatus, or a visible-light tracking optical fiber, or a visible-light tracking blood vessel tracking apparatus as required, and is configured to track a blood vessel, an esophagus, a ureter, a urethra, or another cavity or tube, or track and calibrate the position of a solid tumor such as a uterine fibroid, a lung tumor, a liver tumor, or the like in clinical surgery, to improve the precision of surgery, thereby avoiding accidents during surgery.

## Description

### FIELD OF THE INVENTION

The present invention relates to a medical tracking system, and in particular to a medical optical tracking system.

### BACKGROUND OF THE INVENTION

In clinical surgery, because a blood vessel, a ureter, an oviduct, a trachea, or another cavity or tube is usually covered in tissue, a surgeon needs to receive long-term training and be familiar with anatomical structures before the surgeon can accurately separate a blood vessel, a ureter, an oviduct, a trachea, or another cavity or tube. Even a highly experienced surgeon sometimes accidentally injures a blood vessel, a ureter, an oviduct, a trachea, or another cavity or tube. Therefore, there is a need for an apparatus that can accurately identify a blood vessel, a ureter, an oviduct, a trachea, or another cavity or tube in tissue.

In addition, with the significant increase in the resolution of medical imaging equipment, some tumor tissue such as a pulmonary nodule, a uterine fibroid, an esophagus tumor, a liver tumor, or the like with a very small volume can be discovered. Because such tumor tissue such as a pulmonary nodule has a small volume, it is very difficult to accurately discern the tumor tissue during excision with an endoscope. Therefore, tumor tissue to be excised needs to be identified, to facilitate surgical excision with endoscope.

In addition, lymphadenectomy is usually performed close to blood vessels, and therefore the positions and ranges of blood vessels need to be calibrated, to facilitate surgery with an endoscope.

In summary, visible light identification still cannot be performed on a blood vessel, a ureter, an oviduct, a vas deferens, a trachea, or the like concealed in tissue in existing technologies, resulting in inconvenience in recognition in surgery with an endoscope. Therefore, it is necessary to develop a visible light identification technique and apparatus with an endoscope, making it convenient to recognize a blood vessel, a ureter, an oviduct, a vas deferens, a trachea, or another cavity or tube and a solid tumor with an endoscope.

The present invention discloses a visible light technique-based identification technique for a blood vessel, a ureter, an oviduct, a vas deferens, a trachea, or another cavity or tube and a calibration technique and apparatus for a solid tumor.

### SUMMARY

An objective of the present invention is to solve the problem that a blood vessel, tissue, an organ or a small-size tumor cannot be accurately identified with an endoscope in existing clinical surgery, and through the setting of an optical tracking manner, in clinical surgery, light sources of different colors are disposed to accurately discern a blood vessel, tissue or an organ that requires special protection in clinical surgery, to effectively avoid an accident during surgery, or effectively calibrate a small size tumor, thereby facilitating effective implementation of clinical surgery.

The present invention provides a medical optical tracking system, where the medical optical tracking system 500 includes a light source 1 and an optical tracking carrier 2;
A. the optical tracking carrier 2 includes a light guide material; and
B. light emitted by the light source 1 is conducted through the optical tracking carrier 2, and optical tracking is performed on the optical tracking carrier 2.

A direct contact manner may be used for the light source 1 and the optical tracking carrier 2, and the light emitted by the light source 1 is directly conducted through the light guide material of the optical tracking carrier 2 to perform tracking. A non-contact manner may be used for the light source 1, and the light emitted by the light source 1 is first stored by an energy carrier in the optical tracking carrier 2, for example, a light-retaining self-luminous body 21-1, and then light is emitted through energy conversion to perform tracking. A light-emitting end 11-1 of the light source 1 and the optical tracking carrier 2 may be packaged into a whole, which is directly disposed at a position that requires tracking.

The light source 1 is an LED light source 11, and/or a medical cold light source 12, and/or natural light. The light source 1 may be one of various types of light sources that can emit light, and the light emitted by the light source 1 may be conducted through the optical tracking carrier 2 to perform tracking. Compared with a conventional lighting source, the LED light source 11 has characteristics such as a small volume, high light emission efficiency, and strong light source directivity, and especially in terms of safety, the LED light source has an unrivalled advantage over a conventional light source. First, the LED light source uses low-voltage direct-current power supply, and a power supply voltage only needs to be 6 V to 24 V. Next, no mercury is added to the LED light source, and a human body is kept from poisoning or other damage. Furthermore, more importantly, the LED light source is a cold light source, and no severe heat generation occurs in a working process, which allows safe touch, and causes no accidental high-temperature scalding to a human body. The medical cold light source 12 is a common light source during existing surgery. In addition, the light source 1 may be placed in the back and is readily available in a surgery, and no additional equipment is required.

A color of the light emitted by the light source 1 may be set according to a background color or a transmission requirement. Through the setting of light, in clinical surgery, a doctor can directly see the position of the optical tracking carrier 2 through tissue with naked eyes to accurately discern a blood vessel, tissue or an organ that requires special protection in clinical surgery, to effectively avoid an accident during surgery. The light emitted by the light source 1 may be differently set according to a background color in a body cavity or tissue through which light needs to be transmitted. When light needs to be transmitted through tissue, red light and yellow light are preferred, and purple light and white light are less preferred, and when blood vessel tissue needs to be displayed, green light is preferred.

The light source 1 emits light in a flashing manner. The light source 1 may be set as required to a manner of being intermittently lit, a flashing manner, or another form.

An intensity of the light emitted by the light source 1 may be set. The intensity of the light emitted by the light source 1 may be adjusted as required, to adapt to different clinical environments. The illuminance of the light emitted by the light source 1 usually ranges from 5,000 lux to 150,000 lux.

The light source 1 includes a control system 13. The control system 13 includes a wavelength adjustment mechanism 13-1 and a light intensity adjustment mechanism 13-2. The wavelength adjustment mechanism 13-1 can adjust a wavelength to adjust the color of the emitted light, and the light intensity adjustment mechanism 13-2 can adjust the illuminance of the emitted light.

The LED light source 11 is disposed in vivo and/or in vitro. The volume of the light-emitting end 11-1 of the LED light source 11 may be very small. Therefore, the LED light source 11 can be disposed in vitro to conduct light into a human body through the optical tracking carrier 2, and can be directly disposed in a human body, wrapped with the optical tracking carrier 2 outside, and directly disposed at a position that requires tracking.

The light source 1 and the optical tracking carrier 2 are in non-contact connection or contact connection. The light source 1 may be connected to the optical tracking carrier 2 by a light guide joint 26 to provide a light source, or may illuminate the optical tracking carrier 2 in a non-contact manner. For example, for a light-retaining self-luminous tracking carrier 21, the tracking of the optical tracking carrier 2 is implemented through the storage and conversion of light energy.

The LED light source 11 and the optical tracking carrier 2 are in contact connection, and the LED light source 11 is disposed in the optical tracking carrier 2. The optical tracking carrier 2 is directly covered outside the LED light source 11, and the LED light source 11 and the optical tracking carrier 2 are placed together in a human body, to track a cavity or tube, an organ, a tumor, or the like.

The optical tracking carrier 2 is a light-retaining self-luminous tracking carrier 21. A self-luminous material is a substance that can absorb energy in a manner and convert the energy into nonequilibrium optical radiation, and a process in which the energy absorbed inside the material is converted into nonequilibrium optical radiation is a light emission process. Especially, a light-retaining self-luminous material can continuously emit light for over 12 hours in a dark environment after being irradiated by external light for several minutes or tens of minutes, and can meet tracking requirements of most surgical durations. The light-retaining self-luminous tracking carrier 21 may directly absorb energy of light in a surgery, so that a variety of external light can form the light source 1, and the light source 1 does not need to be directly connected, so that a use process is very simple.

The light-retaining self-luminous tracking carrier 21 includes a light-retaining self-luminous body 21-1 and a protective carrier 21-2.

The protective carrier 21-2 is made of a transparent light guide material, and the light-retaining self-luminous body 21-1 is sealed in the protective carrier 21-2.

The light-retaining self-luminous body 21-1 can absorb external energy, and perform conversion to emit light. The protective carrier 21-2 is made of a transparent medical material, and may directly contact tissue, so that while light emitted through energy conversion of the light-retaining self-luminous body 21-1 can be effectively transmitted to perform effective tracking, biosafety in clinical use is ensured. The light-retaining self-luminous body 21-1 can be disposed at different positions and designed into different shapes, and perform targeted tracking or perform overall tracking as required.

The optical tracking carrier 2 is a light guide optical fiber 22. The light guide optical fiber 22 has a good light guide effect, conducts light to various different positions as required, and may be connected or disconnected as required, so that clinical use is very convenient.

The optical tracking carrier 2 includes at least one light guide optical fiber 22.

Both an end portion and/or a side surface of the light guide optical fiber 22 can emit light. The end portion of the light guide optical fiber 22 can emit light, and the side surface can also emit light. The light guide optical fiber 22 can be completely lit to emit light.

The optical tracking carrier 2 is a combination of a plurality of light guide optical fibers 22.

The optical tracking carrier 2 may be formed by a single light guide optical fiber 22, or may be a combination of a plurality of light guide optical fibers 22, for example, in one of various manners of forming an optical fiber bundle, being plaited in a mesh form, being arranged at different lengths, and the like.

The light guide optical fiber 22 has a smooth surface. When the light guide optical fiber 22 has a smooth surface, because the light guide optical fiber 22 has good light guide performance, light outlets 22-2 of the light guide optical fiber 22 are located at a distal end of the light guide optical fiber 22, so that targeted tracking can be implemented.

The light guide optical fiber 22 has a non-smooth surface 22-1.

The non-smooth surface 22-1 is a non-smooth surface 22-11 that can form reflection and/or scattering. Through the reflection and/or scattering of light by the non-smooth surface 22-1, the overall light emission of the non-smooth surface 22-1 can be implemented, to achieve the effect of overall tracking.

The light outlets 22-2 are intermittently provided in the light guide optical fiber 22. Each of the intermittently provided light outlets 22-2 has a conduction surface 22-21 and a reflection surface 22-22 of light. When light is conducted through the conduction surface 22-21 to reach the reflection surface 22-22, the light is reflected and emitted out of the light outlet 22-2 to form one tracking point, and a plurality of light outlets 22-2 may form a chain tracking belt.

The light outlets 22-2 are non-axial light outlets 22-21, provided in a side surface of the light guide optical fiber 22 in a length direction of the light guide optical fiber 22. When the light outlets 22-2 are completely provided in the length direction of the light guide optical fiber 22, the light guide optical fiber 22 may be completely lit in the length direction of the light guide optical fiber 22, to implement the overall tracking of the light guide optical fiber 22.

The light outlets 22-2 may identify a length of the light guide optical fiber 22 through regular arrangement of the light outlets 22-2.

Intensities of scattered light are different through regular arrangement of the light outlets 22-2 in distribution density to identify the length of the light guide optical fiber 22.

When the light outlets 22-2 are densely arranged, emitted light is stronger, and the visual effect is brighter. When the light outlets 22-2 are dispersedly arranged, emitted light is weaker, and the visual effect is relatively dark. Through the arrangement of the bright and dark effects, a visual effect similar to a ruler may be formed, so that while tracking is performed, size identification can also be implemented.

The light guide optical fiber 22 is plaited in a mesh form, and the light outlets 22-2 are scatteredly distributed at different positions. The light guide optical fiber 22 is plaited in a mesh form. The length of each light guide optical fiber 22 may be set to be different, and the light outlets 22-2 of the light guide optical fiber 22 are different accordingly, and are scatteredly distributed, so that overall tracking in a three-dimensional space can be implemented. Because the light outlets 22-2 do not need to be provided in the middle of each light guide optical fiber 22, light conduction is better, and the visual effect of a single tracking point is very bright. The light outlets 22-2 may also be provided in the side surface, and the light guide optical fiber 22 that can be completely lit is plaited in a mesh form, to implement full-area tracking of an entire cavity. In addition, the shape of plaiting in a mesh form may have good support, and is especially suitable for support and tracking of a large cavity such as a bladder, a uterus, or the like.

During clinical application, the optical tracking carrier 2 may be placed as required at a variety of positions that require tracking, for example, a ureter, a vas deferens, an oviduct, or another cavity or tube, or may be placed in a uterine fibroid, a lung tumor (especially a pulmonary nodule), a liver tumor, or another solid tumor, or may be placed in a blood vessel, and especially a cavity or tube, a blood vessel, a solid tumor, or the like is identified by using the characteristic that the side surface of the light guide optical fiber 22 can emit light to implement overall tracking.

A surface of the medical optical tracking system 500 includes a coating 3.

The coating 3 is an anticoagulant coating, and/or a hydrophilic coating, and/or a hydrophobic coating.

The coating 3 may be designed to be a coating having a different property as required. For example, when the light guide optical fiber 22 needs to enter a blood vessel, the coating 3 may be designed into an anticoagulant coating. When the light guide optical fiber 22 needs to enter a variety of cavities, the coating 3 may be designed into a hydrophilic coating or a hydrophobic coating as required.

The optical tracking carrier 2 includes a delivery portion 23. The delivery portion 23 can deliver a working portion 2-1 of the optical tracking carrier 2 as required to a position that requires tracking, for example, a blood vessel, a cavity, or a tumor surgery position.

The delivery portion 23 is movably disposed on the optical tracking carrier 2. During clinical application, the delivery portion 23 may be removed from the optical tracking carrier 2 or move relative to the optical tracking carrier 2 as required.

The medical optical tracking system 500 further includes a development mechanism 4.

The development mechanism 4 is made of metal, and has a heat conduction function. The heat conduction function of the development mechanism 4 can prevent an accident caused by a part of the medical optical tracking system 500 entering a human body having an excessively high temperature, and the temperature is usually controlled below 37°C.

The development mechanism 4 is a development string 41, and/or a development ring 42, and/or a development block 43. The applicant only illustrates the foregoing several development manners. During actual application, a person skilled in the art may design different development manners as required. The development manners are not illustrated herein one by one by the applicant, but none of the development manners departs from the protection scope of this application.

The development mechanism 4 performs development under X-rays, and/or in MRI, and/or in B-mode ultrasound. The development mechanism 4 can perform a development prompt in X-rays, magnetic navigation, B-mode ultrasound, or another scenario, and the development mechanism 4 facilitates the placement of the optical tracking carrier 2 in a visible or navigation case, and is especially suitable for placement in an important blood vessel, a solid tumor, or the like.

The medical optical tracking system 500 further includes a protection sleeve 5. The optical tracking carrier 2, the development mechanism 4, and the like may be disposed in the protection sleeve 5. The coating 3 may be disposed outside the protection sleeve 5 as required.

The protection sleeve 5 is made of a transparent material, and the optical tracking carrier 2 is disposed in the protection sleeve 5. The protection sleeve 5 is made of a medical transparent material, so that while the optical tracking carrier 2 is protected, the transparent material can still ensure the tracking of the optical tracking carrier 2.

The optical tracking carrier 2 includes a channel 24 inside. The channel 24 may be used as a surgical instrument channel, a body fluid drainage channel, or the like as required.

The optical tracking carrier 2 is made of a flexible medical material, and is movable along a blood vessel, a ureter, an esophagus, a trachea, an oviduct, a vas deferens, or another cavity. The optical tracking carrier 2 has good flexibility, and is movable along a blood vessel or a cavity. Therefore, the optical tracking carrier 2 may be placed at different positions as required.

The optical tracking carrier 2 includes a shaping mechanism 25. The shaping mechanism 25 may shape the optical tracking carrier 2, for example, shape the optical tracking carrier into one of a variety of different shapes such as a circular shape, an arc shape, a spherical shape, and the like as required, to adapt to different tracking requirements.

The shaping mechanism 25 is a shape-memory shaping mechanism 25-1. The shape-memory shaping mechanism 25-1 has a string shape, a strip shape, or another simple structure at room temperature, so that after being placed in a human body, the shape-memory shaping mechanism restores the set shape under the action of body temperature.

The shape-memory shaping mechanism 25-1 is manufactured by plaiting a shape-memory metal wire, and/or is manufactured by carving a shape-memory metal tube or sheet. The shape-memory shaping mechanism 25-1 may be formed by plaiting a shape-memory metal wire into a required shape, or may be formed by directly carving a shape-memory alloy tube or a shape-memory alloy sheet into a required shape.

The present invention provides a medical catheter with a tracking apparatus, including the medical optical tracking system 500.

The medical catheter 900 with a tracking apparatus includes a catheter 900-1, an interface 900-2, and the medical optical tracking system 500;
A. the catheter 900-1 is made of a soft elastic medical material, and a working opening 900-11-1 is provided at a distal end 900-11;
B. the medical catheter 900 with a tracking apparatus includes at least one interface 900-2, and the interface 900-2 is disposed at a proximal end 900-12 of the catheter 900-1; and
C. the optical tracking carrier 2 of the medical optical tracking system 500, disposed on the catheter 900-1, and identifying a position of the catheter 900-1.

The medical catheter 900 with a tracking apparatus includes a development mechanism 4. The development mechanism 4 is disposed on the catheter 900-1. The development mechanism 4 can perform a development prompt in X-rays, magnetic navigation, B-mode ultrasound, or another scenario, to facilitate the placement of the catheter 900-1. The development mechanism 4 may be made of a metal material, and may also have a heat conduction function. To keep the LED light source 11 from causing an excessively high temperature in a body, the temperature usually needs to be controlled within 37°C.

The optical tracking carrier 2 is provided on the catheter 900-1, and the optical tracking carrier 2 may perform local or overall tracking on the catheter 900-1 as required.

The catheter 900-1 is made of a transparent material, and forms the optical tracking carrier 2, the light source of the medical optical tracking system 500 is an LED light source 11, and light-emitting ends 11-1 of the LED light source 11 are disposed in a tube wall of the catheter 900-1, and track the catheter 900-1. The LED light source 11 includes the light-emitting ends 11-1 each having a very small volume, and the size of the light-emitting end 11-1 is usually controlled below 2 mm. For example, an LED lamp with a size of 0.2 mm to 0.5 mm is packaged. The light-emitting ends 11-1 may be directly disposed in the tube wall of the catheter 900-1, is connected to a power supply by a circuit system 11-2, and emits light in the tube wall of the catheter 900-1.

The light-emitting ends 11-1 of the LED light source 11 are disposed at the distal end 900-11 of the catheter 900-1, and track the distal end 900-11 of the catheter 900-1.

The light-emitting ends 11-1 of the LED light source 11 are dispersedly disposed in the tube wall of the catheter 900-1, and form an LED lamp strip or an LED lamp net, to perform overall tracking on the catheter 900-1.

The light-emitting ends 11-1 may be disposed at any positions of the catheter 900-1 as required. When the light-emitting ends are disposed at the distal end, the distal end 900-11 of the catheter 900-1 is tracked. When the light-emitting ends are disposed in the middle of the catheter 900-1, the middle of the catheter 900-1 can be locally tracked. When a flexible circuit board 11-21 is used in the circuit system 11-2, the light-emitting ends 11-1 may be dispersedly disposed at any positions of the flexible circuit board 11-21, to implement partial or overall tracking of the catheter 900-1.

The optical tracking carrier 2 of the medical optical tracking system 500 is a light guide optical fiber 22; and a proximal end of the light guide optical fiber 22 is connected to a light guide joint 26, and the light guide optical fiber 22 is disposed in a length direction of the catheter 900-1, to track the catheter 900-1.

The light guide optical fiber 22 has a smooth surface, and light outlets 22-2 are provided at a distal end of the catheter 1, to track the distal end 900-11 of the catheter 900-1.

The light guide optical fiber 22 has a non-smooth surface 22-1, and light outlets 22-2 are provided in a side surface, to perform overall tracking on the catheter 900-1.

The light guide optical fiber 22 can perform targeted tracking on the distal end 900-11 of the catheter 900-1, or string-shaped tracking can be performed on the catheter 900-1 in the length direction through the light outlets 22-2 in the side surface, or the light guide optical fiber 22 can be plaited in a mesh form to perform overall tracking on the catheter 900-1, and through the plaiting in a mesh form, the elastic support force of the light guide optical fiber 22 can be used to implement tube wall support for the catheter 900-1.

A coating 3, for example, a hydrophilic coating, may be disposed on an outer surface of the catheter 900-1 as required.

The medical catheter 900 with a tracking apparatus is a medical stomach tube 901, or a medical urinary catheter 902, or a medical vas deferens, or a medical oviduct, or a medical trachea. The applicant only lists the foregoing several use scenarios of the medical catheter 900 with a tracking apparatus herein. During actual application, the medical catheter 900 with a tracking apparatus may be disposed according to clinical requirements into structures required for different application scenarios.

The medical catheter 900 with a tracking apparatus is the medical stomach tube 901; the medical stomach tube 901 includes the catheter 900-1, one interface 900-2, and the medical optical tracking system 500, and the distal end 900-11 of the catheter 900-1 is provided with the working opening 900-11-1; and the medical optical tracking system 500 tracks the catheter 900-1.

The medical stomach tube 901 enters the stomach through an esophagus, and is an artificial conveying channel of food. The interface 900-2 may be externally connected to a food injection apparatus. In clinical surgery, it may be only necessary to know the position of the distal end 900-11 of the catheter 900-1 for observation. In this case, the medical optical tracking system 500 only needs to perform local tracking on the distal end of the medical stomach tube 901. In some other cases, for example, surgery along an esophagus, to keep the esophagus from accidental injury, in this case, overall tracking needs to be performed on the medical stomach tube 901.

When distal-end local tracking needs to be performed on the medical stomach tube 901, a technical solution in which the light-emitting ends 11-1 of the LED light source 11 are individually or centrally disposed at the distal end of the catheter 900-1 to perform tracking may be used, or the light guide optical fiber 22 having a smooth surface may be used. A single light guide optical fiber 22 or a light guide bundle formed by a plurality of light guide optical fibers 22 may be used, and the light outlets 22-2 at an end portion are used to track the distal end of the catheter 900-1, or the light guide optical fiber 22 is circumferentially arranged along a side wall of the catheter 900-1 to form a light ring at the distal end of the catheter 900-1 to perform local tracking on the distal end of the catheter 900-1.

When overall or large-range tracking needs to be performed on the medical stomach tube 901, the light-emitting ends 11-1 of the LED light source 11 may be dispersedly distributed in the tube wall of the catheter 900-1 to form a light guide belt or a light guide net to perform overall or large-range tracking on the catheter 900-1. Alternatively, the light guide optical fiber 22 having the non-smooth surface 22-1 may be disposed in the tube wall of the catheter 900-1 in the length direction to form a light guide belt to track the catheter 900-1 in the length direction, or the light guide optical fiber 22 may be plaited in a mesh form, to perform a ring-shaped overall tracking on a section or a whole of the catheter 900-1.

The medical catheter 900 with a tracking apparatus is the medical urinary catheter 902; the medical urinary catheter 902 includes the catheter 900-1, the interface 900-2, and the medical optical tracking system 500, and the distal end 900-11 of the catheter 900-1 is provided with the working opening 900-11-1; and the medical optical tracking system 500 tracks the catheter 900-1.

The medical optical tracking system 500 may perform targeted tracking on the medical urinary catheter 902, or may perform large-range or overall tracking on the medical urinary catheter 902.

When targeted or small-range local tracking needs to be performed on a working end of the medical urinary catheter 902, a technical solution in which the light-emitting ends 11-1 of the LED light source 11 are individually or centrally disposed at the distal end of the catheter 900-1 to perform tracking may be used, or the light guide optical fiber 22 having a smooth surface may be used. A single light guide optical fiber 22 or a light guide bundle formed by a plurality of light guide optical fibers 22 may be used, and the light outlets 22-2 at an end portion are used to track the distal end of the catheter 900-1, or the light guide optical fiber 22 is circumferentially arranged along a side wall of the catheter 900-1 to form a light ring at the distal end of the catheter 900-1 to perform local tracking on the distal end of the catheter 900-1.

For example, when regional tracking needs to be performed on a bladder or overall tracking needs to be performed on the catheter 900-1, the light-emitting ends 11-1 of the LED light source 11 may be dispersedly distributed in the tube wall of the catheter 900-1 to form a light guide belt or a light guide net to perform overall or large-range tracking on the catheter 900-1. Alternatively, the light guide optical fiber 22 having the non-smooth surface 22-1 may be disposed in the tube wall of the catheter 900-1 in the length direction to form a light guide belt to track the catheter 900-1 in the length direction, or the light guide optical fiber 22 may be plaited in a mesh form, to perform overall tracking on a periphery of the balloon 900-13.

The medical urinary catheter 902 is a double-lumen urinary catheter 902-1; the distal end 900-11 of the catheter 900-1 of the double-lumen urinary catheter 902-1 is provided with a balloon 900-13; the catheter 900-1 is provided with two cavity channels 1-1, and each cavity channel 1-1 is correspondingly provided with one interface 900-2; one cavity channel 1-1 forms a urinary lumen 900-14, a distal end of the urinary lumen 900-14 is provided with the working opening 900-11-1, and the interface 900-2 provided at the proximal end forms a fluid drainage port 900-21; and the other cavity channel 1-1 forms a water injection lumen 900-15, a distal end of the water injection lumen 900-15 is connected to the balloon 900-13, and the interface 900-2 connected to the proximal end forms a water injection port 900-22.

To ensure a time requirement of clinical surgery, the balloon 900-13 usually needs to be disposed in the medical urinary catheter 902 used in surgery, and water is injected into the balloon 900-13 from the water injection port 900-22 through the water injection lumen 900-15, so that a water balloon may be formed, to ensure effective fastening of the medical urinary catheter 902 in the bladder, thereby preventing slippage.

The medical urinary catheter 902 is a triple-cavity urinary catheter 902-2; the distal end 900-11 of the catheter 900-1 of the triple-cavity urinary catheter 902-2 is provided with a balloon 900-13; the catheter 900-1 is provided with three cavity channels 1-1, and each cavity channel 1-1 is correspondingly provided with one interface 900-2; the first cavity channel 1-1 forms a urinary lumen 900-14, a distal end of the urinary lumen 900-14 is provided with the working opening 900-11-1, and the interface 900-2 provided at the proximal end forms a fluid drainage port 900-21; the second cavity channel 1-1 forms a water injection lumen 900-15, a distal end of the water injection lumen 900-15 is connected to the balloon 900-13, and the interface 900-2 connected to the proximal end forms a water injection port 900-22; and the third cavity channel 1-1 forms an infusion flushing lumen 900-16, a distal end of the infusion flushing lumen 900-16 is provided with a flushing port 900-16-1 and a stop valve 900-16-2, and the interface 900-2 connected to a proximal end of the infusion flushing lumen 900-16 forms a flushing drug injection port 900-23.

To facilitate flushing, especially drug flushing and disinfection, of the medical urinary catheter 902 during indwelling, the medical urinary catheter 902 may be disposed as the triple-cavity urinary catheter 902-2. The flushing drug injection port 900-23 is provided at the proximal end of the infusion flushing lumen 900-16. The flushing port 900-16-1 at the distal end is in communication with a body cavity. The stop valve 900-16-2 is provided at the flushing port 900-16-1. When drug is injected through the flushing drug injection port 900-23, under the action of a pressure, the stop valve 900-16-2 is opened, and the drug enters the body cavity. When the injection of the drug is stopped, under the action of a pressure in the body cavity, the stop valve 900-16-2 is automatically closed, to prevent body fluid from entering the infusion flushing lumen 900-16 through the flushing port 900-16-1.

The present invention provides a solid tumor tracking apparatus, including the medical optical tracking system 500.

The solid tumor tracking apparatus 910 includes a development mechanism 4. For the development mechanism 4, an anti-displacement mechanism 910-1 made of a metal material may form the development mechanism 4, or the development mechanism 4 may be separately disposed. The development mechanism 4 can identify the position of the optical tracking carrier 2 under X-rays, or in MRI, or in a B-mode ultrasound state.

The solid tumor tracking apparatus 910 includes an anti-displacement mechanism 910-1 and the medical optical tracking system 500; and the optical tracking carrier 2 of the medical optical tracking system 500 is disposed on the anti-displacement mechanism 910-1, to track the anti-displacement mechanism 910-1.

The anti-displacement mechanism 910-1 may be fastened at a solid tumor that needs to be calibrate d, to keep the solid tumor tracking apparatus 910 from displacing along with a movement of a human body, for example, respiratory movement of the lung, intestinal peristalsis, gastric peristalsis, or the like.

During clinical application, under X-rays or in an MRI state, a medical puncture needle is punctured to a solid tumor that needs to be identified, and then the anti-displacement mechanism 910-1 is delivered to the position of the solid tumor, and is fastened on the solid tumor. The optical tracking carrier 2 is disposed on the anti-displacement mechanism 910-1 to position the anti-displacement mechanism 910-1, and in surgery, the position of the solid tumor can be intuitively observed with naked eyes under the guidance of light.

The anti-displacement mechanism 910-1 has a hook-shaped structure, and/or a horn-shaped structure, and/or a dumbbell structure, and/or a spiral spring structure, and/or a pin-shaped structure. The applicant only illustrates the manners of the anti-displacement mechanisms 910-1 with the foregoing several structures. During actual application, a person skilled in the art may design the anti-displacement mechanisms 910-1 with different structures as required, which are not illustrated herein one by one by the applicant, but none of the anti-displacement mechanisms departs from the protection scope of this application.

The anti-displacement mechanism 910-1 has a hook-shaped structure, and the anti-displacement mechanism 910-1 includes at least one positioning hook 910-11. Generally, the anti-displacement mechanism 910-1 includes two or three positioning hooks 910-11, to better prevent the anti-displacement mechanism 910-1 from displacing along with a movement of a human body, for example, respiratory movement of the lung, intestinal peristalsis, or the like.

The optical tracking carrier 2 is a light guide optical fiber 22, a proximal end of the light guide optical fiber 22 is connected to a light guide joint 26, a distal end of the light guide optical fiber 22 is connected and fastened to the anti-displacement mechanism 910-1, and light outlets 22-2 of the light guide optical fiber 22 are provided in the anti-displacement mechanism 910-1, to track the anti-displacement mechanism 910-1.

The light guide optical fiber 22 has flexibility and therefore can be directly wound, connected, and fastened at the anti-displacement mechanism 910-1, and especially on the positioning hook 910-11, and optical tracking is performed on the anti-displacement mechanism 910-1 by using light emitted by the light outlets 22-2 of the light guide optical fiber 22.

The light guide optical fiber 22 has a non-smooth surface 22-1, the light outlets 22-2 are provided in a side surface of the light guide optical fiber 22, and the light guide optical fiber 22 performs overall tracking on the anti-displacement mechanism 910-1.

The light guide optical fiber 22 is made of a flexible medical material, and is connected and fastened to the anti-displacement mechanism 910-1, and when the anti-displacement mechanism 910-1 deforms, the light guide optical fiber 22 deforms along with the anti-displacement mechanism 910-1. The light guide optical fiber 22 can be completely lit, and therefore, after being wound on the anti-displacement mechanism 910-1, especially on the positioning hook 910-11, the light guide optical fiber 22 may deform along with the anti-displacement mechanism 910-1. Especially, when the anti-displacement mechanism 910-1 is made of a shape-memory alloy, as the shape of the anti-displacement mechanism 910-1 changes, the shape of the light guide optical fiber 22 changes accordingly, to ensure the tracking effect of the anti-displacement mechanism 910-1.

The solid tumor tracking apparatus 910 includes an anti-displacement mechanism 910-1, a delivery mechanism 910-2, and the medical optical tracking system 500; and the optical tracking carrier 2 of the medical optical tracking system 500 is disposed on the anti-displacement mechanism 910-1, to track the anti-displacement mechanism 910-1.

The delivery mechanism 910-2 includes a delivery sheath 910-21 and a pushing mechanism 910-22, the anti-displacement mechanism 910-1 is disposed in the delivery sheath 910-21, and the pushing mechanism 910-22 can push the anti-displacement mechanism 910-1 out of the delivery sheath 910-21.

The pushing mechanism 910-22 may be fastened to the anti-displacement mechanism 910-1, and after being pushed out of the delivery sheath 910-21, the anti-displacement mechanism 910-1 remains in vivo together with the delivery sheath. The pushing mechanism 910-22 may be detachably connected to the anti-displacement mechanism 910-1, and after being pushed out of the delivery sheath 910-21, the anti-displacement mechanism 910-1 is disconnected from the anti-displacement mechanism 910-1, and only the anti-displacement mechanism 910-1 is indwelt at a solid tumor.

During clinical application, under X-rays or in an MRI state, the delivery mechanism 910-2 delivers the anti-displacement mechanism 910-1 to the position of the solid tumor, and is fastened on the solid tumor. The optical tracking carrier 2 is disposed on the anti-displacement mechanism 910-1 to position the anti-displacement mechanism 910-1, and in surgery, the position of the solid tumor can be intuitively observed with naked eyes under the guidance of light.

The light source 1 of the medical optical tracking system 500 is an LED light source 11, the optical tracking carrier 2 is made of a transparent material, and light-emitting ends 11-1 of the LED light source 11 are disposed on the optical tracking carrier 2, and are disposed together with the optical tracking carrier 2 on the anti-displacement mechanism 910-1, to track the anti-displacement mechanism 910-1.

When the light source 1 is the LED light source 11, the light-emitting ends 11-1 of the LED light source 11 and the optical tracking carrier 2 may be packaged into a whole, and are disposed together on the anti-displacement mechanism 910-1 at a position that requires tracking. Light emitted by the light-emitting ends 11-1 is conducted by a light guide material of the optical tracking carrier 2 and then displayed for clinically discerning the position of a solid tumor.

The LED light source 11 includes the light-emitting ends 11-1, a circuit system 11-2, a drive board 11-3, and a power supply 11-4; the light-emitting ends 11-1 are connected to the drive board 11-3 and the power supply 11-4 by the circuit system 11-2, and under the control of the drive board 11-3, the power supply 11-4 supplies power to the light-emitting ends 11-1 through the circuit system 11-2, and the light-emitting ends 11-1 emit light; and the drive board 11-3 and the power supply 11-4 are disposed in vitro, and the light-emitting ends 11-1 are disposed in vivo, to track the anti-displacement mechanism 910-1.

The circuit system 11-2 is a flexible circuit board 11-21, and the light-emitting ends 11-1 of the LED light source 11 are dispersedly disposed on the flexible circuit board 11-21 and packaged in the optical tracking carrier 2, to form an LED lamp strip, or an LED lamp net, or an LED lamp bulb, disposed on the anti-displacement mechanism 910-1, to track the anti-displacement mechanism 910-1.

The LED light source 11 may be formed by packaging a single light-emitting end 11-1 to perform targeted tracking. Alternatively, the flexible circuit board 11-21 may be used for the circuit system 11-2, the light-emitting ends 11-1 are dispersedly disposed at any positions of the flexible circuit board 11-21 to form a light-emitting belt, and the light-emitting belt is wound on the anti-displacement mechanism 910-1, or a plurality of LED light sources 11 and the optical tracking carrier 2 are packaged together into a mesh form, a spherical shape, or another shape to perform overall tracking on the anti-displacement mechanism 910-1.

The light-emitting ends 11-1 of the LED light source 11 are disposed on the anti-displacement mechanism 910-1, and are wrapped with the optical tracking carrier 2 made of the transparent material outside, and light emitted by the light-emitting ends 11-1 tracks the anti-displacement mechanism 910-1.

The light-emitting ends 11-1 are dispersedly disposed on the anti-displacement mechanism 910-1, to perform overall tracking on the anti-displacement mechanism 910-1.

The light-emitting ends 11-1 may be directly disposed on the anti-displacement mechanism 910-1, and are then wrapped with the optical tracking carrier 2 made of the transparent material outside. In this way, the light-emitting ends 11-1 may be completely joined to an outer contour of the anti-displacement mechanism 910-1, to perform tracking on any shape of the anti-displacement mechanism 910-1.

Furthermore, the light-emitting ends 11-1 are symmetrically disposed, and are wrapped with the optical tracking carrier 2 outside. Through the internal support of the light-emitting ends 11-1 and the circuit system 11-2 and the volume of the light-emitting ends 11-1, the anti-displacement mechanism 910-1 may be formed. The medical optical tracking system 500 can have both a tracking function and a positioning function of the anti-displacement mechanism 910-1.

The circuit system 11-2 is a flexible circuit board 11-21, the optical tracking carrier 2 is made of a soft transparent material, the light-emitting ends 11-1, the flexible circuit board 11-21, and the development mechanism 4 are disposed together in the optical tracking carrier 2, and are disposed on the anti-displacement mechanism 910-1, and when the anti-displacement mechanism 910-1 deforms, the light-emitting ends 11-1, the flexible circuit board 11-21, the development mechanism 4, and the optical tracking carrier 2 deform together along with the anti-displacement mechanism 910-1. Especially, when the anti-displacement mechanism 910-1 is made of a shape-memory alloy, as the shape of the anti-displacement mechanism 910-1 changes, the shape of the flexible circuit board 11-21 changes accordingly, to ensure the tracking effect of the anti-displacement mechanism 910-1.

The present invention provides a blood vessel tracking apparatus, including the medical optical tracking system 500.

The medical optical tracking system 500 may perform overall tracking on the blood vessel tracking apparatus 920. During clinical use, under the action of the medical optical tracking system 500, when a blood vessel being tracked is approached, it can be seen through tissue that the light emitted by the light source 1 is transmitted through the blood vessel, and the illuminance of the light emitted by the light source 1 ensures that at a distance of about 0 mm to 30 mm from a blood vessel wall, the blood vessel being tracked is clearly visible.

The blood vessel tracking apparatus 920 includes a development mechanism 4. The development mechanism 4 can perform a development prompt in X-rays, magnetic navigation, B-mode ultrasound, or another scenario, to facilitate the placement of the catheter 900-1. The development mechanism 4 may be made of a metal material, and may also have a heat conduction function. To keep the LED light source 11 from causing an excessively high temperature in a body, the temperature usually needs to be controlled within 37°C.

The blood vessel tracking apparatus 920 includes a coating 3, and the coating 3 is an anticoagulant coating.

The blood vessel tracking apparatus 920 includes a delivery end 920-1, a guide wire 920-2, a protection sleeve 5, and the medical optical tracking system 500; the medical optical tracking system 500 and the guide wire 920-2 are disposed in the protection sleeve 5, a coating 3 is provided outside the protection sleeve 5, and the coating 3 is an anticoagulant coating; the guide wire 920-2 is made of a metal material, and can perform development under X-rays, to form a development mechanism 4, and also has a heat conduction function; and the delivery end 920-1 can deliver the blood vessel tracking apparatus 920 to a position requiring blood vessel tracking.

Because the blood vessel tracking apparatus 920 includes the guide wire 920-2, under X-rays, the blood vessel tracking apparatus 920 may directly perform development. In addition, the guide wire 920-2 is made of a metal material, has a particular strength, and can be conveniently delivered. Therefore, the blood vessel tracking apparatus 920 does not require the cooperation of an additional separate medical guide wire to perform operation. The coating 3 is an anticoagulant coating, and can ensure that the blood vessel tracking apparatus 920 can safely remain in a blood vessel that requires tracking during surgery. The guide wire 920-2 also has a heat conduction function, and can keep the LED light source 11 from causing an excessively high temperature in a body, and the temperature usually needs to be controlled within 37°C.

The guide wire 920-2 is made of a shape-memory alloy, and a distal end of the blood vessel tracking apparatus 920 is shaped, to form a shaping mechanism 25. The guide wire 920-2 may shape the distal end of the blood vessel tracking apparatus 920 as required to adapt to a radian of a blood vessel, making a delivery process more convenient.

The medical optical tracking system 500 is provided with a channel 24, and the channel 24 forms a guide wire operation hole 920-3. In the guide wire operation hole 920-3, a separate medical guide wire may be inserted for cooperation to perform a delivery operation, and the structure of the medical optical tracking system 500 is simple.

A side wall of the optical tracking carrier 2 is made of a transparent material, a light guide optical fiber 22 is provided in the side wall, the light guide optical fiber 22 has a non-smooth surface 22-1, light outlets 22-2 are provided in a side surface in a length direction, a coating 3 is provided on an outer surface of the optical tracking carrier 2, and the coating 3 is an anticoagulant coating; a proximal end of the light guide optical fiber 22 is connected to a light guide joint 26, and under the action of the light emitted by the light source 1, the light guide optical fiber 22 may overall emit light, to track a blood vessel. The light guide optical fiber 22 has a simple structure and a small size and is suitable for tracking a blood vessel with a small size.

The light source 1 is a medical cold light source 12. The medical cold light source 12 does not cause a rise in a temperature of the optical tracking carrier 2 disposed in vivo, and a blood vessel tracking process is safer.

A side wall of the optical tracking carrier 2 is made of a transparent material, the light source 1 is an LED light source 11, and light-emitting ends 11-1 of the LED light source 11 are dispersedly disposed in the side wall of the optical tracking carrier 2, to form an LED lamp strip or an LED lamp net, to track a blood vessel. The LED light source 11 directly tracks a blood vessel, an illumination effect is good, and the tracking of a large blood vessel has a very good effect.

During clinical use, in an X-ray environment, a blood vessel interventional therapy technique is used, the blood vessel tracking apparatus 920 is placed at a position that requires tracking, and a power supply is turned on. Under the action of the light source 1, the optical fiber tracking carrier 2 works to emit visible light to track a blood vessel. In surgery, when a blood vessel being tracked is approached, it can be seen through tissue that the light emitted by the light source 1 is transmitted through the blood vessel, and the illuminance of the light emitted by the light source 1 ensures that at a distance of about 0 mm to 30 mm from a blood vessel wall, the blood vessel being tracked is clearly visible.

A medical optical tracking system of the present invention includes a light source 1 and an optical tracking carrier 2. The optical tracking carrier 2 includes a light guide material. Light emitted by the light source 1 is conducted through the optical tracking carrier 2, and optical tracking is performed on the optical tracking carrier 2. The light source 1 may be a micro LED light source 11, and light-emitting ends 11-1 of the LED light source 11 and the optical tracking carrier 2 are directly integrated and placed in a human body to perform optical tracking. The medical optical tracking system of the present invention may be disposed as required in the medical catheter 900 with a tracking apparatus, or the solid tumor tracking apparatus 910, or the blood vessel tracking apparatus 920. In clinical surgery, light sources of different colors are disposed to accurately discern a blood vessel, a cavity or tube, tissue or an organ that requires special protection in clinical surgery, to effectively avoid an accident during surgery, or effectively calibrate a small size tumor, thereby facilitating effective implementation of clinical surgery.

### BRIEF DESCRIPTION OF THE DRAWINGS

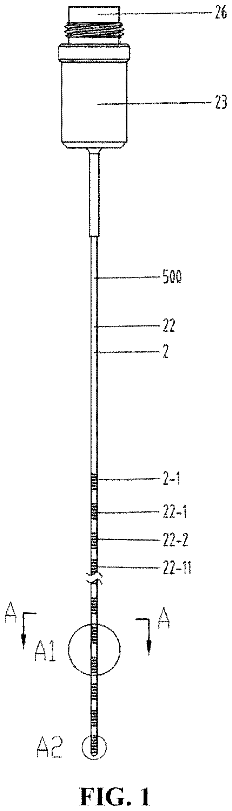

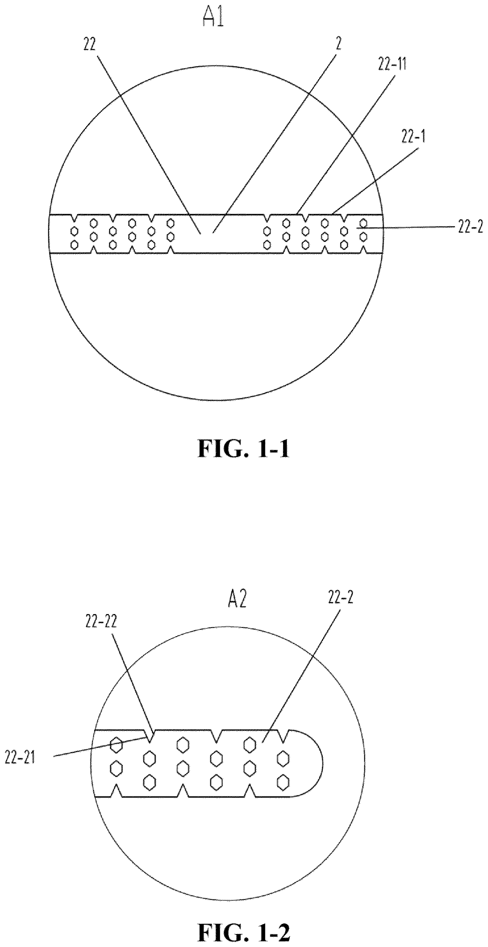

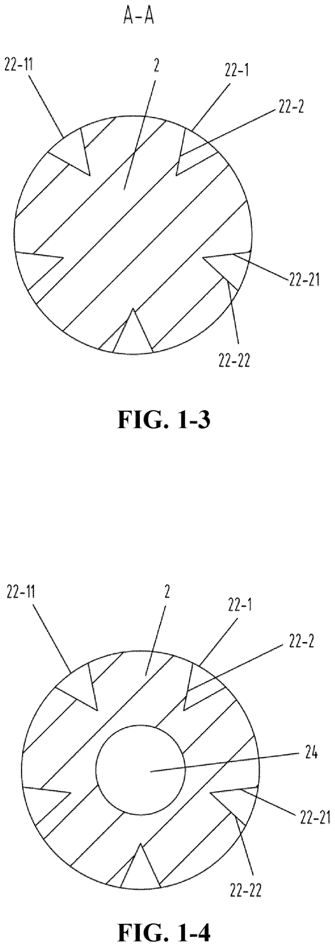

FIG. 1 is a schematic structural diagram of a light guide optical fiber that has a non-smooth surface and is provided with irregular light outlets.
FIG. 1-1 is an enlarged view of A1 in FIG. 1.
FIG. 1-2 is an enlarged view of A2 in FIG. 1.
FIG. 1-3 is a cross-sectional view along A-A in FIG. 1.
FIG. 1-4 is a sectional view of a light guide optical fiber with a channel.
FIG. 2 is a schematic structural diagram of a light guide optical fiber that has a non-smooth surface and is provided with annular light outlets.
FIG. 2-1 is an enlarged view of B1 in FIG. 2.
FIG. 2-2 is an enlarged view of B2 in FIG. 2.
FIG. 3 is a schematic structural diagram of a light guide optical fiber that has a non-smooth surface and is provided with spiral light outlets.
FIG. 3-1 is an enlarged view of C1 in FIG. 3.
FIG. 3-2 is an enlarged view of C2 in FIG. 3.
FIG. 4 is a schematic structural diagram of a system with a development string and an optical fiber being disposed in a protection sleeve.
FIG. 4-1 is a cross-sectional view along D-D in FIG. 4.
FIG. 4-2 is a sectional view of a system including a plurality of light guide optical fibers.
FIG. 5 is a schematic structural diagram of a system including a development block.
FIG. 5-1 is an enlarged view of E1 in FIG. 5.
FIG. 6 is a schematic structural diagram of a system including a development ring.
FIG. 6-1 is an enlarged view of F1 in FIG. 6.
FIG. 7 is a schematic structural diagram of a system including a shaping mechanism.
FIG. 8 is a schematic diagram of a joint of a system including a coating.
FIG. 8-1 is a cross-sectional view along G-G in FIG. 8.
FIG. 9 is a medical optical tracking system of the present invention including a medical cold light source.
FIG. 10 is a schematic structural diagram of an optical tracking carrier including a light-retaining self-luminous tracking carrier.
FIG. 10-1 is a cross-sectional view along H-H in FIG. 10.
FIG. 11 is a schematic structural diagram of an optical tracking carrier in which light-emitting ends of an LED light source are embedded.
FIG. 11-1 is a cross-sectional view along I-I in FIG. 11.
FIG. 11-2 is a sectional view along J-J in FIG. 11.
FIG. 11-3 is an enlarged view of I1 in FIG. 11-1.
FIG. 12 is a schematic structural diagram of an optical tracking carrier in which light-emitting ends of an LED light source including a development string are embedded.
FIG. 12-1 is a cross-sectional view along K-K in FIG. 12.
FIG. 12-2 is a cross-sectional view along L-L in FIG. 12.
FIG. 12-3 is an enlarged view of K1 in FIG. 12-1.
FIG. 13 is a medical optical tracking system of the present invention including an LED light source.
FIG. 14 is a schematic structural diagram of a blood vessel tracking apparatus including a development wire.
FIG. 14-1 is a cross-sectional view along M-M in FIG. 14.
FIG. 14-2 is a sectional view of a blood vessel tracking apparatus including a guide wire operation hole.
FIG. 15 shows an optical tracking carrier with an optical fiber plaited in a mesh form.
FIG. 16 is a diagram of a working principle of a blood vessel tracking apparatus being placed in a blood vessel.
FIG. 17 is a three-dimensional schematic structural diagram of a medical stomach tube with a visible-light tracking apparatus of the present invention.
FIG. 17-1 is a cross-sectional view along N-N in FIG. 17.
FIG. 17-2 is an enlarged view of N1 in FIG. 17-1.
FIG. 17-3 is a schematic structural diagram after an LED light source is connected in FIG. 17.
FIG. 17-4 is a schematic structural diagram after a cold light source is connected in FIG. 17.
FIG. 17-5 is a schematic structural diagram of a medical stomach tube including a light guide optical fiber with a smooth surface.
FIG. 17-6 is an enlarged view of N2 in FIG. 17-5.
FIG. 17-7 is an enlarged view of N3 in FIG. 17-6.
FIG. 17-8 is a schematic structural diagram of a medical stomach tube with an LED light-emitting end disposed at an end portion.
FIG. 17-9 is an enlarged view of N4 in FIG. 17-8.

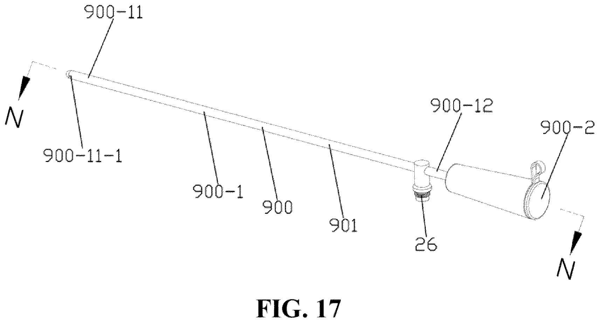

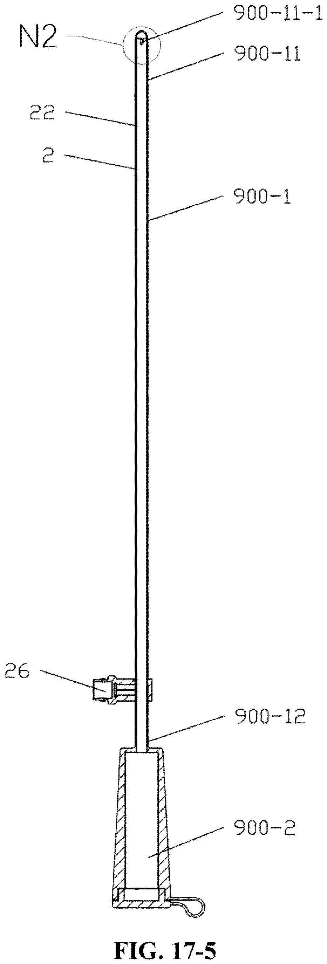

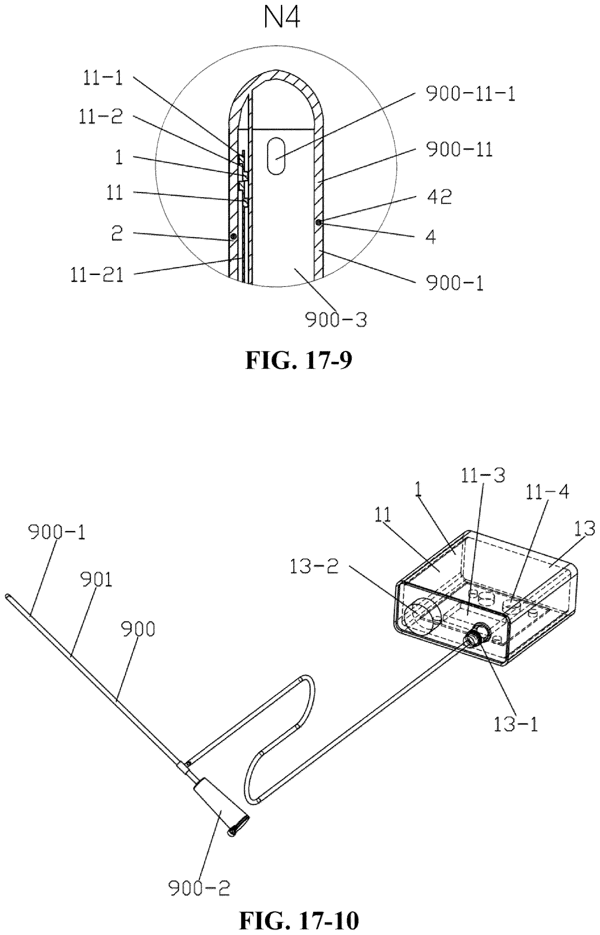

FIG. 17-10 is a schematic structural diagram after an LED light source is connected in FIG. 17-5.

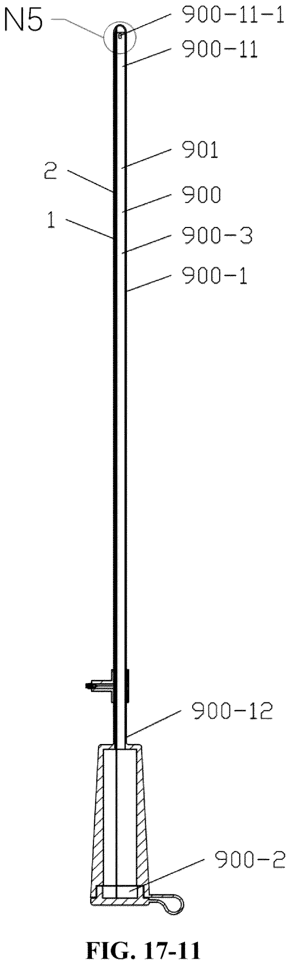

FIG. 17-11 is a schematic structural diagram of a medical stomach tube with LED light-emitting ends disposed overall.

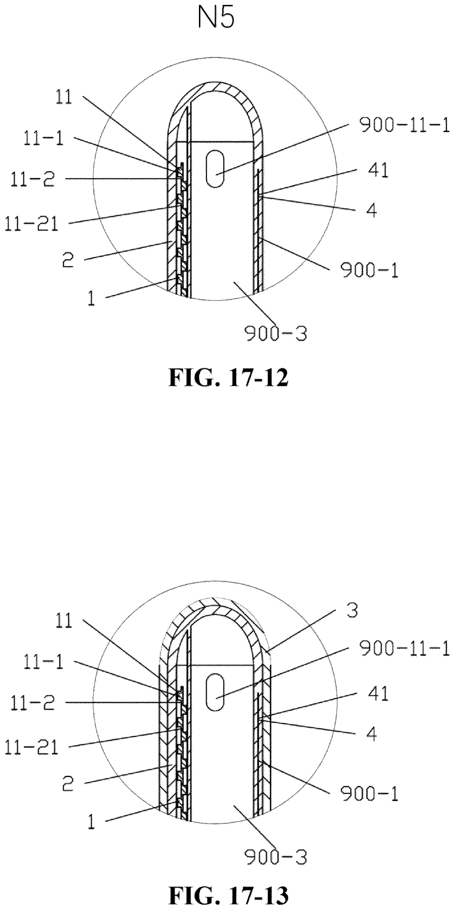

FIG. 17-12 is an enlarged view of N5 in FIG. 17-11.
FIG. 17-13 is a schematic structural diagram of a medical stomach tube including a hydrophilic coating.

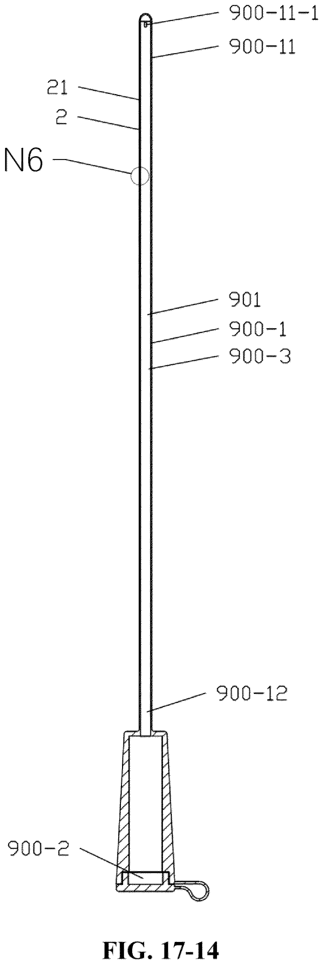

FIG. 17-14 is a schematic structural diagram of a medical stomach tube including a light-retaining self-luminous tracking carrier.

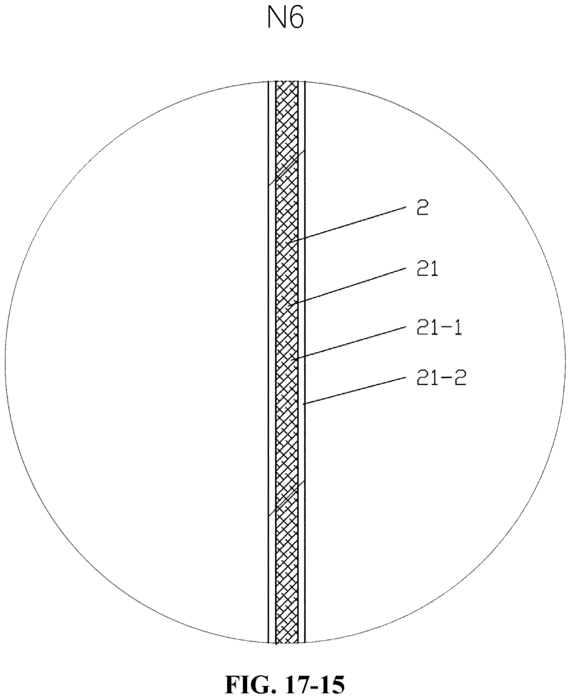

FIG. 17-15 is an enlarged view of N6 in FIG. 17-14.

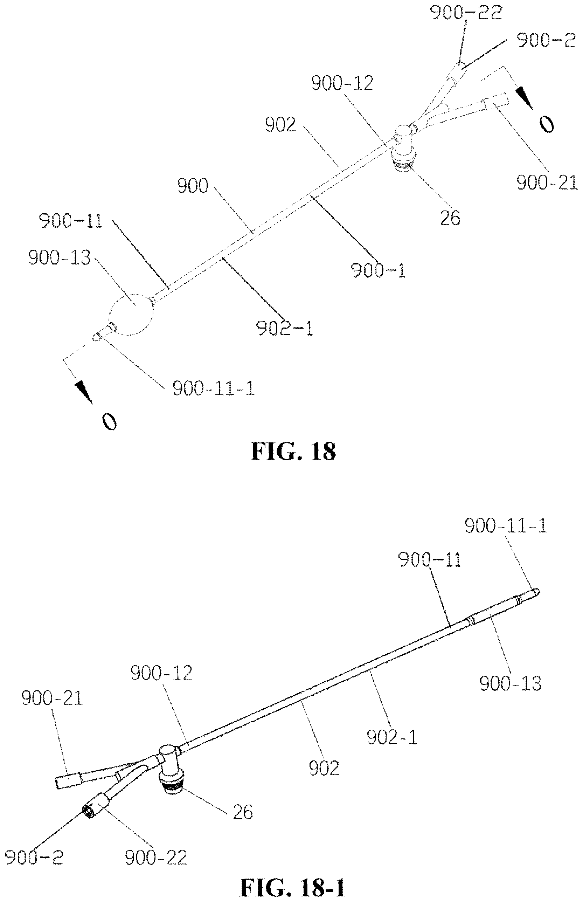

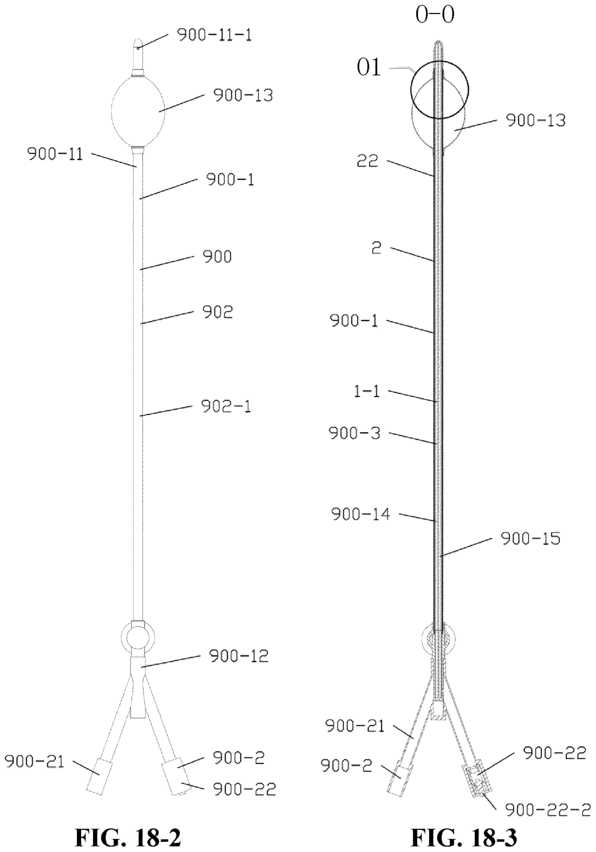

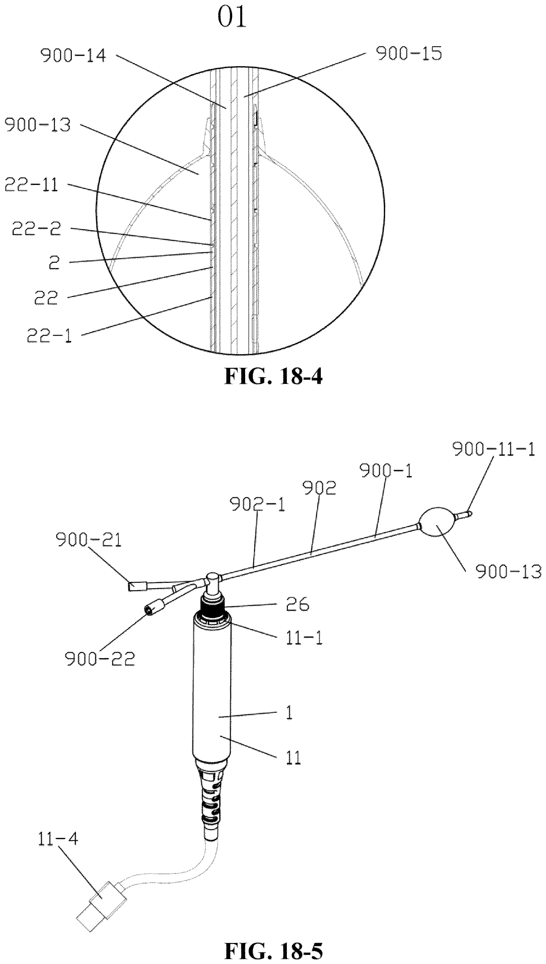

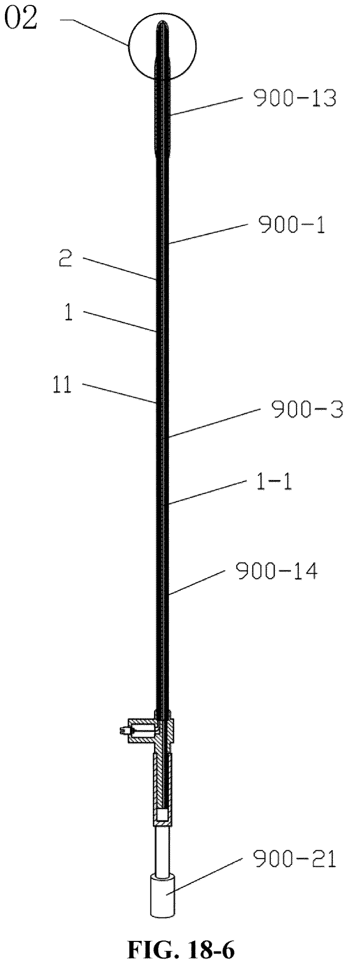

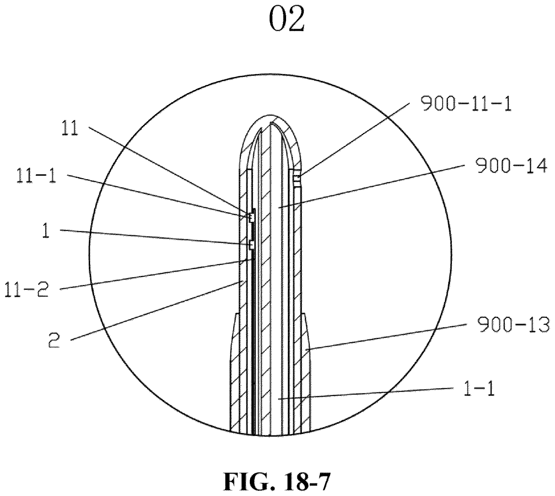

FIG. 18 is a schematic structural diagram when a balloon of a 2-lumen medical urinary catheter expands.
FIG. 18-1 is a schematic structural diagram when the balloon in FIG. 18 contracts.
FIG. 18-2 is a front view of FIG. 18.
FIG. 18-3 is a cross-sectional view along O-O in FIG. 18.
FIG. 18-4 is an enlarged view of O1 in FIG. 18-3.
FIG. 18-5 is a schematic structural diagram when an LED light source is connected in FIG. 18.
FIG. 18-6 is a schematic structural diagram of a 2-lumen medical urinary catheter with LED light-emitting ends provided in a tube wall.
FIG. 18-7 is an enlarged view of O2 in FIG. 18-6.

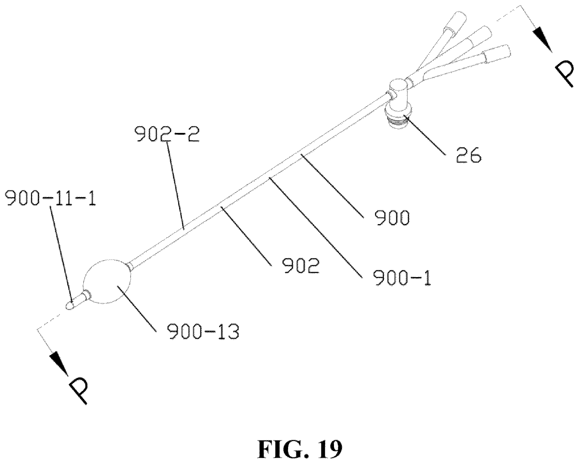

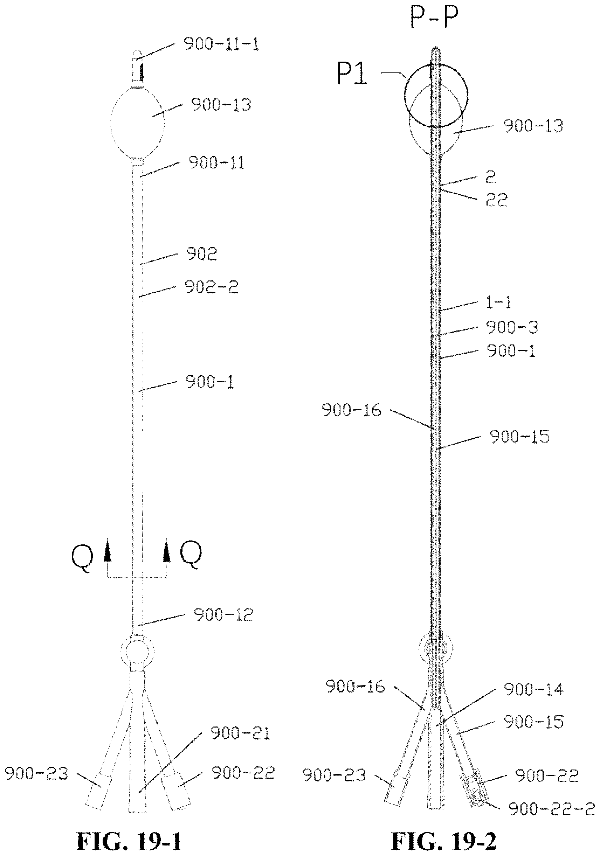

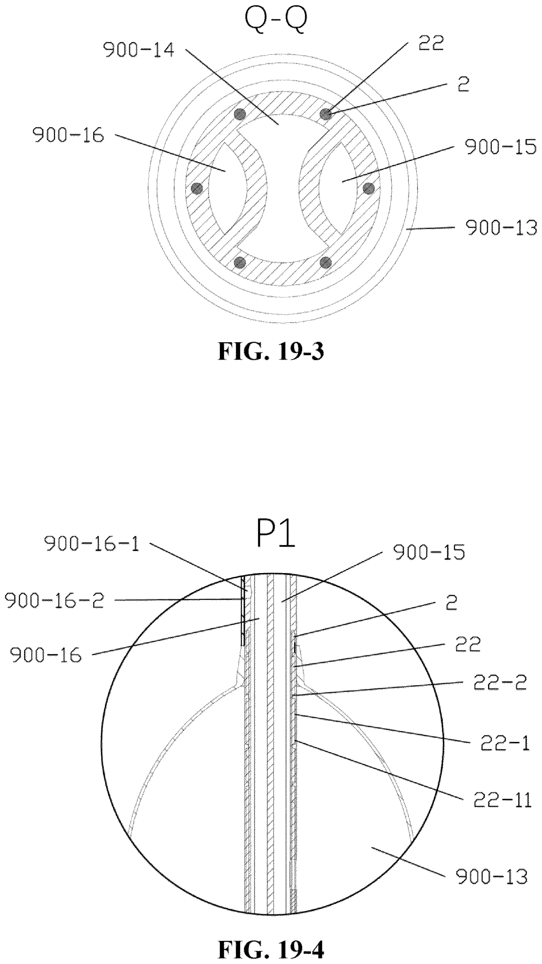

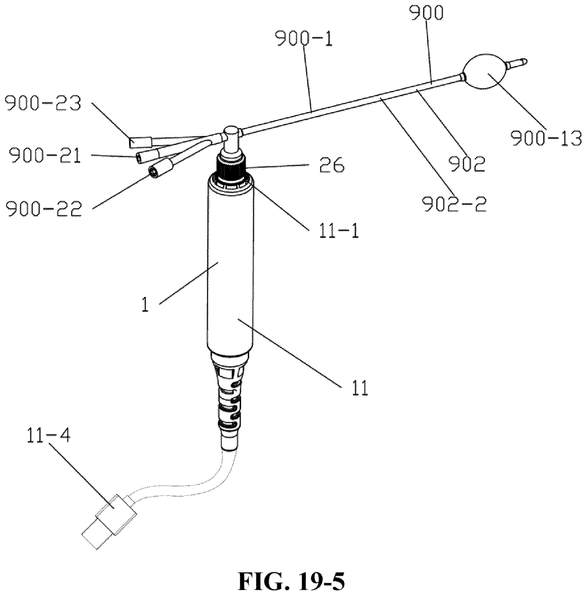

FIG. 19 is a schematic structural diagram when a balloon of a 3-lumen medical urinary catheter expands.
FIG. 19-1 is a front view of FIG. 19.
FIG. 19-2 is a cross-sectional view along P-P in FIG. 19.
FIG. 19-3 is a cross-sectional view along Q-Q in FIG. 19-1.
FIG. 19-4 is an enlarged view of P1 in FIG. 19-2.
FIG. 19-5 is a schematic structural diagram when an LED light source is connected in FIG. 19.

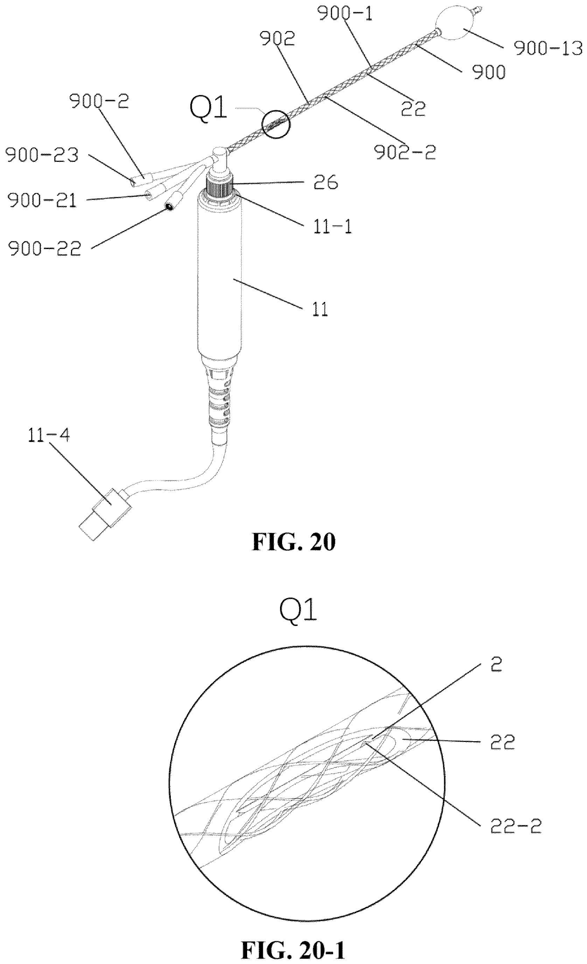

FIG. 20 is a schematic structural diagram when a 3-lumen medical urinary catheter with an optical fiber plaited in a mesh form connected to an LED light source.
FIG. 20-1 is an enlarged view of Q1 in FIG. 20.

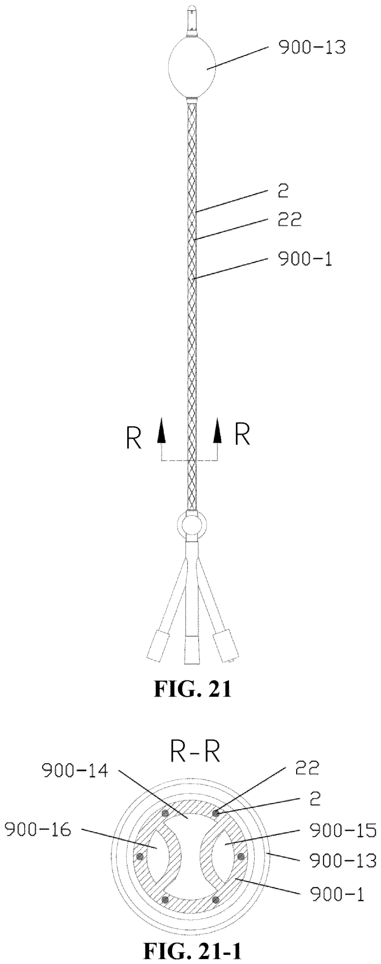

FIG. 21 is a schematic structural diagram of a 3-lumen medical urinary catheter with an optical fiber plaited in a mesh form.
FIG. 21-1 is a cross-sectional view along R-R in FIG. 21.

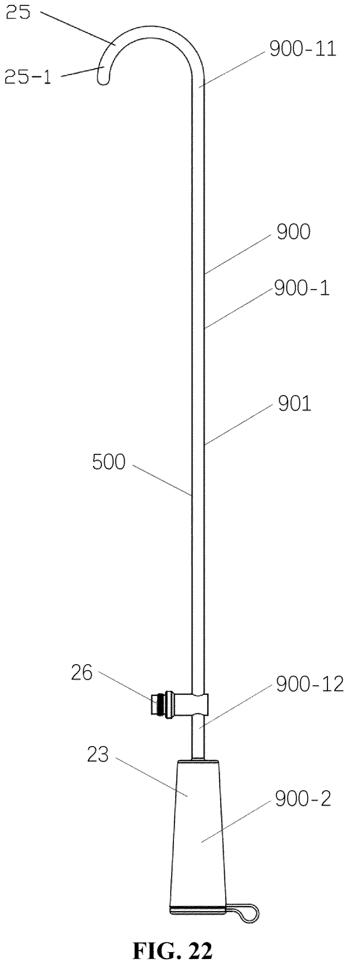

FIG. 22 is a schematic structural diagram of a medical catheter including a shaping mechanism.

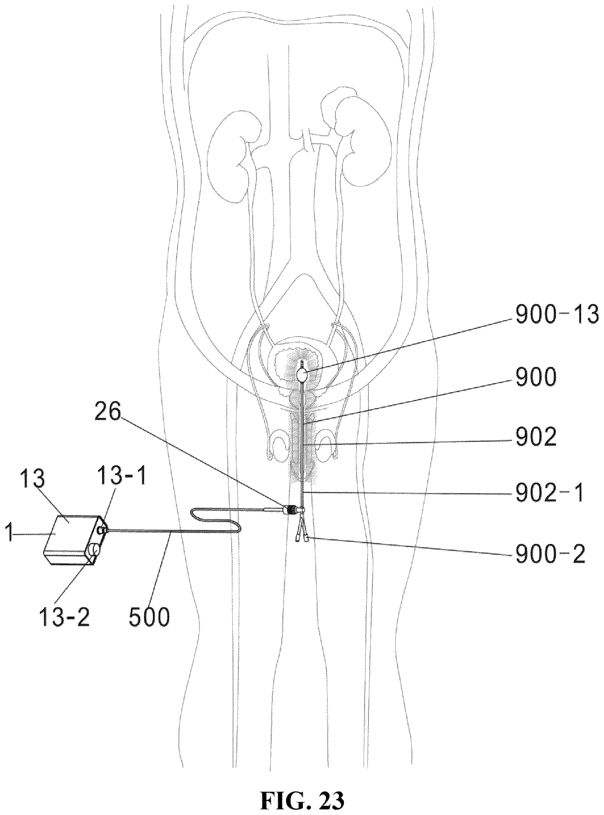

FIG. 23 is a diagram of a working principle when a medical urinary catheter is inserted into a bladder.

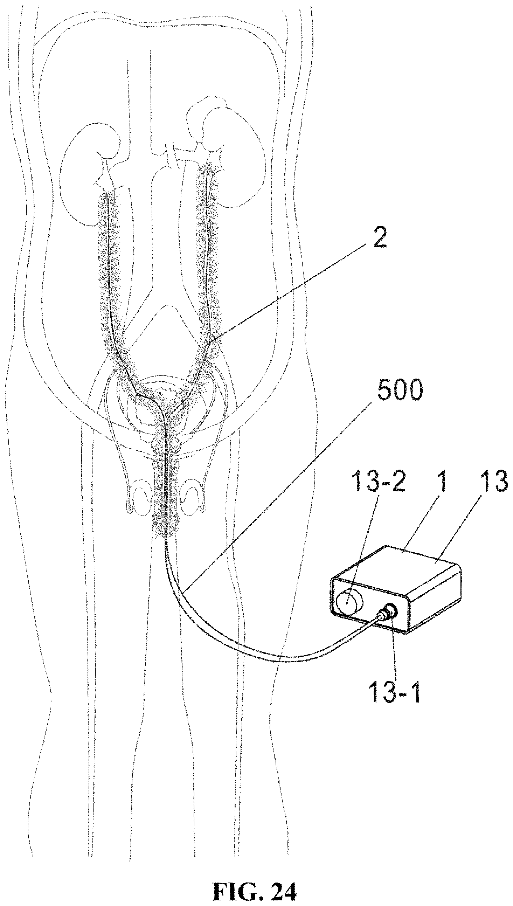

FIG. 24 is a diagram of a working principle when a medical urinary catheter is inserted into a ureter.

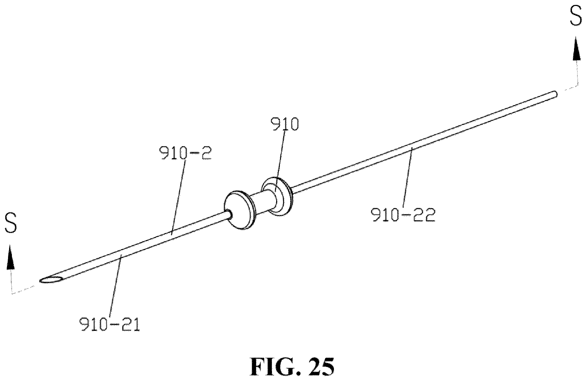

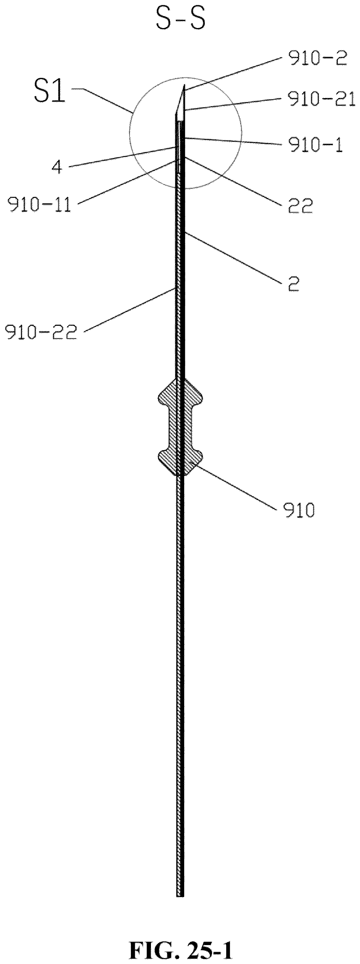

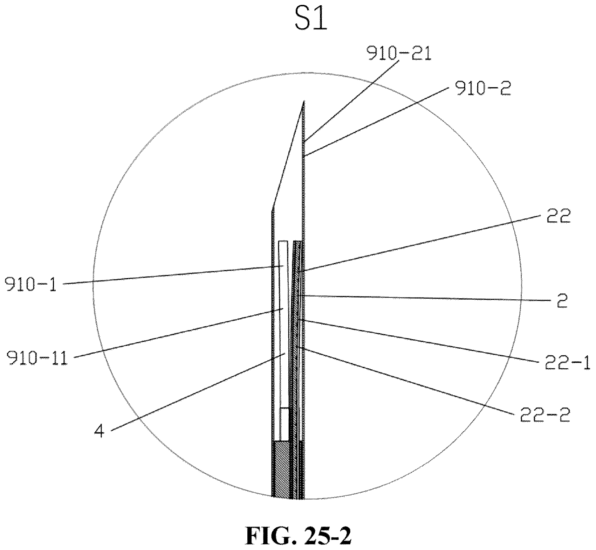

FIG. 25 is a three-dimensional schematic structural diagram of a positioning hook of a solid tumor tracking apparatus of the present invention being retracted in a delivery sheath.
FIG. 25-1 is a cross-sectional view along S-S in FIG. 25.
FIG. 25-2 is an enlarged view of S1 in FIG. 25.

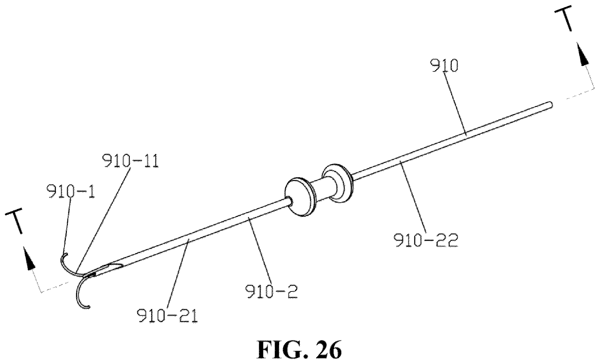

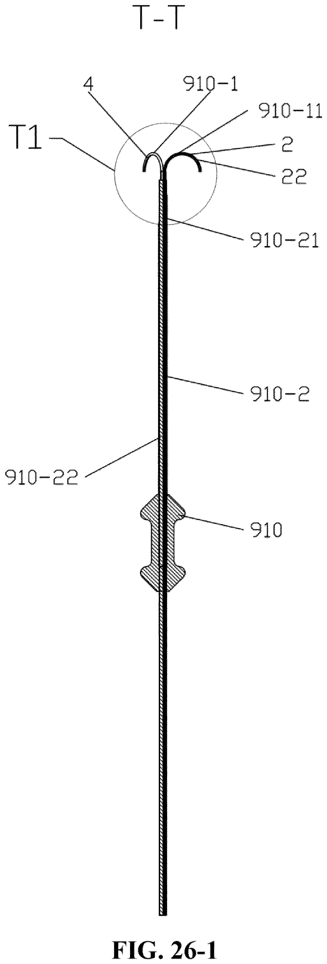

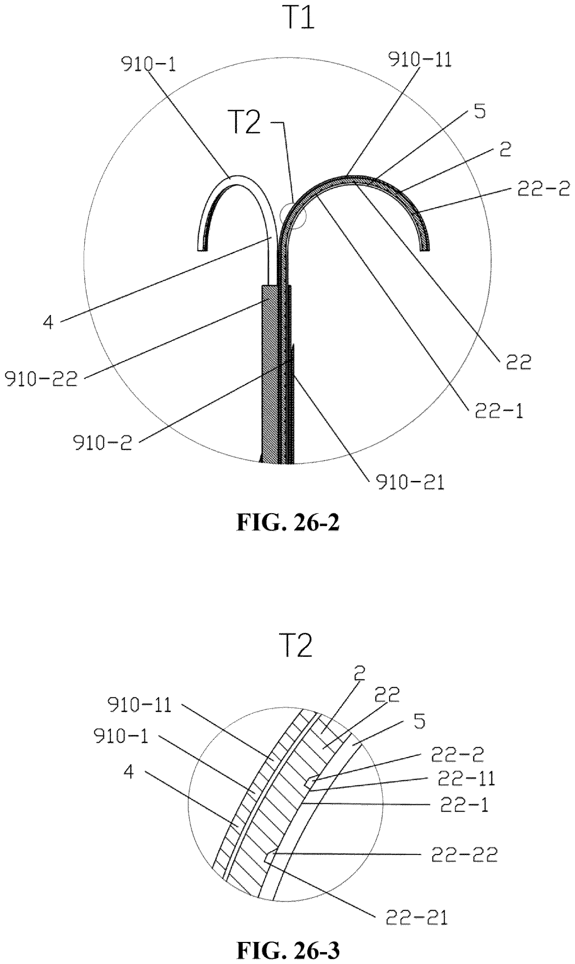

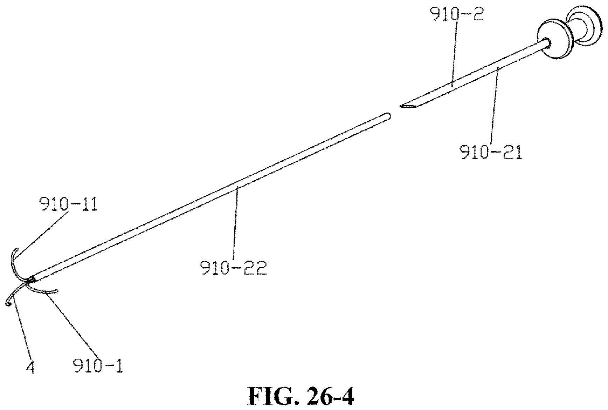

FIG. 26 is a schematic structural diagram when the positioning hook in FIG. 25 is pushed out.
FIG. 26-1 is a cross-sectional view along T-T in FIG. 26.
FIG. 26-2 is an enlarged view of T1 in FIG. 26-1.
FIG. 26-3 is an enlarged view of T2 in FIG. 26-2.
FIG. 26-4 is an exploded view of FIG. 26.

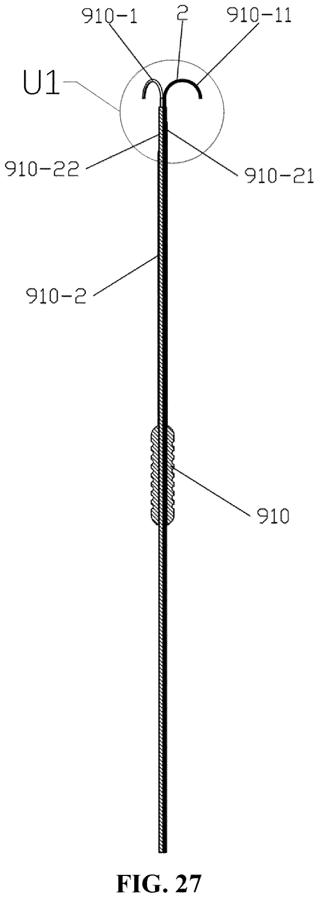

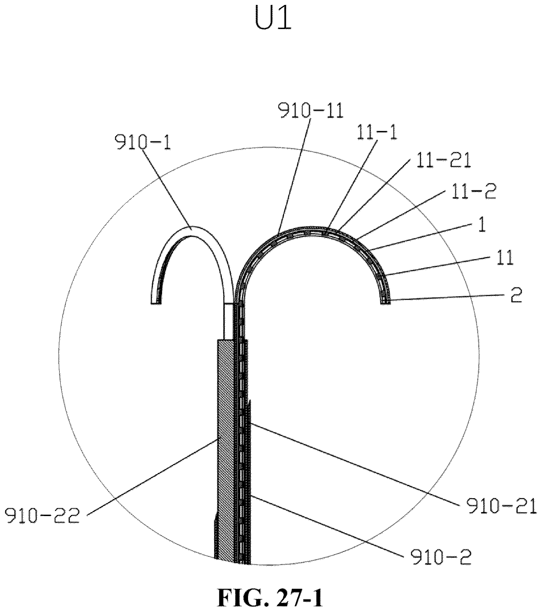

FIG. 27 is a schematic structural diagram of a light-emitting end of an LED light source being disposed on a positioning hook.
FIG. 27-1 is an enlarged view of U1 in FIG. 27.

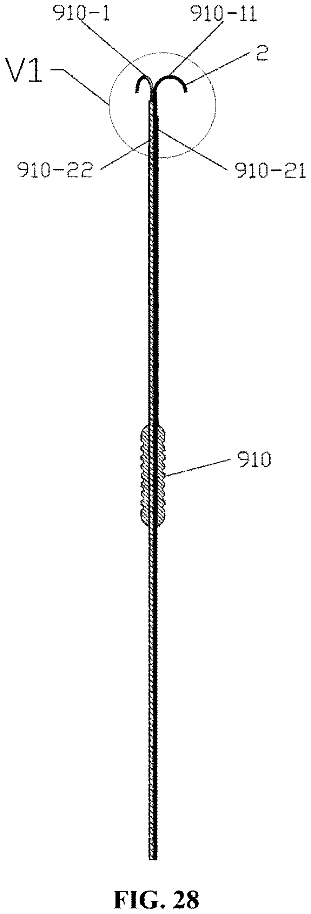

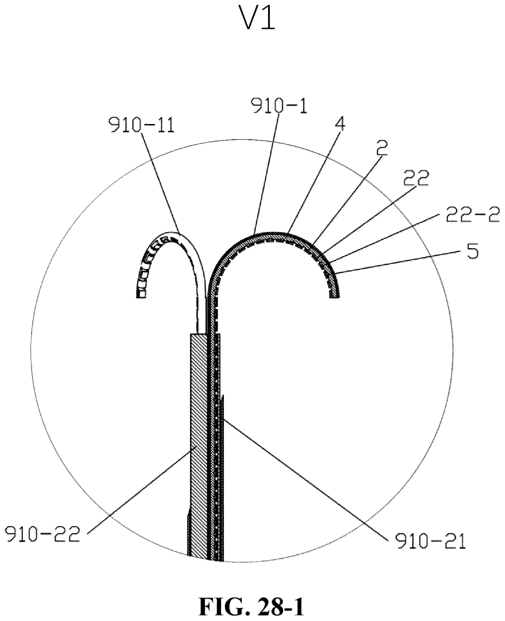

FIG. 28 is a schematic structural diagram of a solid tumor tracking apparatus of the present invention including intermittent opening sleeves.
FIG. 28-1 is an enlarged view of V1 in FIG. 28.

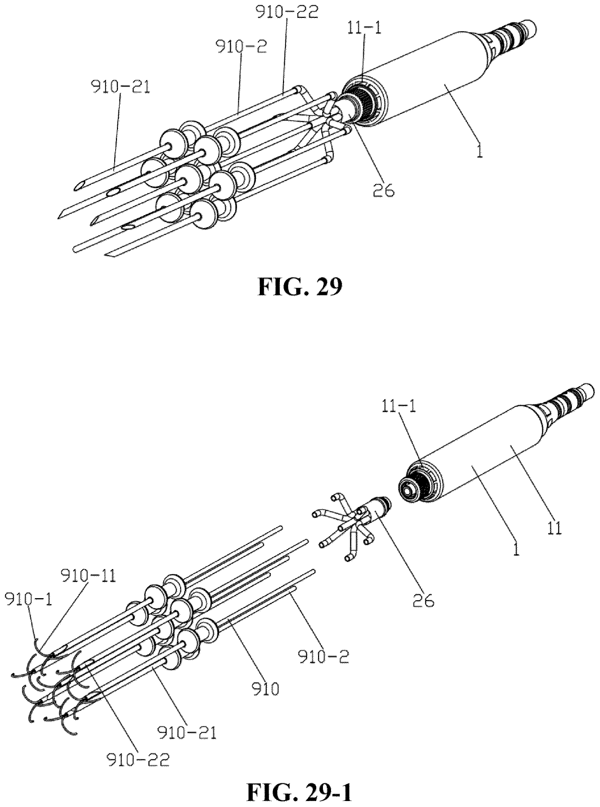

FIG. 29 is a schematic structural diagram including a plurality of solid tumor tracking apparatuses.
FIG. 29-1 is an exploded view of FIG. 29.

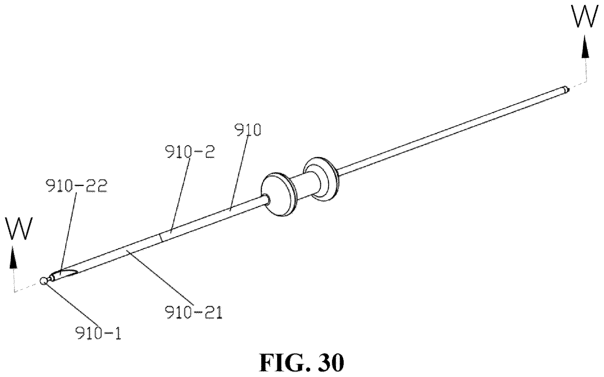

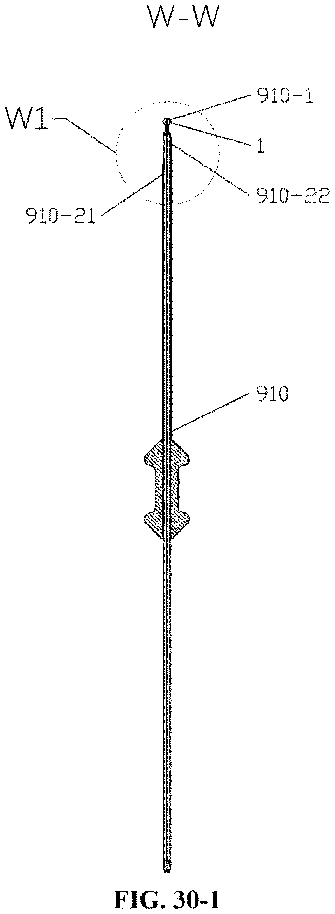

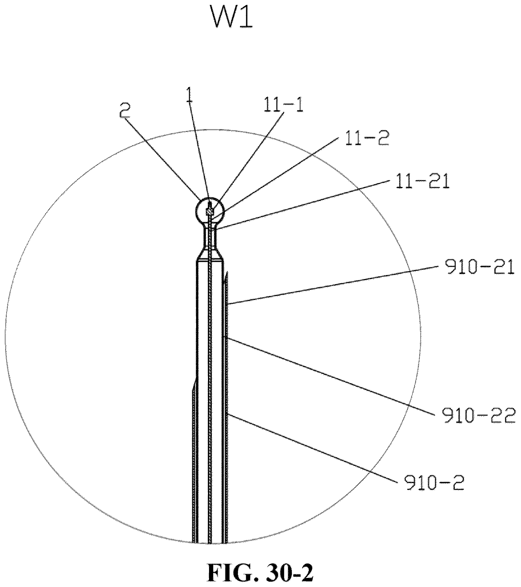

FIG. 30 is a schematic structural diagram of a solid tumor tracking apparatus of the present invention including a spherical anti-displacement mechanism.
FIG. 30-1 is a cross-sectional view along W-W in FIG. 30.
FIG. 30-2 is an enlarged view of W1 in FIG. 30-1.

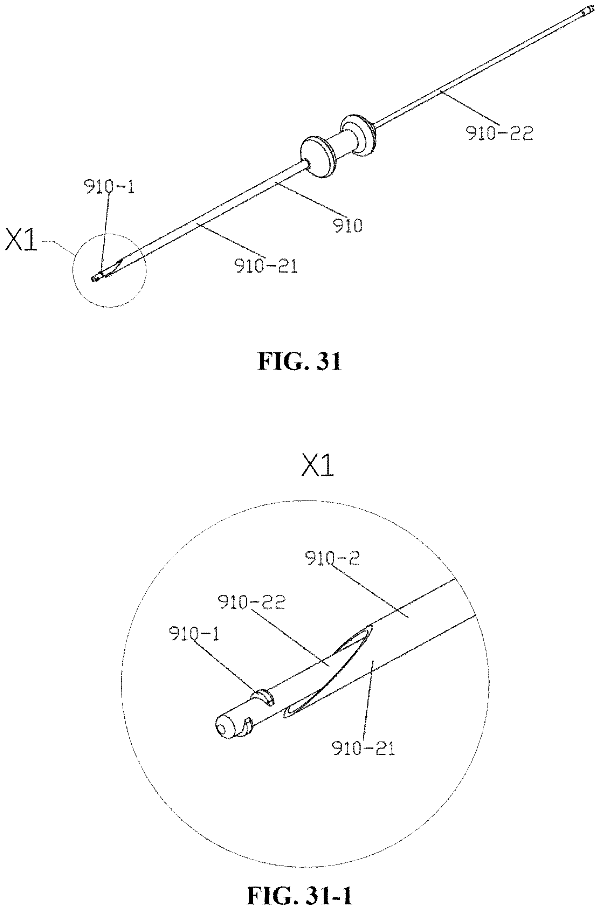

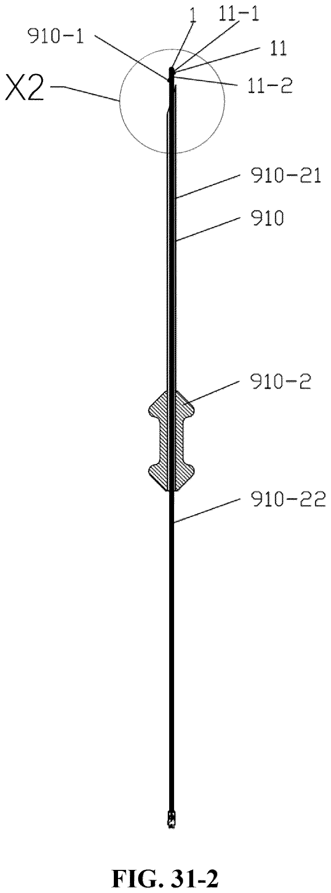

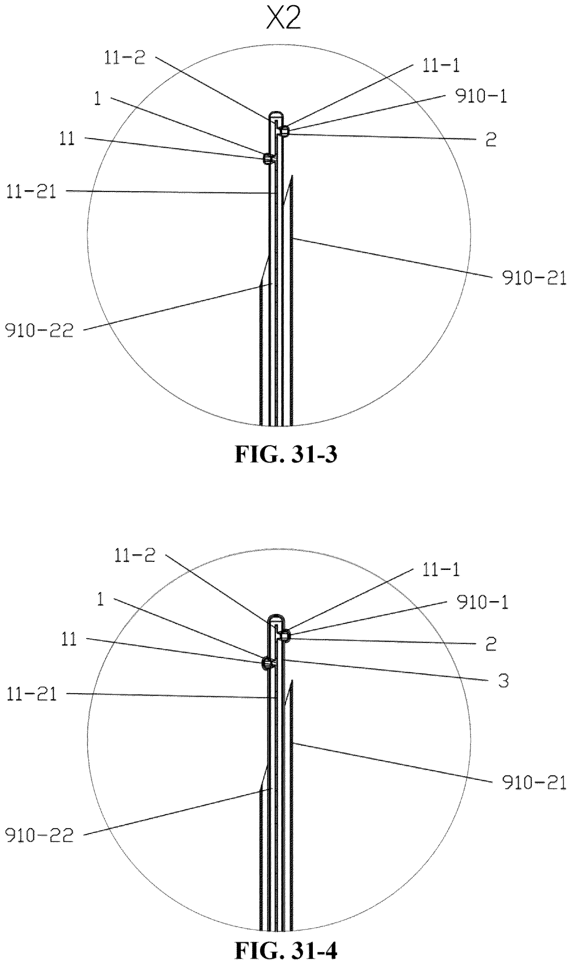

FIG. 31 is a schematic structural diagram of a solid tumor tracking apparatus of the present invention including a dumbbell anti-displacement mechanism.
FIG. 31-1 is an enlarged view of X1 in FIG. 31.
FIG. 31-2 is a cross-sectional view of FIG. 31.
FIG. 31-3 is an enlarged view of X2 in FIG. 31-2.
FIG. 31-4 is a schematic structural diagram including a dumbbell anti-displacement mechanism with a coating.

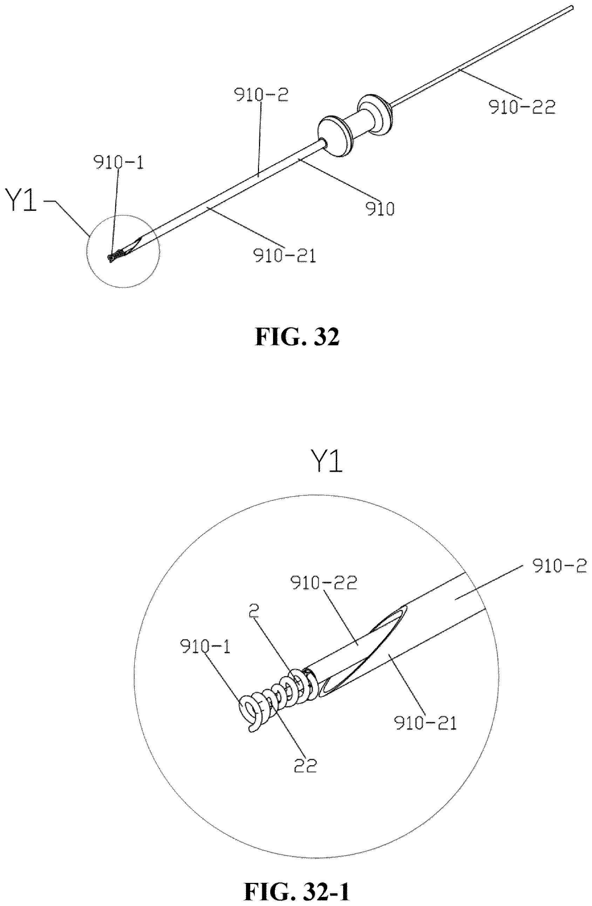

FIG. 32 is a schematic structural diagram of a solid tumor tracking apparatus of the present invention including a spiral-type anti-displacement mechanism.
FIG. 32-1 is an enlarged view of Y1 in FIG. 32.

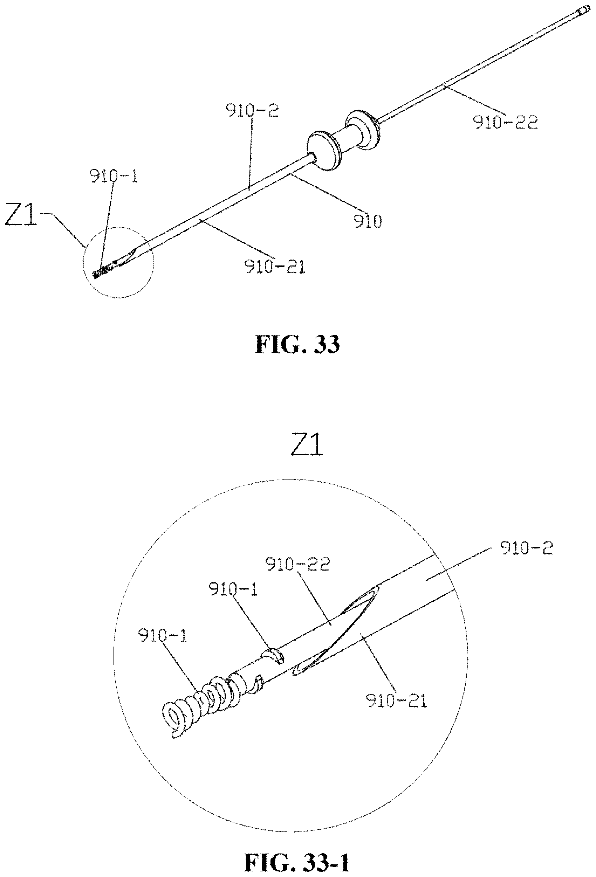

FIG. 33 is a schematic structural diagram of a solid tumor tracking apparatus of the present invention including a spiral-type anti-displacement mechanism and a dumbbell anti-displacement mechanism.
FIG. 33-1 is an enlarged view of Z1 in FIG. 33.

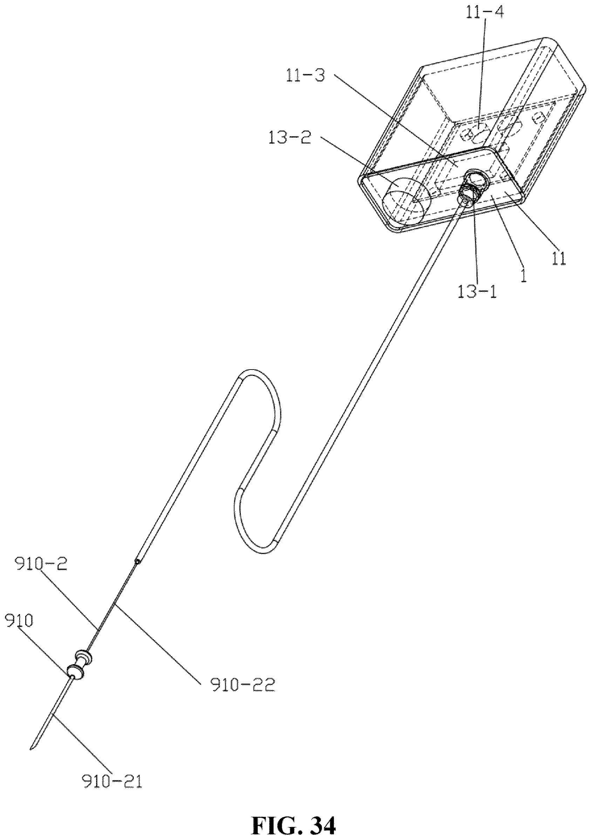

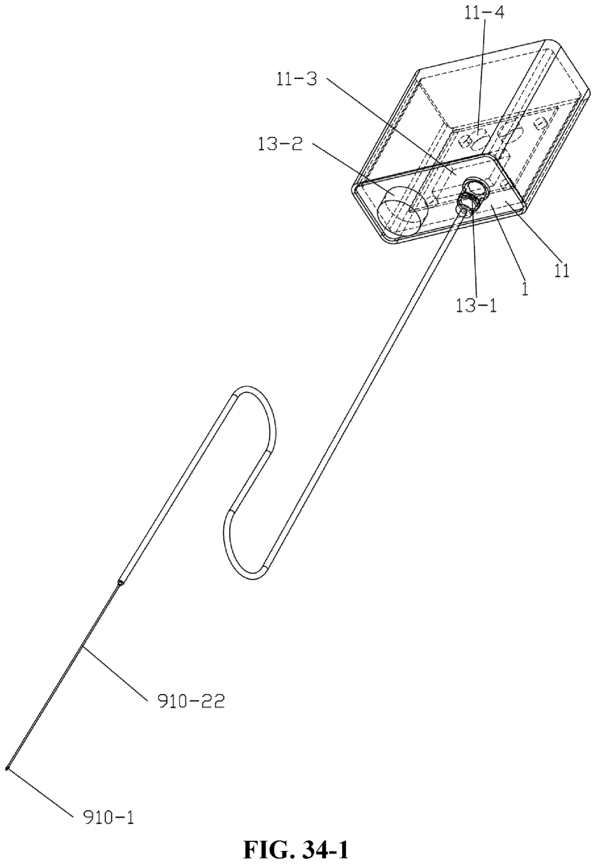

FIG. 34 is a schematic structural diagram of a solid tumor tracking apparatus of the present invention including an LED light source.
FIG. 34-1 is a schematic structural diagram after a delivery mechanism in FIG. 34 is removed.

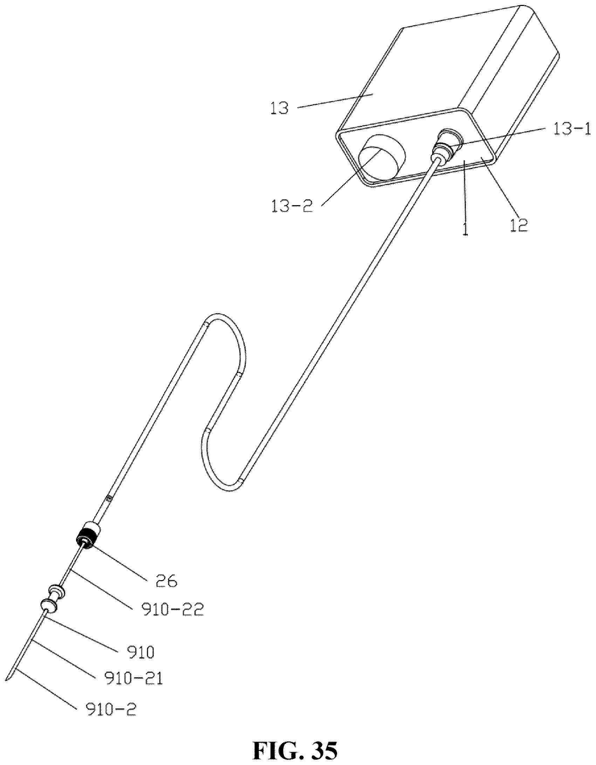

FIG. 35 is a schematic structural diagram of a solid tumor tracking apparatus of the present invention including a medical cold light source.

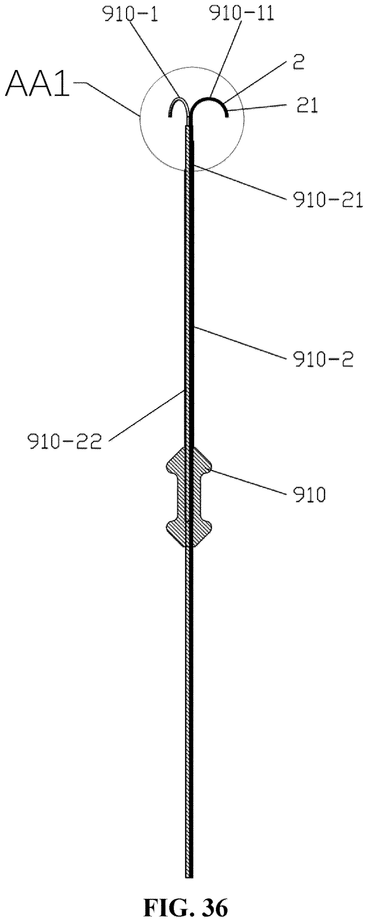

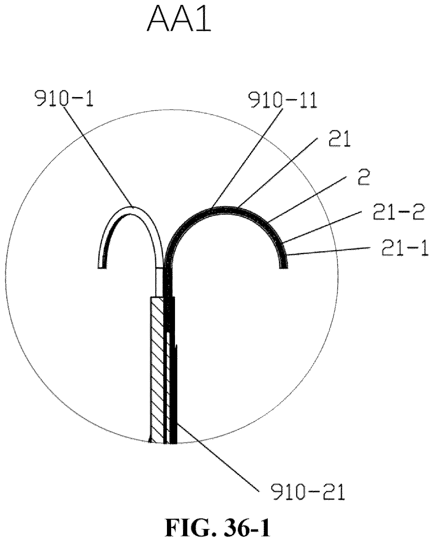

FIG. 36 is a schematic structural diagram of a solid tumor tracking apparatus of the present invention including a light-retaining self-luminous tracking carrier.
FIG. 36-1 is an enlarged view of AA1 in FIG. 36.

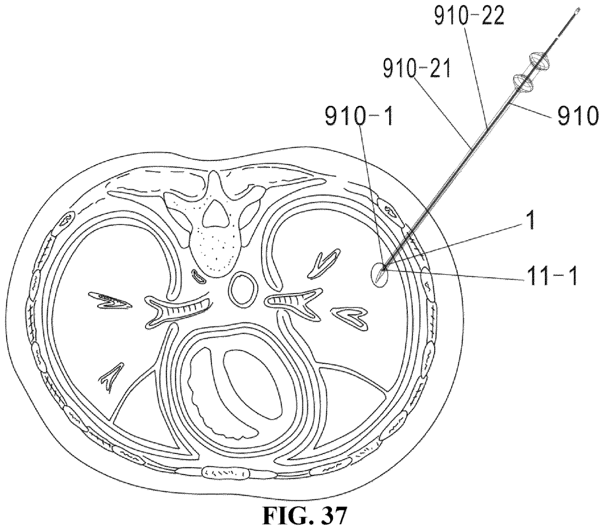

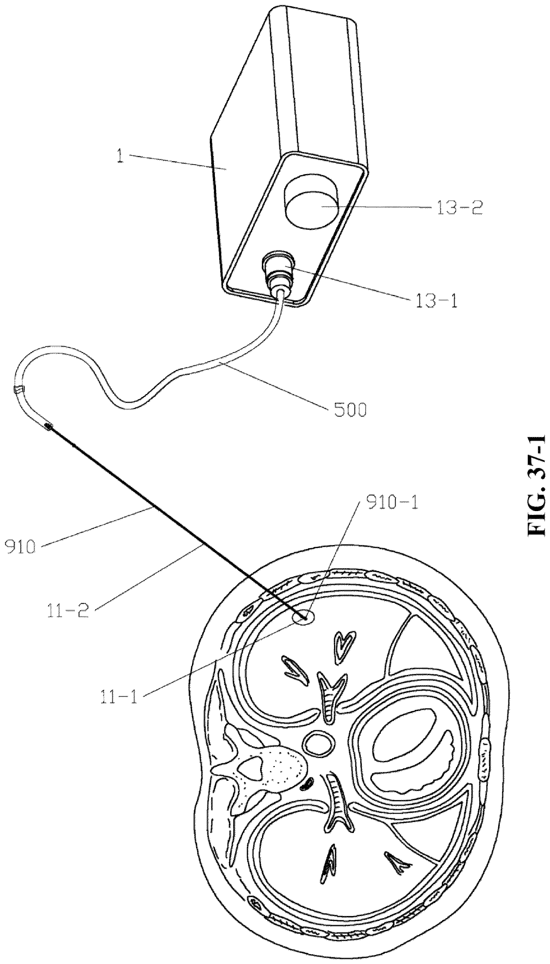

FIG. 37 is a diagram of a working principle when a solid tumor tracking apparatus of the present invention is inserted into a pulmonary nodule.
FIG. 37-1 is a diagram of a working principle after a delivery mechanism is removed and a light guide optical fiber is connected to a light source in FIG. 37.

In the figures:
500 is a medical optical tracking system of the present invention, 900 is a medical catheter with a tracking apparatus of the present invention, 901 is a medical stomach tube, 902 is a medical urinary catheter, 910 is a solid tumor tracking apparatus of the present invention, and 920 is a blood vessel tracking apparatus of the present invention.
1 is a light source, 2 is an optical tracking carrier, 3 is a coating, 4 is a development mechanism, and 5 is a protection sleeve.
11 is an LED light source, 12 is a medical cold light source, 13 is a control system, 11-1 is a light-emitting end, 11-2 is a circuit system, 11-3 is a drive board, 11-4 is a power supply, 13-1 is a wavelength adjustment mechanism, 13-2 is a light intensity adjustment mechanism, and 11-21 is a flexible circuit board.
2-1 is a working portion, 21 is a light-retaining self-luminous tracking carrier, 22 is a light guide optical fiber, 23 is a delivery portion, 24 is a channel, 25 is a shaping mechanism, and 26 is a light guide joint; 21-1 is a light-retaining self-luminous body, and 21-2 is a protective carrier; 22-1 is a non-smooth surface, 22-2 is a light outlets, 23-1 is a delivery handle, and 25-1 is a shape-memory shaping mechanism; and 22-11 is a non-smooth surface that can form reflection and/or scattering, 22-21 is a conduction surface, and 22-22 is a reflection surface.
41 is a development string, 42 is a development ring, and 43 is a development block.
1-1 is a cavity channel, 900-1 is a catheter, 900-2 is an interface, 900-3 is a working channel, 902-1 is a double-lumen urinary catheter, and 902-2 is a triple-cavity urinary catheter; and 900-11 is a distal end, 900-12 is a proximal end, 900-13 is a balloon, 900-14 is a urinary lumen, 900-15 is a water injection lumen, 900-16 is an infusion flushing lumen, 900-21 is a fluid drainage port, 900-22 is a water injection port, 900-23 is a flushing drug injection port, 900-11-1 is a working opening, 900-16-1 is a flushing port, 900-16-2 is a stop valve, and 900-22-2 is a shut-off valve.
910-1 is an anti-displacement mechanism, and 910-2 is a delivery mechanism; and 910-11 is a positioning hook, 910-21 is a delivery sheath, and 910-22 is a pushing mechanism.
920-1 is a delivery end, 920-2 is a guide wire, and 920-3 is a guide wire operation hole.

### DESCRIPTION OF EMBODIMENTS

### Embodiment 1: Medical optical tracking system of the present invention

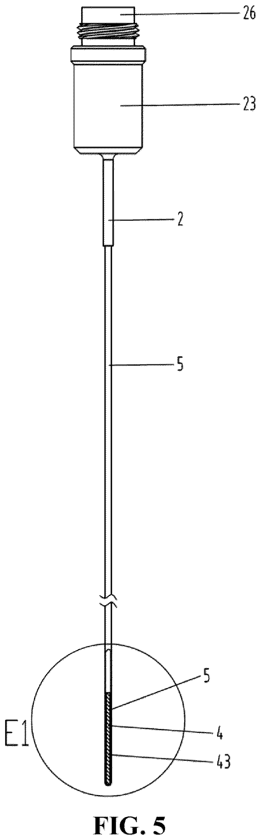

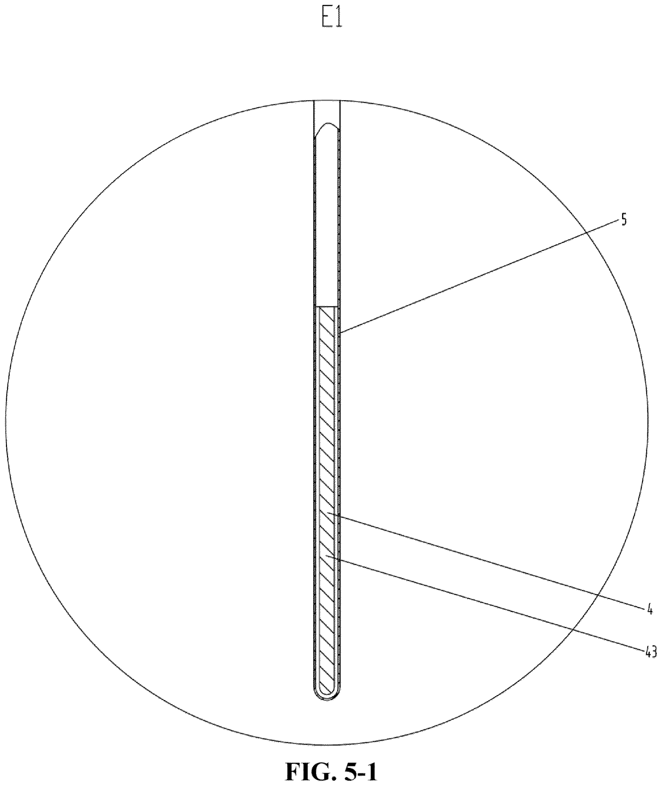

Referring to FIG. 1 to FIG. 13, a medical optical tracking system in this embodiment includes a light source 1 and an optical tracking carrier 2.

The optical tracking carrier 2 includes a light guide material. Light emitted by the light source 1 is conducted through the optical tracking carrier 2, and optical tracking is performed on the optical tracking carrier 2.

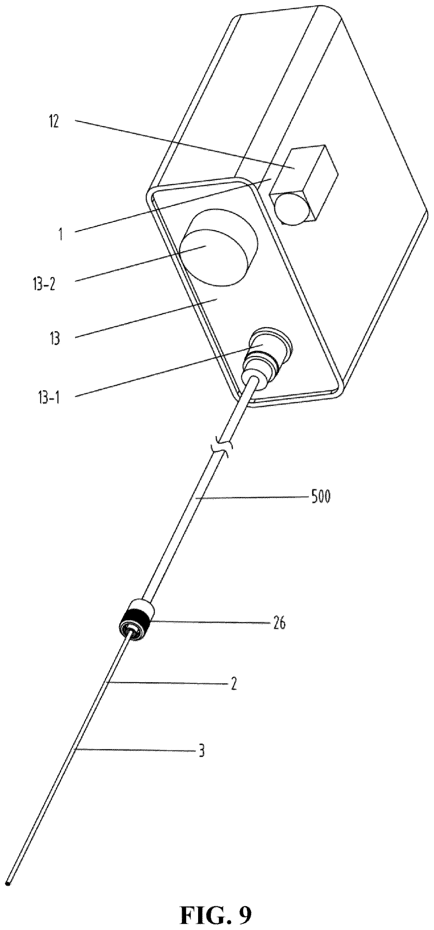

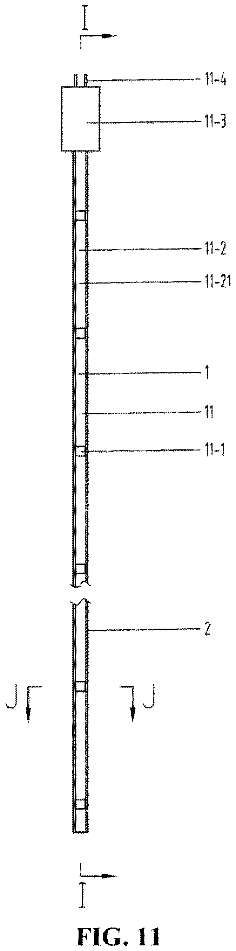

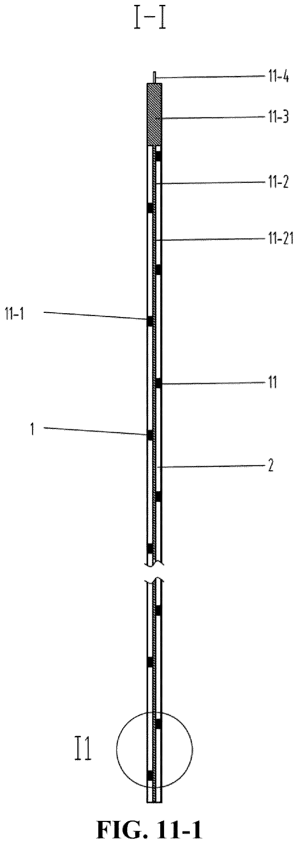

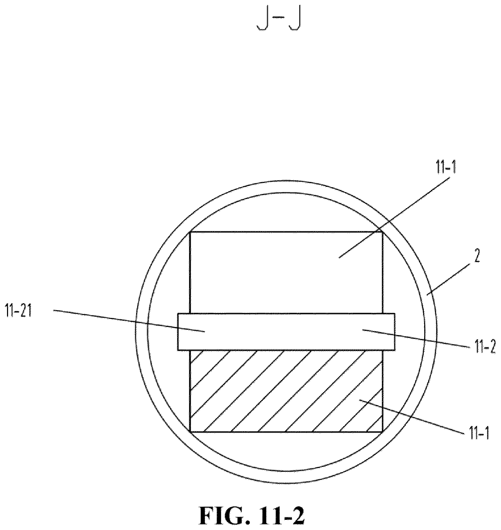

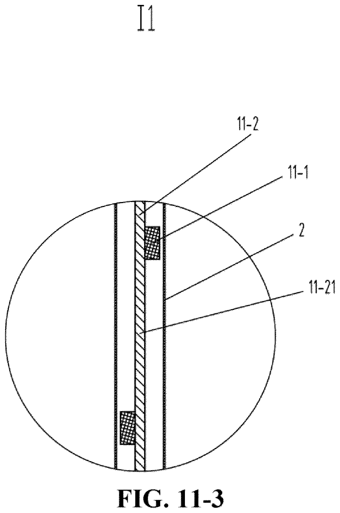

The light source 1 is an LED light source 11, and/or a medical cold light source 12, and/or natural light. The light source 1 may be one of various types of light sources that can emit light, and the light emitted by the light source 1 may be conducted through the optical tracking carrier 2 to perform tracking. Referring to FIG. 11, compared with a conventional lighting source, the LED light source 11 has characteristics such as a small volume, high light emission efficiency, and strong light source directivity, and especially in terms of safety, the LED light source has an unrivalled advantage over a conventional light source. First, the LED light source uses low-voltage direct-current power supply, and a power supply voltage only needs to be 6 V to 24 V. Next, no mercury is added to the LED light source, and a human body is kept from poisoning or other damage. Furthermore, more importantly, the LED light source is a cold light source, and no severe heat generation occurs in a working process, which allows safe touch, and causes no accidental high-temperature scalding to a human body. The medical cold light source 12 is a common light source during existing surgery. In addition, the light source 1 may be placed in the back and is readily available in a surgery, and no additional equipment is required. In addition, the medical cold light source 12 does not easily cause a rise in an in-vivo temperature, referring to FIG. 9.

A color of the light emitted by the light source 1 may be set according to a background color or a transmission requirement.

Through the setting of light, in clinical surgery, a doctor can directly see the position of the optical tracking carrier 2 through tissue with naked eyes to accurately discern a blood vessel, tissue or an organ that requires special protection in clinical surgery, to effectively avoid an accident during surgery. The light emitted by the light source 1 may be differently set according to a background color in a body cavity or tissue through which light needs to be transmitted. When light needs to be transmitted through tissue, red light and yellow light are preferred, and purple light and white light are less preferred, and when blood vessel tissue needs to be displayed, green light is preferred.

The light source 1 may be set as required to a manner of being intermittently lit, a flashing manner, or another form. The intensity of the light emitted by the light source 1 may be adjusted as required, to adapt to different clinical environments. The illuminance of the light emitted by the light source 1 may be up to 300,000 lux, and preferably ranges from 5,000 lux to 150,000 lux.

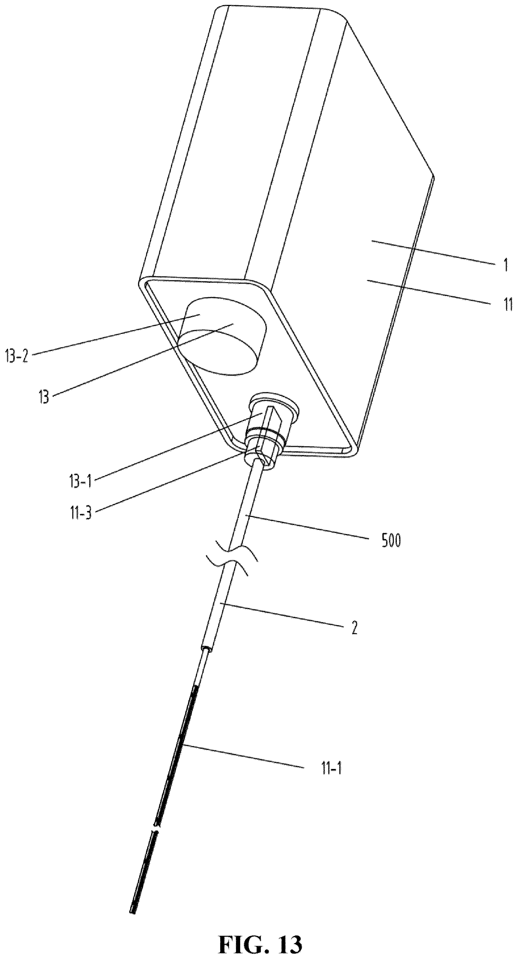

Referring to FIG. 9 and FIG. 13, the light source 1 includes a control system 13. The control system 13 includes a wavelength adjustment mechanism 13-1 and a light intensity adjustment mechanism 13-2. The wavelength adjustment mechanism 13-1 can adjust a wavelength to adjust the color of the emitted light, and the light intensity adjustment mechanism 13-2 can adjust the illuminance of the emitted light.

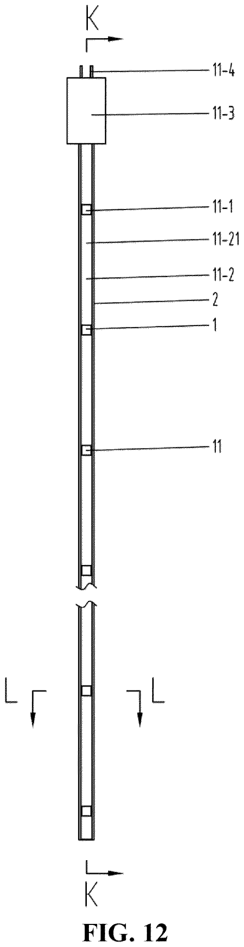

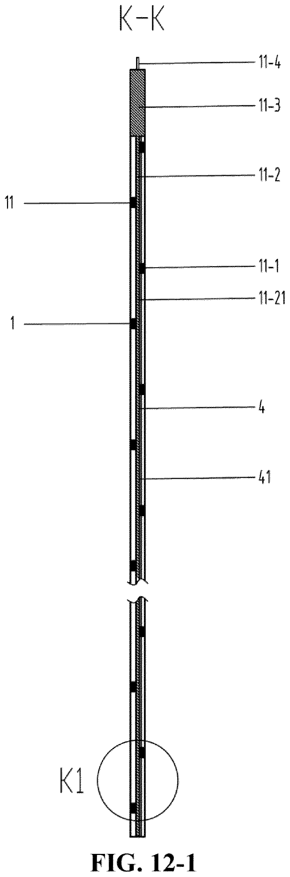

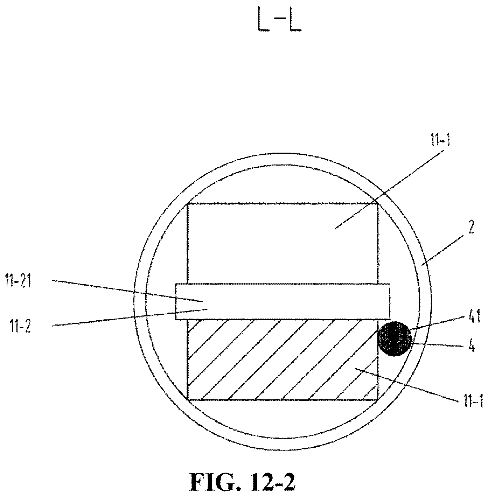

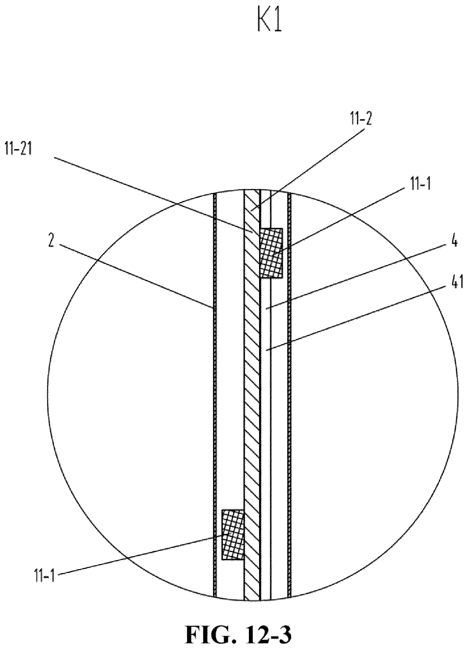

The LED light source 11 may be disposed in vivo or may be disposed in vitro. The volume of the light-emitting end 11-1 of the LED light source 11 may be very small. The size of the light-emitting end 11-1 is usually controlled below 2 mm. For example, an LED lamp with a size of 0.2 mm to 0.5 mm is packaged. Therefore, the LED light source 11 can be disposed in vitro to conduct light into a human body through the optical tracking carrier 2, and can be directly disposed in a human body, wrapped with the optical tracking carrier 2 outside, and directly disposed at a position that requires tracking, referring to FIG. 11 to FIG.12-3.

In this embodiment, the light source 1 may be connected to the optical tracking carrier 2 by a light guide joint 26 to provide a light source, or the LED light source 11 is disposed in the optical tracking carrier 2. The optical tracking carrier 2 is directly covered outside the LED light source 11, and the LED light source 11 and the optical tracking carrier 2 are placed together in a human body, to track a cavity or tube, an organ, a tumor, or the like.

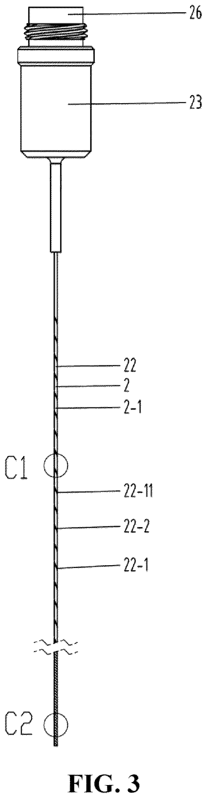

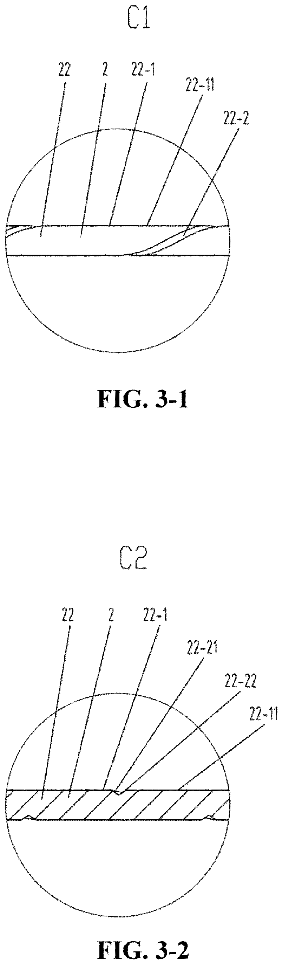

Referring to FIG. 1 to FIG. 3-2, the optical tracking carrier 2 is a light guide optical fiber 22. The light guide optical fiber 22 has a good light guide effect, conducts light to various different positions as required, and may be connected or disconnected as required, so that clinical use is very convenient.

The optical tracking carrier 2 includes at least one light guide optical fiber 22.

Both an end portion and/or a side surface of the light guide optical fiber 22 can emit light. The end portion of the light guide optical fiber 22 can emit light, and the side surface can also emit light. The light guide optical fiber 22 can be completely lit to emit light.

The optical tracking carrier 2 is a combination of a plurality of light guide optical fibers 22.

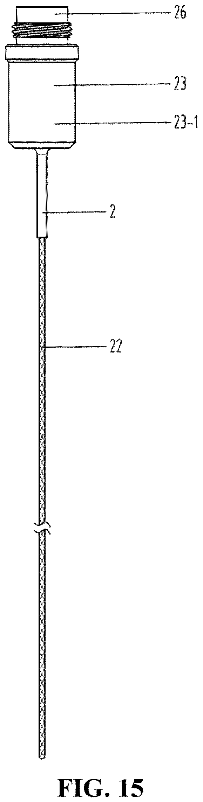

The optical tracking carrier 2 may be formed by a single light guide optical fiber 22 (referring to FIG. 1, FIG. 2, and FIG. 3), or may be a combination of a plurality of light guide optical fibers 22, for example, in one of various manners of forming an optical fiber bundle, being plaited in a mesh form, being arranged at different lengths, and the like, referring to FIG. 15.

The light guide optical fiber 22 has a smooth surface. When the light guide optical fiber 22 has a smooth surface, because the light guide optical fiber 22 has good light guide performance, light outlets 22-2 of the light guide optical fiber 22 are located at a distal end of the light guide optical fiber 22, so that targeted tracking can be implemented.

The light guide optical fiber 22 has a non-smooth surface 22-1.

The non-smooth surface 22-1 is a non-smooth surface 22-11 that can form reflection and/or scattering. Through the reflection and/or scattering of light by the non-smooth surface 22-1, the overall light emission of the non-smooth surface 22-1 can be implemented, to achieve the effect of overall tracking.

The light outlets 22-2 are intermittently provided in the light guide optical fiber 22. Each of the intermittently provided light outlets 22-2 has a conduction surface 22-21 and a reflection surface 22-22 of light. When light is conducted through the conduction surface 22-21 to reach the reflection surface 22-22, the light is reflected and emitted out of the light outlet 22-2 to form one tracking point, and a plurality of light outlets 22-2 may form a chain tracking belt, referring to FIG. 1.

The light outlets 22-2 are non-axial light outlets 22-21, provided in a side surface of the light guide optical fiber 22 in a length direction of the light guide optical fiber 22. When the light outlets 22-2 are completely provided in the length direction of the light guide optical fiber 22, the light guide optical fiber 22 may be completely lit in the length direction of the light guide optical fiber 22, to implement the overall tracking of the light guide optical fiber 22.

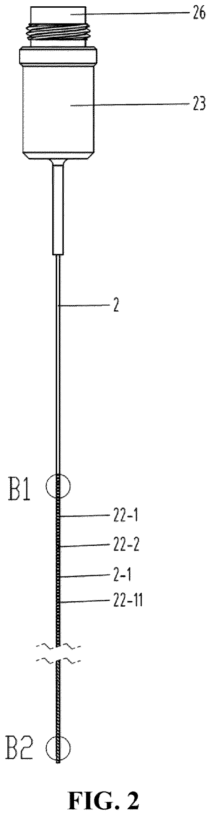

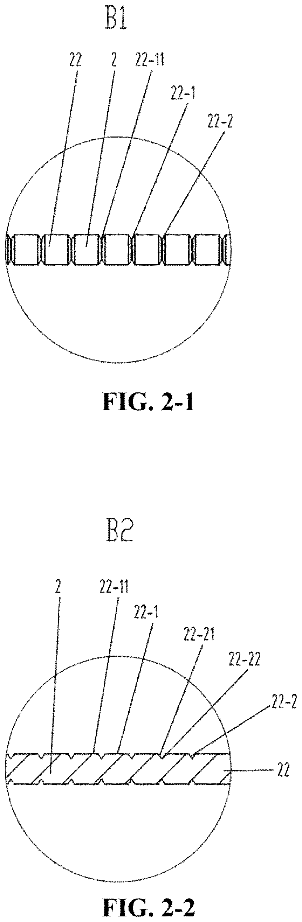

The non-axial light outlets 22-21 may be dotted intermittent light outlets manufactured in a molding manner, referring to FIG. 1 to FIG. 1-4, or may be annular light outlets or spiral light outlets obtained through overall injection molding or wire cutting, referring to FIG. 2 to FIG. 3-2.

The light outlets 22-2 may identify a length of the light guide optical fiber 22 through regular arrangement of the light outlets 22-2.

Intensities of scattered light are different through regular arrangement of the light outlets 22-2 in distribution density to identify the length of the light guide optical fiber 22.

When the light outlets 22-2 are densely arranged, emitted light is stronger, and the visual effect is brighter. When the light outlets 22-2 are dispersedly arranged, emitted light is weaker, and the visual effect is relatively dark. Through the arrangement of the bright and dark effects, a visual effect similar to a ruler may be formed, so that while tracking is performed, size identification can also be implemented, referring to FIG. 1.

Referring to the figure 15, the light guide optical fiber 22 may be plaited in a mesh form, and the light outlets 22-2 are scatteredly distributed at different positions. The light guide optical fiber 22 is plaited in a mesh form. The length of each light guide optical fiber 22 may be set to be different, and the light outlets 22-2 of the light guide optical fiber 22 are different accordingly, and are scatteredly distributed, so that overall tracking in a three-dimensional space can be implemented. Because the light outlets 22-2 do not need to be provided in the middle of each light guide optical fiber 22, light conduction is better, and the visual effect of a single tracking point is very bright. The light outlets 22-2 may also be provided in the side surface, and the light guide optical fiber 22 that can be completely lit is plaited in a mesh form, to implement full-area tracking of an entire cavity. In addition, the shape of plaiting in a mesh form may have good support, and is especially suitable for support and tracking of a large cavity such as a bladder, a uterus, or the like.

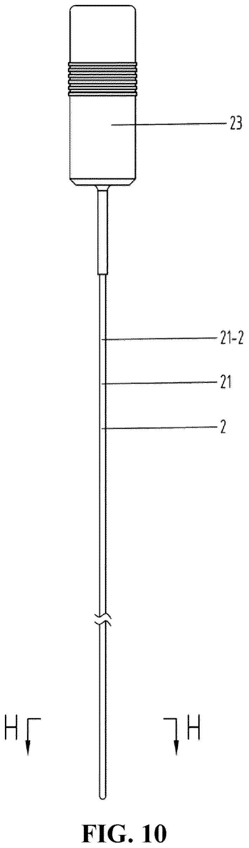

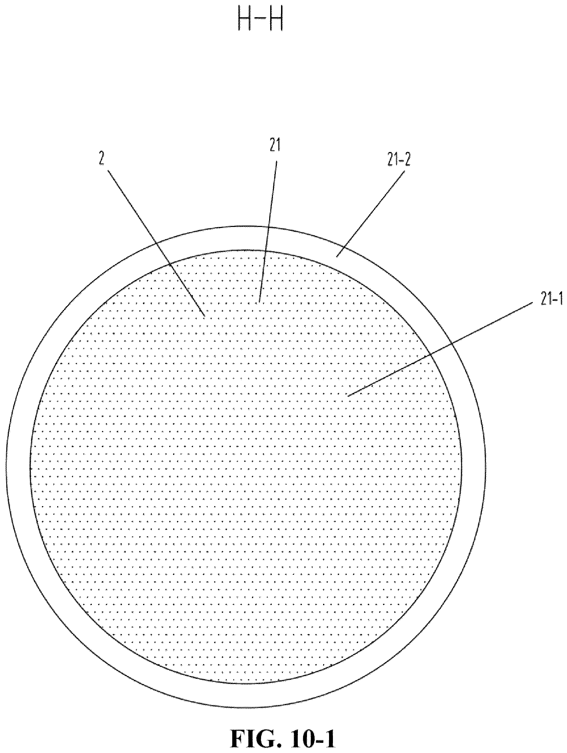

Referring to FIG. 10 and FIG. 10-1, the light source may be connected to the optical tracking carrier 2 in a non-contact manner. The optical tracking carrier 2, for example, a light-retaining self-luminous tracking carrier 21, may be illuminated, and the tracking of the optical tracking carrier 2 is implemented through the storage and conversion of light energy. The optical tracking carrier 2 is a light-retaining self-luminous tracking carrier 21. A self-luminous material is a substance that can absorb energy in a manner and convert the energy into nonequilibrium optical radiation, and a process in which the energy absorbed inside the material is converted into nonequilibrium optical radiation is a light emission process. Especially, a light-retaining self-luminous material can continuously emit light for over 12 hours in a dark environment after being irradiated by external light for several minutes or tens of minutes, and can meet tracking requirements of most surgical durations. The light-retaining self-luminous tracking carrier 21 may directly absorb energy of light in a surgery, so that a variety of external light can form the light source 1, and the light source 1 does not need to be directly connected, so that a use process is very simple.

The light-retaining self-luminous tracking carrier 21 usually includes a light-retaining self-luminous body 21-1 and a protective carrier 21-2. The protective carrier 21-2 is made of a transparent light guide material, and the light-retaining self-luminous body 21-1 is sealed in the protective carrier 21-2.

The light-retaining self-luminous body 21-1 can absorb external energy, and perform conversion to emit light. The protective carrier 21-2 is made of a transparent medical material, and may directly contact tissue, so that while light emitted through energy conversion of the light-retaining self-luminous body 21-1 can be effectively transmitted to perform effective tracking, biosafety in clinical use is ensured. The light-retaining self-luminous body 21-1 can be disposed at different positions and designed into different shapes, and perform targeted tracking or perform overall tracking as required.

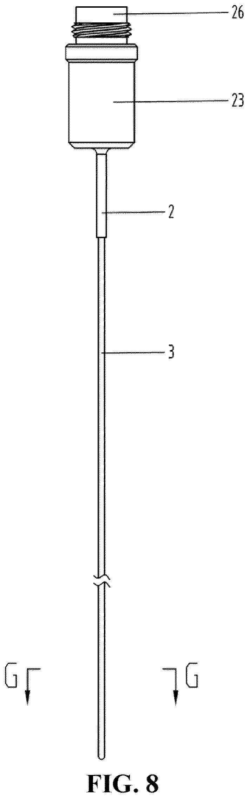

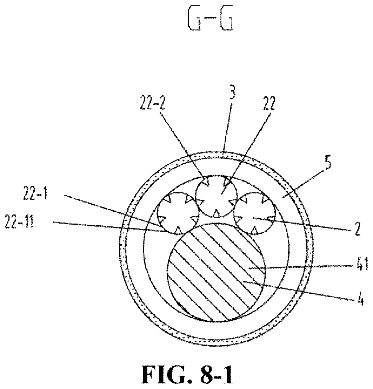

Referring to FIG. 8 and FIG. 8-1, a surface of the medical optical tracking system 500 further includes a coating 3.

The coating 3 is an anticoagulant coating, and/or a hydrophilic coating, and/or a hydrophobic coating.

The coating 3 may be designed to be a coating having a different property as required. For example, when the optical tracking carrier 2 needs to enter a blood vessel, the coating 3 may be designed into an anticoagulant coating. When the optical tracking carrier 2 needs to enter a variety of cavities, the coating 3 may be designed into a hydrophilic coating or a hydrophobic coating as required.

Referring to FIG. 1 and FIG. 15, the optical tracking carrier 2 includes a delivery portion 23. The delivery portion 23 can deliver a working portion 2-1 of the optical fiber tracking carrier 2 as required to a position that requires tracking, for example, a blood vessel, a cavity, or a tumor surgery position. The delivery portion 23 includes a delivery handle 23-1.

Referring to FIG. 37 and FIG. 37-1, the delivery portion 23 is movably disposed on the optical tracking carrier 2. During clinical application, the delivery portion 23 may be removed from the optical tracking carrier 2 or move relative to the optical tracking carrier 2 as required.

The medical optical tracking system 500 further includes a development mechanism 4.

The development mechanism 4 is made of metal, and has a heat conduction function. The heat conduction function of the development mechanism 4 can prevent an accident caused by a part of the medical optical tracking system 500 entering a human body having an excessively high temperature, and the temperature is usually controlled below 37°C.

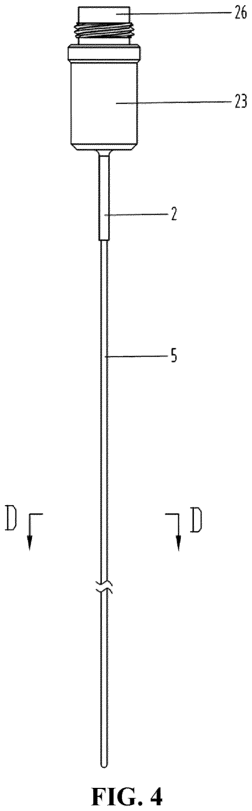

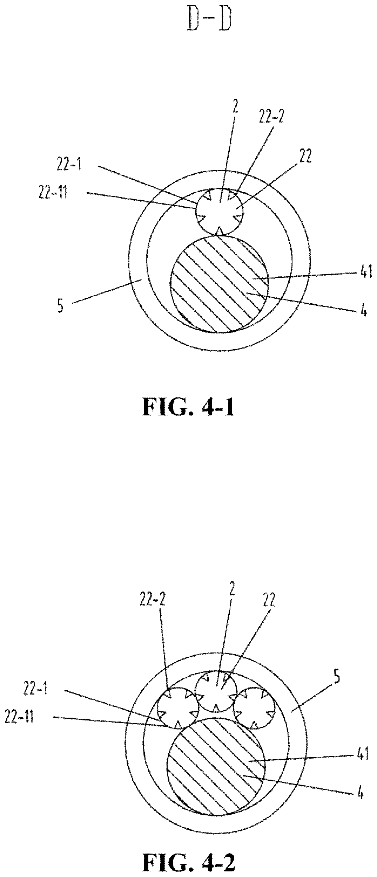

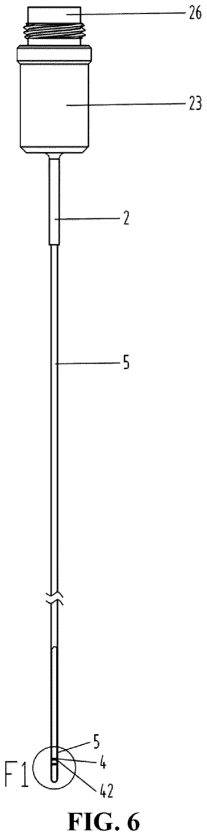

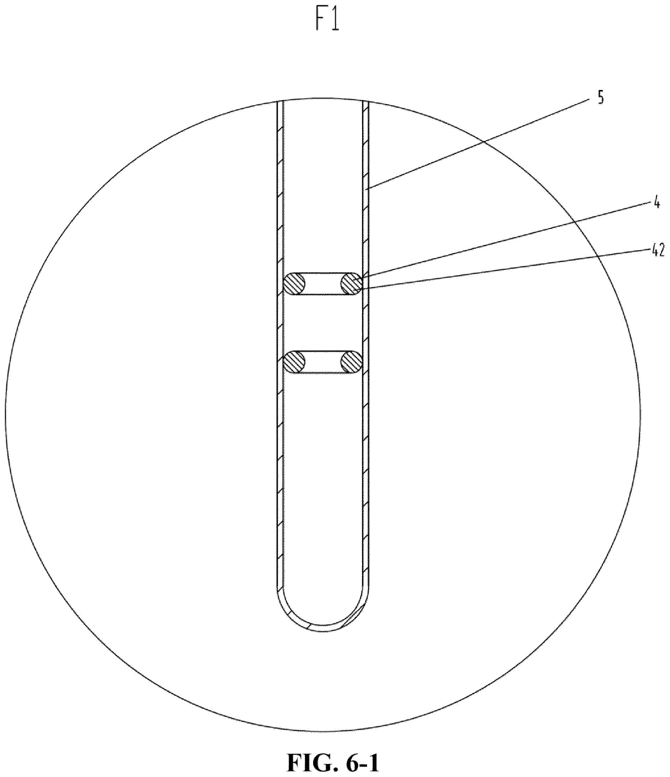

Referring to FIG. 4 to FIG. 6-1, the development mechanism 4 is a development string 41, and/or a development ring 42, and/or a development block 43. The applicant only illustrates the foregoing several development manners. During actual application, a person skilled in the art may design different development manners as required. The development manners are not illustrated herein one by one by the applicant, but none of the development manners departs from the protection scope of this application.

The development mechanism 4 may perform development under X-rays, and/or in MRI, and/or in B-mode ultrasound. The development mechanism 4 can perform a development prompt in X-rays, magnetic navigation, B-mode ultrasound, or another scenario, and the development mechanism 4 facilitates the placement of the optical tracking carrier 2 in a visible or navigation case, and is especially suitable for placement in an important blood vessel, a solid tumor, or the like.

Referring to FIG. 4 to FIG. 4-2, the medical optical tracking system 500 further includes a protection sleeve 5. The optical tracking carrier 2, the development mechanism 4, and the like may be disposed in the protection sleeve 5. The coating 3 may be disposed outside the protection sleeve 5 as required.

The protection sleeve 5 is made of a transparent material, and the optical tracking carrier 2 is disposed in the protection sleeve 5. The protection sleeve 5 is made of a medical transparent material, so that while the optical tracking carrier 2 is protected, the transparent material can still ensure the tracking of the optical tracking carrier 2.

Referring to FIG. 1-4, the optical tracking carrier 2 includes a channel 24 inside. The channel 24 may be used as a surgical instrument channel, a body fluid drainage channel, or the like as required.

The optical tracking carrier 2 is made of a flexible medical material, and is movable along a blood vessel, a ureter, an esophagus, a trachea, an oviduct, a vas deferens, or another cavity. The optical tracking carrier 2 has good flexibility, and is movable along a blood vessel or a cavity. Therefore, the optical tracking carrier 2 may be placed at different positions as required.

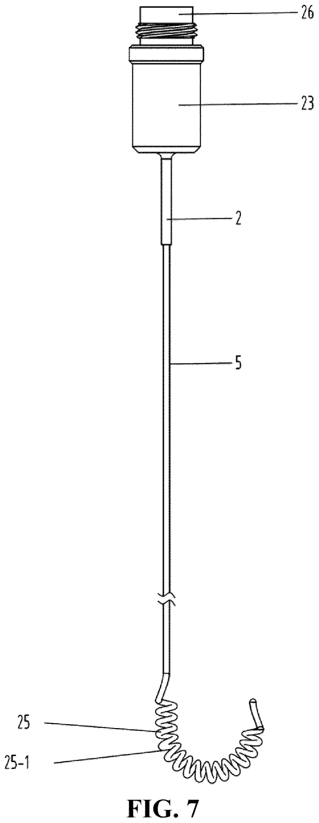

Referring to FIG. 7, the optical tracking carrier 2 includes a shaping mechanism 25. The shaping mechanism 25 may shape the optical tracking carrier 2, for example, shape the optical tracking carrier into one of a variety of different shapes such as a circular shape, an arc shape, a spherical shape, and the like as required, to adapt to different tracking requirements.

The shaping mechanism 25 is a shape-memory shaping mechanism 25-1. The shape-memory shaping mechanism 25-1 has a string shape, a strip shape, or another simple structure at room temperature, so that after being placed in a human body, the shape-memory shaping mechanism restores the set shape under the action of body temperature.

The shape-memory shaping mechanism 25-1 is manufactured by plaiting a shape-memory metal wire, and/or is manufactured by carving a shape-memory metal tube or sheet. The shape-memory shaping mechanism 25-1 may be formed by plaiting a shape-memory metal wire into a required shape, or may be formed by directly carving a shape-memory alloy tube or a shape-memory alloy sheet into a required shape.

During clinical application, the optical tracking carrier 2 of the medical optical tracking system in this embodiment may be placed as required at a variety of positions that require tracking, for example, a ureter, a vas deferens, an oviduct, or another cavity or tube, or may be placed in a uterine fibroid, a lung tumor (especially a pulmonary nodule), a liver tumor, or another solid tumor, or may be placed in a blood vessel, and especially a cavity or tube, a blood vessel, a solid tumor, or the like is identified by using the characteristic that the side surface of the light guide optical fiber 22 can emit light to implement overall tracking.

For the medical optical tracking system in this embodiment, after the light emitted by the light source 1 is conducted through the optical tracking carrier 2, optical tracking may be performed on the optical tracking carrier 2. Light sources of different colors are disposed, so that the optical tracking carrier 2 can accurately identify a blood vessel, a cavity or tube, tissue or an organ that requires special protection in clinical surgery, to effectively avoid an accident during surgery, or effectively calibrate a small size tumor, thereby facilitating effective implementation of clinical surgery.

### Embodiment 2: Blood vessel tracking apparatus of the present invention

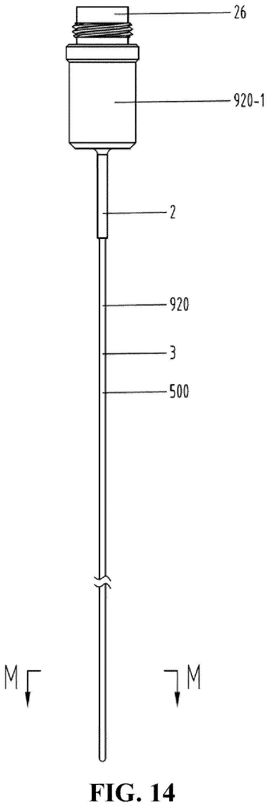

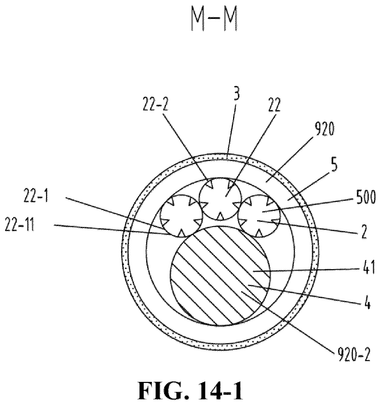

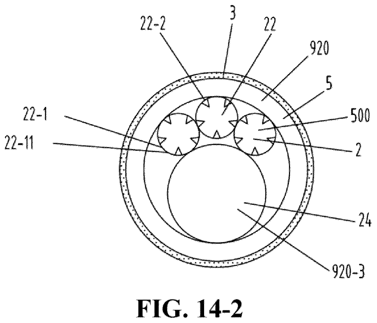

Referring to FIG. 14, this embodiment discloses a blood vessel visible light calibration technique and apparatus. The blood vessel tracking apparatus in this embodiment includes the medical optical tracking system 500 in Embodiment 1.

Referring to FIG. 14 and FIG. 14-1, the blood vessel tracking apparatus 920 includes a delivery end 920-1, a guide wire 920-2, a protection sleeve 5, and the medical optical tracking system 500.

In this embodiment, the light source 1 of the medical optical tracking system 500 is a medical cold light source 12, the medical cold light source 12 can better prevent a rise in a temperature of the optical tracking carrier 2 disposed in vivo, and a blood vessel tracking process is safer.

The light source 1 includes a control system 13. The control system 13 includes a wavelength adjustment mechanism 13-1 and a light intensity adjustment mechanism 13-2. The wavelength adjustment mechanism 13-1 can adjust a wavelength to adjust the color of the emitted light, and the light intensity adjustment mechanism 13-2 can adjust the illuminance of the emitted light.

A color of the light emitted by the light source 1 may be set according to a background color or a transmission requirement. Through the setting of light, in clinical surgery, a doctor can directly see the position of the optical tracking carrier 2 through tissue with naked eyes to accurately discern a blood vessel that requires special protection in clinical surgery, to effectively avoid an accident during surgery. The light emitted by the light source 1 may be differently set according to a background color in a body cavity or tissue through which light needs to be transmitted. When light needs to be transmitted through tissue, red light and yellow light are preferred, and purple light and white light are less preferred, and when blood vessel tissue needs to be displayed, green light is preferred.

The light source 1 may be set as required to a manner of being intermittently lit, a flashing manner, or another form. The intensity of the light emitted by the light source 1 may be adjusted as required, to adapt to different clinical environments. The illuminance of the light emitted by the light source 1 may be up to 300,000 lux, and preferably ranges from 5,000 lux to 150,000 lux.

Referring to FIG. 14-1, in this embodiment, the medical optical tracking carrier 2 is a combination of a plurality of light guide optical fibers 22. The light guide optical fiber 22 has a non-smooth surface 22-1, light outlets 22-2 are provided in a side surface in a length direction, and a proximal end of the light guide optical fiber 22 is connected to a light guide joint 26. Under the action of the light source 1, the side surface of the light guide optical fiber 22 can emit scattered light, to implement the tracking of the light guide optical fiber 22 in the length direction.

The protection sleeve 5 is made of a transparent material, and the light guide optical fiber 22 and the guide wire 920-2 are disposed in the protection sleeve 5. A coating 3 is provided outside the protection sleeve 5, and the coating 3 is an anticoagulant coating.

The guide wire 920-2 is made of a metal material, and can perform development under X-rays, to form a development mechanism 4, and also has a heat conduction function, to prevent an accident caused by a part of the working portion 2-1 of the light guide optical fiber 22 entering a human body having an excessively high temperature, and the temperature is usually controlled below 37°C.

Referring to FIG. 7, the guide wire 920-2 may be made of a shape-memory alloy, and a distal end of the blood vessel tracking apparatus 920 is shaped, to form a shaping mechanism 25. The guide wire 920-2 may shape the distal end of the blood vessel tracking apparatus 920 as required to adapt to a radian of a blood vessel, making a delivery process more convenient. In a process of shaping the guide wire 920-2, the light guide optical fiber 22 changes accordingly, to ensure the tracking effect of the blood vessel tracking apparatus 920.

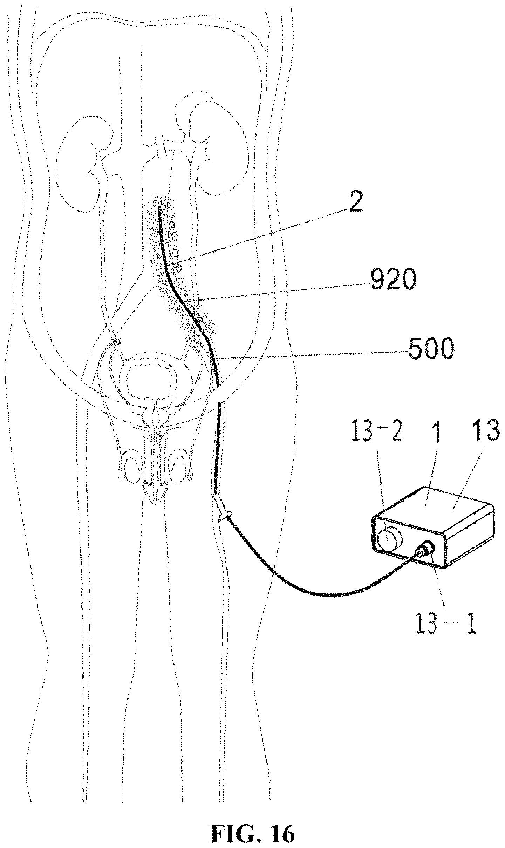

Referring to FIG. 16, during clinical use, a radial artery puncture technique is first performed, an artery sheath is fastened, and then a medical super smooth guide wire is delivered into an angiography catheter. The catheter is placed in the artery sheath, and then the super smooth guide wire is entered in an X-ray environment. After the guide wire is delivered to a working position, the guide wire is fastened, then the angiography catheter is delivered to the working position along the guide wire, and then the super smooth guide wire is removed. The blood vessel tracking apparatus in this embodiment is placed at the working position along the angiography catheter, and then the angiography catheter is removed. The blood vessel tracking apparatus 920 remains in a blood vessel that requires tracking. The light source 1 is turned on, and the blood vessel tracking apparatus 920 can track the blood vessel through a visible light technique. In surgery, when a blood vessel being tracked is approached, it can be seen through tissue that the light emitted by the light source 1 is transmitted through the blood vessel, and the illuminance of the light emitted by the light source 1 ensures that at a distance of about 0 mm to 30 mm from a blood vessel wall, the blood vessel being tracked is clearly visible.

Furthermore, the blood vessel tracking apparatus 920 may be designed into a structure without the guide wire 920-2.

Referring to FIG. 14-2, the medical optical tracking system 500 is provided with a channel 24, and the channel 24 forms a guide wire operation hole 920-3. In the guide wire operation hole 920-3, a separate medical guide wire may be inserted for cooperation to perform a delivery operation.

A side wall of the optical tracking carrier 2 is made of a transparent material, a light guide optical fiber 22 and a development string 41 are provided in the side wall, the light guide optical fiber 22 has a non-smooth surface 22-1, light outlets 22-2 are provided in a side surface in a length direction, a coating 3 is provided on an outer surface of the optical tracking carrier 2, and the coating 3 is an anticoagulant coating; a proximal end of the light guide optical fiber 22 is connected to a light guide joint 26, and under the action of the light emitted by the light source 1, the light guide optical fiber 22 may overall emit light, to track a blood vessel.

For the optical tracking carrier, apart from the foregoing design of the light guide optical fiber 22, light-emitting ends of the LED light source 11 may be directly placed in a human body to perform tracking.

Referring to FIG. 14-1, the optical tracking carrier 2 is made of a transparent material to form the protection sleeve 5 provided with the coating 3 outside, and the coating 3 is an anticoagulant coating.

The light source 1 is an LED light source 11. The LED light source 11 uses the flexible circuit board 11-21 as the circuit system 11-2 to be connected to a power supply. The light-emitting ends 11-1 of the LED light source 11 are dispersedly disposed on the flexible circuit board 11-21, and are then packaged together with the development mechanism 4 on the optical tracking carrier 2. The LED light source 11 directly tracks a blood vessel in the blood vessel, an illumination effect is better, and the tracking of a large blood vessel has a very good effect.

In this embodiment, the medical optical tracking system 500 may perform overall tracking on the blood vessel tracking apparatus 920. During clinical use, under the action of the medical optical tracking system 500, when a blood vessel being tracked is approached, it can be seen through tissue that the light emitted by the light source 1 is transmitted through the blood vessel, and the illuminance of the light emitted by the light source 1 ensures that at a distance of about 0 mm to 30 mm from a blood vessel wall, the blood vessel being tracked is clearly visible. The light is stronger at a position closer to the blood vessel, a prompt effect is clearer, so that the tracking of blood vessel can be effectively performed in surgery, and a medical person is prompted to protect important blood vessels, to avoid accidental injury of a blood vessel during surgery, making clinical use very convenient and safe.

### Embodiment 3: Medical catheter with a tracking apparatus of the present invention

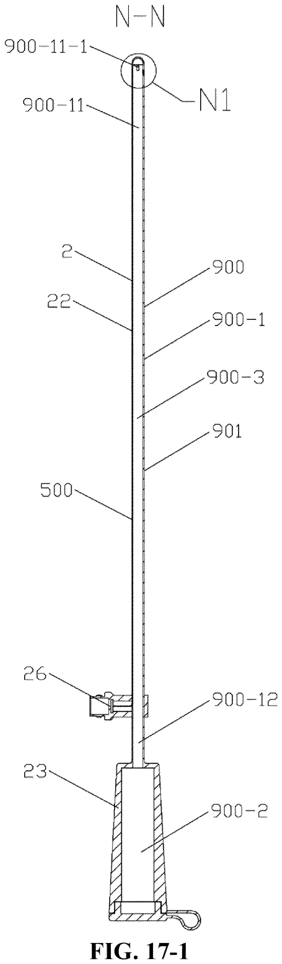

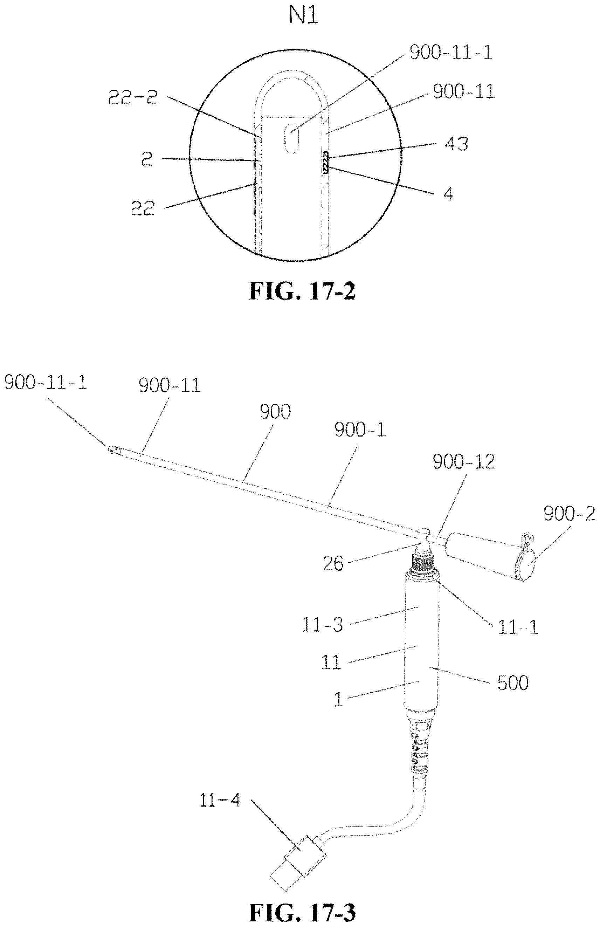

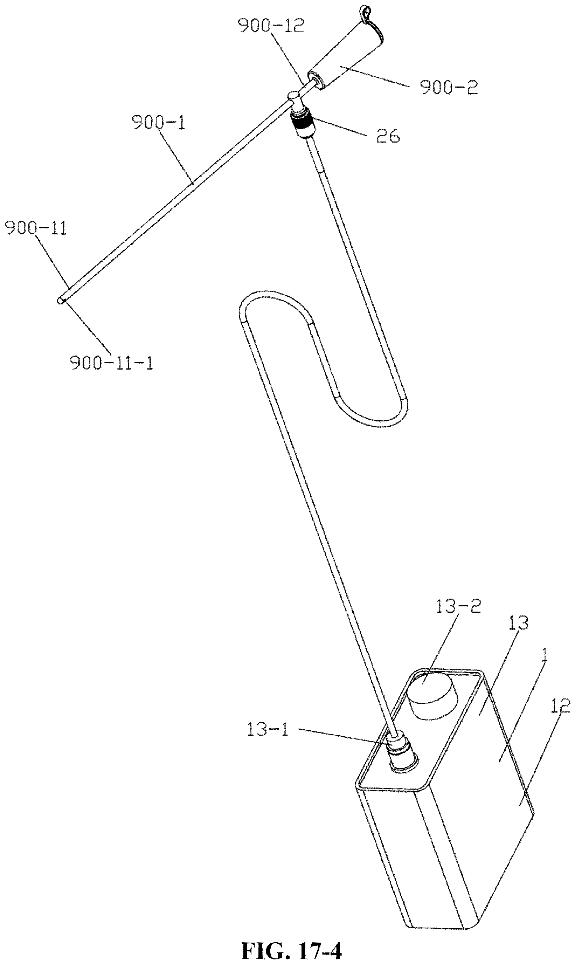

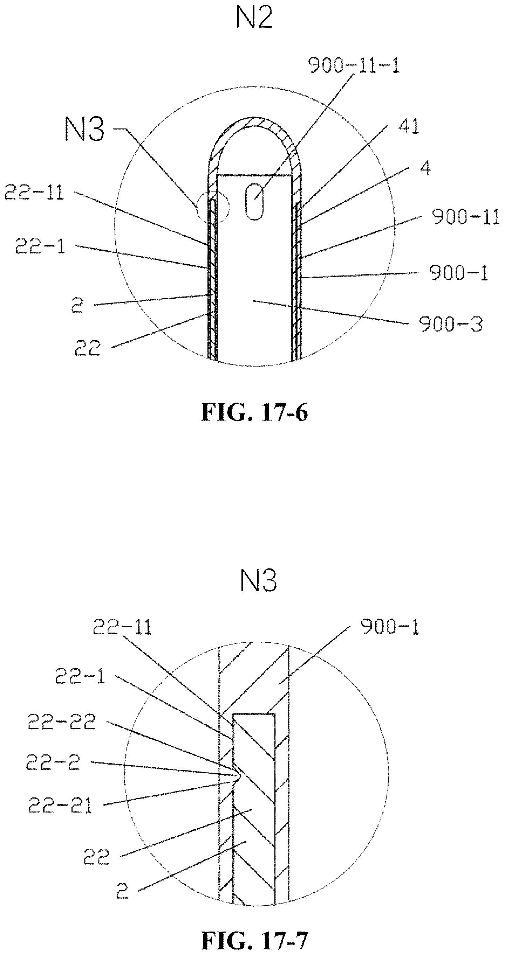

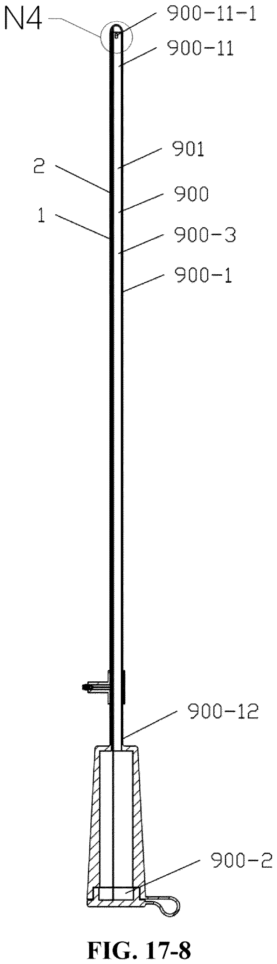

Referring to FIG. 17 to FIG. 24, this embodiment discloses a medical catheter visible light calibration technique and apparatus. The medical catheter with a tracking apparatus in this embodiment includes the medical optical tracking system in Embodiment 1.

Referring to FIG. 17 to FIG. 17-3, the medical catheter 900 with a tracking apparatus includes a catheter 900-1, an interface 900-2, and the medical optical tracking system 500.

The catheter 900-1 is made of a soft elastic medical material, and is provided with a working channel 900-3 inside, and a working opening 900-11-1 is provided at a distal end 900-11.

The medical catheter 900 with a tracking apparatus includes a development mechanism 4. The development mechanism 4 is disposed on the catheter 900-1. The development mechanism 4 can perform a development prompt in X-rays, magnetic navigation, B-mode ultrasound, or another scenario, to facilitate the placement of the catheter 900-1. The development mechanism 4 may be made of a metal material, and may also have a heat conduction function. To keep the LED light source 11 from causing an excessively high temperature in a body, the temperature usually needs to be controlled within 37°C.

The medical catheter 900 with a tracking apparatus includes at least one interface 900-2, and the interface 900-2 is disposed at a proximal end 900-12 of the catheter 900-1.

The optical tracking carrier 2 of the medical optical tracking system 500, disposed on the catheter 900-1, and identifying a position of the catheter 900-1.

The optical tracking carrier 2 is provided on the catheter 900-1, and the optical tracking carrier 2 may perform local or overall tracking on the catheter 900-1 as required.

In this embodiment, the light source 1 is an LED light source 11, and includes light-emitting ends 11-1 and a circuit system 11-2. A color of the light emitted by the light source 1 may be set according to a background color or a transmission requirement.

Referring to FIG. 17-4, the light source 1 includes a control system 13. The control system 13 includes a wavelength adjustment mechanism 13-1 and a light intensity adjustment mechanism 13-2. The wavelength adjustment mechanism 13-1 can adjust a wavelength to adjust the color of the emitted light, and the light intensity adjustment mechanism 13-2 can adjust the illuminance of the emitted light.

Through the setting of light, in clinical surgery, a doctor can directly see the position of the optical tracking carrier 2 through tissue with naked eyes to accurately discern a blood vessel, tissue or an organ that requires special protection in clinical surgery, to effectively avoid an accident during surgery. The light emitted by the light source 1 may be differently set according to a background color in a body cavity or tissue through which light needs to be transmitted. When light needs to be transmitted through tissue, red light and yellow light are preferred, and purple light and white light are less preferred, and when blood vessel tissue needs to be displayed, green light is preferred.

The light source 1 may be set as required to a manner of being intermittently lit, a flashing manner, or another form. The intensity of the light emitted by the light source 1 may be adjusted as required, to adapt to different clinical environments. The illuminance of the light emitted by the light source 1 usually ranges from 5,000 lux to 150,000 lux.

The LED light source 11 may be disposed in vitro, and is connected to the optical tracking carrier 2 by a light guide joint 26, to track the optical tracking carrier 2. The LED light source 11 may use a micro light-emitting end design, and the light-emitting ends 11-1 are disposed on the catheter 900-1 and enter a human body along with the catheter 900-1 to perform tracking.

When the micro light-emitting end design is used, the size of the light-emitting end 11-1 is usually controlled below 2 mm. For example, an LED lamp with a size of 0.2 mm to 0.5 mm is packaged. The catheter 900-1 is made of a transparent material, and forms the optical tracking carrier 2. The light source 1 of the medical optical tracking system 500 is an LED light source 11, and light-emitting ends 11-1 of the LED light source 11 are disposed in a tube wall of the catheter 900-1, and track the catheter 900-1. The LED light source 11 includes the light-emitting ends 11-1 each having a very small volume, so that the light-emitting ends 11-1 may be directly disposed in the tube wall of the catheter 900-1, is connected to a power supply by a circuit system 11-2, and emits light in the tube wall of the catheter 900-1.

The light-emitting ends 11-1 may be disposed at any positions of the catheter 900-1 as required, to implement local or overall tracking of the catheter 900-1.

Referring to FIG. 17-8 and FIG. 17-9, when the light-emitting ends 11-1 of the LED light source 11 are disposed at the distal end 900-11 of the catheter 900-1, and local targeted tracking may be performed on the distal end 900-11 of the catheter 900-1.

Referring to FIG. 17-11 and FIG. 17-12, when the flexible circuit board 11-21 is used for the circuit system 11-2, the light-emitting ends 11-1 may be dispersedly disposed at any positions of the flexible circuit board 11-21. The flexible circuit board 11-21 is disposed in the tube wall of the catheter 1, so that an LED lamp strip or an LED lamp net may be formed, to perform overall tracking on the catheter 900-1.

Referring to FIG. 17-1 to FIG. 17-7, when the LED light source 11 is disposed in vitro, the optical tracking carrier 2 of the medical optical tracking system 500 is a light guide optical fiber 22; and a proximal end of the light guide optical fiber 22 is connected to a light guide joint 26, and the light guide optical fiber 22 is disposed in a length direction of the catheter 900-1, to track the catheter 900-1.

Referring to FIG. 17-1 and FIG. 17-2, when the light guide optical fiber 22 has a smooth surface, light outlets 22-2 are provided at a distal end of the catheter 900-1, to track the distal end 900-11 of the catheter 900-1.

Referring to FIG. 17-5 to FIG. 17-7, when the light guide optical fiber 22 has a non-smooth surface 22-1, and light outlets 22-2 are provided in a side surface, so that scattered light can be emitted, to perform overall tracking on the catheter 900-1.

Referring to FIG. 20 to FIG. 21-1, for the light guide optical fiber 22, the light guide optical fiber 22 can be plaited in a mesh form to perform overall tracking on the catheter 900-1, and through the plaiting in a mesh form, the elastic support force of the light guide optical fiber 22 can be used to implement tube wall support for the catheter 900-1.

Referring to FIG. 17-13, a coating 3, for example, a hydrophilic coating, may be disposed on an outer surface of the catheter 900-1 as required.

The medical catheter 900 with a tracking apparatus may be a medical stomach tube 901, or a medical urinary catheter 902, or a medical vas deferens, or a medical oviduct, or a medical trachea. The applicant only lists the foregoing several use scenarios of the medical catheter 900 with a tracking apparatus herein. During actual application, the medical catheter 900 with a tracking apparatus may be disposed according to clinical requirements into structures required for different application scenarios.

Referring to FIG. 22, the medical catheter 900 may be further provided with a shaping mechanism 25. The shaping mechanism 25 may shape the catheter 900-1 to adapt to different cavities or tubes.

Referring to FIG. 17 to FIG. 17-15, the medical catheter 900 with a tracking apparatus is a medical stomach tube 901.

The medical stomach tube 901 includes the catheter 900-1, one interface 900-2, and the medical optical tracking system 500, and the distal end 900-11 of the catheter 900-1 is provided with the working opening 900-11-1; and the medical optical tracking system 500 tracks the catheter 900-1.

The medical stomach tube 901 enters the stomach through an esophagus, and is an artificial conveying channel of food. The interface 900-2 may be externally connected to a food injection apparatus. In clinical surgery, it may be only necessary to know the position of the distal end 900-11 of the catheter 900-1 for observation. In this case, the medical optical tracking system 500 only needs to perform local tracking on the distal end of the medical stomach tube 901. In some other cases, for example, surgery along an esophagus, to keep the esophagus from accidental injury, in this case, overall tracking needs to be performed on the medical stomach tube 901.

Referring to FIG. 17-8 and FIG. 17-9, when distal-end local tracking needs to be performed on the medical stomach tube 901, a technical solution in which the light-emitting ends 11-1 of the LED light source 11 are individually or centrally disposed at the distal end of the catheter 900-1 to perform tracking may be used, or the light guide optical fiber 22 having a smooth surface may be used. A single light guide optical fiber 22 or a light guide bundle formed by a plurality of light guide optical fibers 22 may be used, and the light outlets 22-2 at an end portion are used to track the distal end of the catheter 900-1, or the light guide optical fiber 22 is circumferentially arranged along a side wall of the catheter 900-1 to form a light ring at the distal end of the catheter 900-1 to perform local tracking on the distal end of the catheter 900-1.

Referring to FIG. 17-11 and FIG. 17-12, when overall or large-range tracking needs to be performed on the medical stomach tube 901, the light-emitting ends 11-1 of the LED light source 11 may be dispersedly distributed in the tube wall of the catheter 900-1 to form a light guide belt or a light guide net to perform overall or large-range tracking on the catheter 900-1. Alternatively, the light guide optical fiber 22 having the non-smooth surface 22-1 may be disposed in the tube wall of the catheter 900-1 in the length direction to form a light guide belt to track the catheter 900-1 in the length direction, or the light guide optical fiber 22 may be plaited in a mesh form, to perform a ring-shaped overall tracking on a section or a whole of the catheter 900-1.

The medical catheter 900 with a tracking apparatus is a medical urinary catheter 902.

The medical urinary catheter 902 includes the catheter 900-1, the interface 900-2, and the medical optical tracking system 500, and the distal end 900-11 of the catheter 900-1 is provided with the working opening 900-11-1; and the medical optical tracking system 500 tracks the catheter 900-1.

The medical optical tracking system 500 may perform targeted tracking on the medical urinary catheter 902, or may perform large-range or overall tracking on the medical urinary catheter 902.

When targeted or small-range local tracking needs to be performed on a working end of the medical urinary catheter 902, a technical solution in which the light-emitting ends 11-1 of the LED light source 11 are individually or centrally disposed at the distal end of the catheter 900-1 to perform tracking may be used, or the light guide optical fiber 22 having a smooth surface may be used. A single light guide optical fiber 22 or a light guide bundle formed by a plurality of light guide optical fibers 22 may be used, and the light outlets 22-2 at an end portion are used to track the distal end of the catheter 900-1, or the light guide optical fiber 22 is circumferentially arranged along a side wall of the catheter 900-1 to form a light ring at the distal end of the catheter 900-1 to perform local tracking on the distal end of the catheter 900-1.

For example, when regional tracking needs to be performed on a bladder or overall tracking needs to be performed on the catheter 900-1, the light-emitting ends 11-1 of the LED light source 11 may be dispersedly distributed in the tube wall of the catheter 900-1 to form a light guide belt or a light guide net to perform overall or large-range tracking on the catheter 900-1. Alternatively, the light guide optical fiber 22 having the non-smooth surface 22-1 may be disposed in the tube wall of the catheter 900-1 in the length direction to form a light guide belt to track the catheter 900-1 in the length direction, or the light guide optical fiber 22 may be plaited in a mesh form, to perform overall tracking on a periphery of the balloon 900-13.

Referring to FIG. 18 to FIG. 18-7, the medical urinary catheter 902 is a double-lumen urinary catheter 902-1.

The distal end 900-11 of the catheter 900-1 of the double-lumen urinary catheter 902-1 is provided with a balloon 900-13; the catheter 900-1 is provided with two cavity channels 1-1, and each cavity channel 1-1 is correspondingly provided with one interface 900-2; one cavity channel 1-1 forms a urinary lumen 900-14, a distal end of the urinary lumen 900-14 is provided with the working opening 900-11-1, and the interface 900-2 provided at the proximal end forms a fluid drainage port 900-21; and the other cavity channel 1-1 forms a water injection lumen 900-15, a distal end of the water injection lumen 900-15 is connected to the balloon 900-13, and the interface 900-2 connected to the proximal end forms a water injection port 900-22.

To ensure a time requirement of clinical surgery, the balloon 900-13 usually needs to be disposed in the medical urinary catheter 902 used in surgery, and water is injected into the balloon 900-13 from the water injection port 900-22 through the water injection lumen 900-15, so that a water balloon may be formed, to ensure effective fastening of the medical urinary catheter 902 in the bladder, thereby preventing slippage.

Referring to FIG. 19 to FIG. 21-1, the medical urinary catheter 902 is a triple-cavity urinary catheter 902-2.

The distal end 900-11 of the catheter 900-1 of the triple-cavity urinary catheter 902-2 is provided with a balloon 900-13; the catheter 900-1 is provided with three cavity channels 1-1, and each cavity channel 1-1 is correspondingly provided with one interface 900-2; the first cavity channel 1-1 forms a urinary lumen 900-14, a distal end of the urinary lumen 900-14 is provided with the working opening 900-11-1, and the interface 900-2 provided at the proximal end forms a fluid drainage port 900-21; the second cavity channel 1-1 forms a water injection lumen 900-15, a distal end of the water injection lumen 900-15 is connected to the balloon 900-13, and the interface 900-2 connected to the proximal end forms a water injection port 900-22; and the third cavity channel 1-1 forms an infusion flushing lumen 900-16, a distal end of the infusion flushing lumen 900-16 is provided with a flushing port 900-16-1 and a stop valve 900-16-2, and the interface 900-2 connected to a proximal end of the infusion flushing lumen 900-16 forms a flushing drug injection port 900-23.

To facilitate flushing, especially drug flushing and disinfection, of the medical urinary catheter 902 during indwelling, the medical urinary catheter 902 may be disposed as the triple-cavity urinary catheter 902-2. The flushing drug injection port 900-23 is provided at the proximal end of the infusion flushing lumen 900-16. The flushing port 900-16-1 at the distal end is in communication with a body cavity. The stop valve 900-16-2 is provided at the flushing port 900-16-1. When drug is injected through the flushing drug injection port 900-23, under the action of a pressure, the stop valve 900-16-2 is opened, and the drug enters the body cavity. When the injection of the drug is stopped, under the action of a pressure in the body cavity, the stop valve 900-16-2 is automatically closed, to prevent body fluid from entering the infusion flushing lumen 900-16 through the flushing port 900-16-1.

Referring to FIG. 23, during clinical use, a medical person inserts the medical urinary catheter 902 into a bladder, and injects physiological saline into the balloon 900-13 through the water injection port 900-22, the balloon 900-13 expands, and the medical urinary catheter 902 is fastened in the bladder. The light source 1 is turned on, and the medical optical tracking system 500 tracks the catheter 900-1. When the medical urinary catheter 902 needs to be inserted into a ureter, the development string 41 is provided on the catheter 900-1. Under X-rays, the medical urinary catheter 902 is delivered to a position that requires tracking, then the light source 1 is turned on, and under the action of the medical optical tracking system 500, the ureter is identified by visible light, referring to FIG. 24.

The medical catheter with a tracking apparatus in this embodiment includes the medical optical tracking system 500, and therefore, after entering a human body, the medical catheter is tracked through visible light. In clinical surgery, when a cavity or tube being tracked is approached, it can be seen through tissue that the light emitted by the light source 1 is transmitted through the cavity or tube, and the illuminance of the light emitted by the light source 1 ensures that at a distance of about 0 mm to 30 mm from a cavity or tube wall, the cavity or tube being tracked is clearly visible. The light is stronger at a position closer to the cavity or tube, a prompt effect is clearer, so that the tracking of cavity or tube can be effectively performed in surgery, and a medical person is prompted to protect important blood vessels, to avoid accidental injury during surgery, making clinical use very convenient and safe.

### Embodiment 4: Solid tumor tracking apparatus of the present invention

Referring to FIG. 25, this embodiment discloses a visible light calibration technique and apparatus for a solid tumor. The solid tumor tracking apparatus in this embodiment includes the medical optical tracking system in Embodiment 1.

Referring to FIG. 25 to FIG. 29-1, the solid tumor tracking apparatus 910 includes an anti-displacement mechanism 910-1, a delivery mechanism 910-2, a development mechanism 4, and the medical optical tracking system 500; and the optical tracking carrier 2 of the medical optical tracking system 500 is disposed on the anti-displacement mechanism 910-1, to track the anti-displacement mechanism 910-1.

In this embodiment, the anti-displacement mechanism 910-1 is made of a metal material to form the development mechanism 4. Referring to FIG. 25-1 to FIG. 26, during actual application, to enhance a development effect, the development mechanism 4 may be separately disposed.

The anti-displacement mechanism 910-1 may be fastened at a solid tumor that needs to be calibrate d, to keep the solid tumor tracking apparatus 910 from displacing along with a movement of a human body, for example, respiratory movement of the lung, intestinal peristalsis, gastric peristalsis, or the like.

The anti-displacement mechanism 910-1 has a hook-shaped structure (referring to FIG. 26), and/or a spherical structure (referring to FIG. 30), and/or a horn-shaped structure, and/or a dumbbell structure (referring to FIG. 31 to FIG. 31-4), and/or a spiral spring structure (referring to FIG. 32 to FIG. 32-1), and/or a pin-shaped structure. The applicant only illustrates the manners of the anti-displacement mechanisms 910-1 with the foregoing several structures. During actual application, a person skilled in the art may design the anti-displacement mechanisms 910-1 with different structures or a combination of a plurality of structures as required, for example, a combination of a dumbbell and a spiral spring (referring to FIG. 33 and FIG. 33-1), which are not illustrated herein one by one by the applicant, but none of the anti-displacement mechanisms departs from the protection scope of this application. The coating 3 may be further disposed on an outer surface of the anti-displacement mechanism 910-1 as required, referring to FIG. 31-4.

Referring to FIG. 26, the anti-displacement mechanism 910-1 has a hook-shaped structure, and the anti-displacement mechanism 910-1 includes at least one positioning hook 910-11. Generally, the anti-displacement mechanism 910-1 includes two or three positioning hooks 910-11, to better prevent the anti-displacement mechanism 910-1 from displacing along with a movement of a human body, for example, respiratory movement of the lung, intestinal peristalsis, or the like.

Referring to FIG. 25-1 to FIG. 29, in this embodiment, the optical tracking carrier 2 is a light guide optical fiber 22, a proximal end of the light guide optical fiber 22 is connected to a light guide joint 26, a distal end of the light guide optical fiber 22 is connected and fastened to the anti-displacement mechanism 910-1, and light outlets 22-2 of the light guide optical fiber 22 are provided in the anti-displacement mechanism 910-1, to track the anti-displacement mechanism 910-1.

Referring to FIG. 26-1 to FIG. 26-3, the light guide optical fiber 22 has a non-smooth surface 22-1, the light outlets 22-2 are provided in a side surface of the light guide optical fiber 22, and the light guide optical fiber 22 performs overall tracking on the anti-displacement mechanism 910-1. The light guide optical fiber 22 can be completely lit, and therefore, after being wound on the anti-displacement mechanism 910-1, especially on the positioning hook 910-11, the light guide optical fiber 22 may perform overall tracking on the anti-displacement mechanism 910-1.

Referring to FIG. 26, the light guide optical fiber 22 is made of a flexible medical material, and is connected and fastened to the anti-displacement mechanism 910-1, and when the anti-displacement mechanism 910-1 deforms, the light guide optical fiber 22 deforms along with the anti-displacement mechanism 910-1. The light guide optical fiber 22 can be completely lit, and therefore, after being wound on the anti-displacement mechanism 910-1, especially on the positioning hook 910-11, the light guide optical fiber 22 may deform along with the anti-displacement mechanism 910-1. Especially, when the anti-displacement mechanism 910-1 is made of a shape-memory alloy, as the shape of the anti-displacement mechanism 910-1 changes, the shape of the light guide optical fiber 22 changes accordingly, to ensure the tracking effect of the anti-displacement mechanism 910-1.

Referring to FIG. 25, the delivery mechanism 910-2 includes a delivery sheath 910-21 and a pushing mechanism 910-22, the anti-displacement mechanism 910-1 is disposed in the delivery sheath 910-21, and the pushing mechanism 910-22 can push the anti-displacement mechanism 910-1 out of the delivery sheath 910-21, referring to FIG. 26.

The pushing mechanism 910-22 may be fastened to the anti-displacement mechanism 910-1, and after being pushed out of the delivery sheath 910-21, the anti-displacement mechanism 910-1 remains in vivo together with the delivery sheath. The pushing mechanism 910-22 may be detachably connected to the anti-displacement mechanism 910-1, and after being pushed out of the delivery sheath 910-21, the anti-displacement mechanism 910-1 is disconnected from the anti-displacement mechanism 910-1, and only the anti-displacement mechanism 910-1 is indwelt at a solid tumor, referring to FIG. 37 and FIG. 37-1.

Referring to FIG. 27 and FIG. 27-1, in addition, when the light source 1 of the medical optical tracking system 500 is a micro LED light source 11, the size of the light-emitting end 11-1 is usually controlled below 2 mm. For example, an LED lamp with a size of 0.2 mm to 0.5 mm is packaged. The light-emitting end 11-1 may be disposed in vivo.

The optical tracking carrier 2 is made of a transparent material, and light-emitting ends 11-1 of the LED light source 11 may be packaged on the optical tracking carrier 2, and disposed together with the optical tracking carrier 2 on the anti-displacement mechanism 910-1, to track the anti-displacement mechanism 910-1.

Light emitted by the light-emitting ends 11-1 is conducted by a light guide material of the optical tracking carrier 2 and then displayed for clinically discerning the position of a solid tumor.

The LED light source 11 includes the light-emitting ends 11-1, a circuit system 11-2, a drive board 11-3, and a power supply 11-4; the light-emitting ends 11-1 are connected to the drive board 11-3 and the power supply 11-4 by the circuit system 11-2, and under the control of the drive board 11-3, the power supply 11-4 supplies power to the light-emitting ends 11-1 through the circuit system 11-2, and the light-emitting ends 11-1 emit light; and the drive board 11-3 and the power supply 11-4 are disposed in vitro, and the light-emitting ends 11-1 are disposed in vivo, to track the anti-displacement mechanism 910-1.

The circuit system 11-2 is a flexible circuit board 11-21, and the light-emitting ends 11-1 of the LED light source 11 are dispersedly disposed on the flexible circuit board 11-21 and packaged in the optical tracking carrier 2, to form an LED lamp strip, or an LED lamp net, or an LED lamp bulb, disposed on the anti-displacement mechanism 910-1, to track the anti-displacement mechanism 910-1.

The LED light source 11 may be formed by packaging a single light-emitting end 11-1 to perform targeted tracking.

The circuit system 11-2 is a flexible circuit board 11-21, and the light-emitting ends 11-1 of the LED light source 11 are dispersedly disposed on the flexible circuit board 11-21 and packaged together in the optical tracking carrier 2, to form an LED lamp strip, or an LED lamp net, or an LED lamp bulb, disposed on the anti-displacement mechanism 910-1, to perform overall tracking on the anti-displacement mechanism 910-1.

The light-emitting ends 11-1 of the LED light source 11 may be directly disposed on the anti-displacement mechanism 910-1, and are wrapped with the optical tracking carrier 2 made of the transparent material outside, and light emitted by the light-emitting ends 11-1 tracks the anti-displacement mechanism 910-1.

The light-emitting ends 11-1 are dispersedly disposed on the anti-displacement mechanism 910-1, and are then wrapped with the optical tracking carrier 2 made of the transparent material outside. In this way, the light-emitting ends 11-1 may be completely joined to an outer contour of the anti-displacement mechanism 910-1, to perform tracking of any shape on the anti-displacement mechanism 910-1.

Furthermore, the light-emitting ends 11-1 are symmetrically disposed, and are wrapped with the optical tracking carrier 2 outside. Through the internal support of the light-emitting ends 11-1 and the circuit system 11-2 and the volume of the light-emitting ends 11-1, the anti-displacement mechanism 910-1 may be formed. The medical optical tracking system 500 can have both a tracking function and a positioning function of the anti-displacement mechanism 910-1, referring to FIG. 31 to FIG. 31-4.

The circuit system 11-2 is a flexible circuit board 11-21, the optical tracking carrier 2 is made of a soft transparent material, the light-emitting ends 11-1, the flexible circuit board 11-21, and the development mechanism 4 are disposed together in the optical tracking carrier 2, and are disposed on the anti-displacement mechanism 910-1, and when the anti-displacement mechanism 910-1 deforms, the light-emitting ends 11-1, the flexible circuit board 11-21, the development mechanism 4, and the optical tracking carrier 2 deform together along with the anti-displacement mechanism 910-1. Especially, when the anti-displacement mechanism 910-1 is made of a shape-memory alloy, as the shape of the anti-displacement mechanism 910-1 changes, the shape of the flexible circuit board 11-21 changes accordingly, to ensure the tracking effect of the anti-displacement mechanism 910-1.

Referring to FIG. 37, during clinical application, when a lung tumor (especially a pulmonary nodule), or a liver tumor, or the like needs to be calibrated, the anti-displacement mechanism 910-1 is disposed in the delivery sheath 910-21. In an X-rays state, the delivery sheath 910-21 is inserted at the position of the solid tumor, then the anti-displacement mechanism 910-1 is pushed out by using the pushing mechanism 910-22, and is fastened on the lung tumor (especially the pulmonary nodule), or the liver tumor, or another solid tumor, and then the delivery sheath 910-21 is removed. The anti-displacement mechanism 910-1 remains and is fastened on the solid tumor, and then the light guide optical fiber 22 is connected to the light source 1. Referring to FIG. 37-1, during surgery, the light source 1 is connected, the optical tracking carrier 2 tracks the anti-displacement mechanism 910-1, so that the position of the lung tumor (especially the pulmonary nodule), or the liver tumor, or another solid tumor can be intuitively observed with naked eyes under the guidance of light.

During clinical application, for example, a uterine fibroid needs to be calibrated. Under the guidance of B-mode ultrasound, the anti-displacement mechanism 910-1 may be delivered to the uterine fibroid and fastened on the uterine fibroid. During surgery, the light source 1 is turned on, and the optical tracking carrier 2 tracks the anti-displacement mechanism 910-1, so that under the guidance of light, the position of the uterine fibroid can be intuitively observed with naked eyes.

For the solid tumor tracking apparatus in this implementation, because the medical optical tracking system 500 is designed, after the system enters a human body, a solid tumor can be tracked through visible light. During surgery, the light source 1 is turned on, and the optical tracking carrier tracks the anti-displacement mechanism 910-1, so that under the guidance of light, the position of the solid tumor can be intuitively observed with naked eyes, making clinical surgery safer and more convenient.

It is to be noted that the structures disclosed and described in this specification may be replaced with other structures having the same effect, and the embodiments described in the present invention are not the only structures for implementing the present invention. Although preferred embodiments of the present invention have been described and described herein, a person skilled in the art clearly knows that these embodiments are merely examples. A person skilled in the art may make countless variations, improvements, and replacements without departing from the present invention. Therefore, the protection scope of the present invention should be defined according to the spirit and scope of the appended claims of the present invention.

## Claims

1. A medical optical tracking system, wherein the medical optical tracking system (500) comprises a light source (1) and an optical tracking carrier (2);
A. the optical tracking carrier (2) comprises a light guide material; and
B. light emitted by the light source (1) is conducted through the optical tracking carrier (2), and optical tracking is performed on the optical tracking carrier (2).

2. The medical optical tracking system according to claim 1, wherein the light source (1) is an LED light source (11), and/or a medical cold light source (12), and/or natural light.

3. The medical optical tracking system according to claim 1, wherein a color of the light emitted by the light source (1) may be set according to a background color or a transmission requirement.

4. The medical optical tracking system according to claim 1, wherein the light source (1) emits light in a flashing manner.

5. The medical optical tracking system according to claim 1, wherein an intensity of the light emitted by the light source (1) may be set.

6. The medical optical tracking system according to claim 2, wherein the LED light source (11) is disposed in vivo and/or in vitro.

7. The medical optical tracking system according to claim 1, wherein the light source (1) and the optical tracking carrier (2) are in non-contact connection or contact connection.

8. The medical optical tracking system according to claim 7, wherein the LED light source (11) and the optical tracking carrier (2) are in contact connection, and the LED light source (11) is disposed in the optical tracking carrier (2).

9. The medical optical tracking system according to claim 1, wherein the optical tracking carrier (2) is a light-retaining self-luminous tracking carrier (21).

10. The medical optical tracking system according to claim 9, wherein the light-retaining self-luminous tracking carrier (21) comprises a light-retaining self-luminous body (21-1) and a protective carrier (21-2).

11. The medical optical tracking system according to claim 10, wherein the protective carrier (21-2) is made of a transparent light guide material, and the light-retaining self-luminous body (21-1) is sealed in the protective carrier (21-2).

12. The medical optical tracking system according to claim 1, wherein the optical tracking carrier (2) is a light guide optical fiber (22).

13. The medical optical tracking system according to claim 1, wherein the optical tracking carrier (2) comprises at least one light guide optical fiber (22).

14. The medical optical tracking system according to claim 12, wherein both an end portion and/or a side surface of the light guide optical fiber (22) can emit light.

15. The medical optical tracking system according to claim 13, wherein the optical tracking carrier (2) is a combination of a plurality of light guide optical fibers (22).

16. The medical optical tracking system according to claim 12, wherein the light guide optical fiber (22) has a smooth surface.

17. The medical optical tracking system according to claim 12, wherein the light guide optical fiber (22) has a non-smooth surface (22-1).

18. The medical optical tracking system according to claim 17, wherein the non-smooth surface (22-1) is a non-smooth surface (22-11) that can form reflection and/or scattering.

19. The medical optical tracking system according to claim 12, wherein light outlets (22-2) are intermittently provided in the light guide optical fiber (22).

20. The optical fiber mechanism for medical optical tracking according to claim 19, wherein the light outlets (22-2) are non-axial light outlets (22-21), provided in a side surface of the light guide optical fiber (22) in a length direction of the light guide optical fiber (22).

21. The optical fiber mechanism for medical optical tracking according to claim 20, wherein the light outlets (22-2) may identify a length of the light guide optical fiber (22) through regular arrangement of the light outlets (22-2).

22. The optical fiber mechanism for medical optical tracking according to claim 21, wherein intensities of scattered light are different through regular arrangement of the light outlets (22-2) in distribution density to identify the length of the light guide optical fiber (22).

23. The medical optical tracking system according to claim 12, wherein the light guide optical fiber (22) is plaited in a mesh form, and light outlets (22-2) are scatteredly distributed at different positions.

24. The medical optical tracking system according to claim 1, wherein a surface of the medical optical tracking system (500) comprises a coating (3).

25. The optical fiber mechanism for medical optical tracking according to claim 24, wherein the coating (3) is an anticoagulant coating, and/or a hydrophilic coating, and/or a hydrophobic coating.

26. The medical optical tracking system according to claim 1, wherein the optical tracking carrier (2) comprises a delivery portion (23).

27. The medical optical tracking system according to claim 26, wherein the delivery portion (23) is movably disposed on the optical tracking carrier (2).

28. The medical optical tracking system according to claim 1, wherein the medical optical tracking system (500) further comprises a development mechanism (4).

29. The medical optical tracking system according to claim 28, wherein the development mechanism (4) is made of metal, and has a heat conduction function.

30. The medical optical tracking system according to claim 28, wherein the development mechanism (4) is a development string (41), and/or a development ring (42), and/or a development block (43).

31. The medical optical tracking system according to claim 28, wherein the development mechanism (4) performs development under X-rays, and/or in MRI, and/or in B-mode ultrasound.

32. The medical optical tracking system according to claim 1, wherein the medical optical tracking system (500) further comprises a protection sleeve (5).

33. The medical optical tracking system according to claim 32, wherein the protection sleeve (5) is made of a transparent material, and the optical tracking carrier (2) is disposed in the protection sleeve (5).

34. The medical optical tracking system according to claim 1, wherein the optical tracking carrier (2) comprises a channel (24) inside.

35. The medical optical tracking system according to claim 1, wherein the optical tracking carrier (2) is made of a flexible medical material, and is movable along a blood vessel, a ureter, an esophagus, a trachea, an oviduct, a vas deferens, or another cavity.

36. The medical optical tracking system according to claim 1, wherein the optical tracking carrier (2) comprises a shaping mechanism (25).

37. The medical optical tracking system according to claim 36, wherein the shaping mechanism (25) is a shape-memory shaping mechanism (25-1).

38. The medical optical tracking system according to claim 37, wherein the shape-memory shaping mechanism (25-1) is manufactured by plaiting a shape-memory metal wire, and/or is manufactured by carving a shape-memory metal tube or sheet.

39. A medical catheter with a tracking apparatus, wherein the medical catheter (900) with a tracking apparatus comprises the medical optical tracking system (500) according to claim 1.

40. The medical catheter with a tracking apparatus according to claim 39, wherein the medical catheter (900) with a tracking apparatus comprises a catheter (900-1), an interface (900-2), and the medical optical tracking system (500);
A. the catheter (900-1) is made of a soft elastic medical material, and is provided with a working channel (900-3) inside;
B. the medical catheter (900) with a tracking apparatus comprises at least one interface (900-2), and the interface (900-2) is disposed at a proximal end (900-12) of the catheter (900-1); and
C. the optical tracking carrier (2) of the medical optical tracking system (500), disposed on the catheter (900-1), and identifying a position of the catheter (900-1).

41. The medical catheter with a tracking apparatus according to claim 40, wherein the medical catheter (900) with a tracking apparatus comprises a development mechanism (4).

42. The medical catheter with a tracking apparatus according to claim 40, wherein the catheter (900-1) is made of a transparent material, and forms the optical tracking carrier (2), the light source of the medical optical tracking system (500) is an LED light source (11), and light-emitting ends (11-1) of the LED light source (11) are disposed in a tube wall of the catheter (900-1), and track the catheter (900-1).

43. The medical catheter with a tracking apparatus according to claim 42, wherein the light-emitting ends (11-1) of the LED light source (11) are disposed at a distal end of the catheter (900-1), and track the distal end of the catheter (900-1).

44. The medical catheter with a tracking apparatus according to claim 42, wherein the light-emitting ends (11-1) of the LED light source (11) are dispersedly disposed in the tube wall of the catheter (1), and form an LED lamp strip or an LED lamp net, to perform overall tracking on the catheter (900-1).

45. The medical catheter with a tracking apparatus according to claim 40, wherein the optical tracking carrier (2) of the medical optical tracking system (500) is a light guide optical fiber (22); and a proximal end of the light guide optical fiber (22) is connected to a light guide joint (26), and the light guide optical fiber (22) is disposed in a length direction of the catheter (900-1), to track the catheter (900-1).

46. The medical catheter with a tracking apparatus according to claim 45, wherein the light guide optical fiber (22) has a smooth surface, and light outlets (22-2) are provided at a distal end of the catheter (900-1), to track the distal end of the catheter (900-1).

47. The medical catheter with a tracking apparatus according to claim 45, wherein the light guide optical fiber (22) has a non-smooth surface (22-1), and light outlets (22-2) are provided in a side surface, to perform overall tracking on the catheter (900-1).

48. The medical catheter with a tracking apparatus according to claim 40, wherein the medical catheter (900) with a tracking apparatus is a medical stomach tube (901), or a medical urinary catheter (902), or a medical vas deferens, or a medical oviduct, or a medical trachea.

49. The medical catheter with a tracking apparatus according to claim 48, wherein the medical catheter (900) with a tracking apparatus is the medical stomach tube (901); the medical stomach tube (901) comprises the catheter (900-1), one interface (900-2), and the medical optical tracking system (500), and the distal end (900-11) of the catheter (900-1) is provided with a working opening (900-11-1); and the medical optical tracking system (500) tracks the catheter (900-1).

50. The medical catheter with a tracking apparatus according to claim 48, wherein the medical catheter (900) with a tracking apparatus is the medical urinary catheter (902); the medical urinary catheter (902) comprises the catheter (900-1), the interface (900-2), and the medical optical tracking system (500), and the distal end (900-11) of the catheter (900-1) is provided with a working opening (900-11-1); and the medical optical tracking system (500) tracks the catheter (900-1).

51. The medical catheter with a tracking apparatus according to claim 50, wherein the medical urinary catheter (902) is a double-lumen urinary catheter (902-1); the distal end (900-11) of the catheter (900-1) of the double-lumen urinary catheter (902-1) is provided with a balloon (900-13); the catheter (900-1) is provided with two cavity channels (1-1), and each cavity channel (1-1) is correspondingly provided with one interface (900-2); one cavity channel (1-1) forms a urinary lumen (900-14), a distal end of the urinary lumen (900-14) is provided with the working opening (900-11-1), and the interface (900-2) provided at the proximal end forms a fluid drainage port (900-21); and the other cavity channel (1-1) forms a water injection lumen (900-15), a distal end of the water injection lumen (900-15) is connected to the balloon (900-13), and the interface (900-2) connected to the proximal end forms a water injection port (900-22).

52. The medical catheter with a tracking apparatus according to claim 50, wherein the medical urinary catheter (902) is a triple-cavity urinary catheter (902-2); the distal end (900-11) of the catheter (900-1) of the triple-cavity urinary catheter (902-2) is provided with a balloon (900-13); the catheter (900-1) is provided with three cavity channels (1-1), and each cavity channel (1-1) is correspondingly provided with one interface (900-2); the first cavity channel (1-1) forms a urinary lumen (900-14), a distal end of the urinary lumen (900-14) is provided with the working opening (900-11-1), and the interface (900-2) provided at the proximal end forms a fluid drainage port (900-21); the second cavity channel (1-1) forms a water injection lumen (900-15), a distal end of the water injection lumen (900-15) is connected to the balloon (900-13), and the interface (900-2) connected to the proximal end forms a water injection port (900-22); and the third cavity channel (1-1) forms an infusion flushing lumen (900-16), a distal end of the infusion flushing lumen (900-16) is provided with a flushing port (900-16-1) and a stop valve (900-16-2), and the interface (900-2) connected to a proximal end of the infusion flushing lumen (900-16) forms a flushing drug injection port (900-23).

53. A solid tumor tracking apparatus, wherein the solid tumor tracking apparatus (910) comprises the medical optical tracking system (500) according to claim 1.

54. The solid tumor tracking apparatus according to claim 53, wherein the solid tumor tracking apparatus (910) comprises a development mechanism (4).

55. The solid tumor tracking apparatus according to claim 53, wherein the solid tumor tracking apparatus (910) comprises an anti-displacement mechanism (910-1) and the medical optical tracking system (500); and the optical tracking carrier (2) of the medical optical tracking system (500) is disposed on the anti-displacement mechanism (910-1), to track the anti-displacement mechanism (910-1).

56. The solid tumor tracking apparatus according to claim 55, wherein the anti-displacement mechanism (910-1) has a hook-shaped structure, and/or a horn-shaped structure, and/or a dumbbell structure, and/or a spiral spring structure, and/or a pin-shaped structure.

57. The solid tumor tracking apparatus according to claim 56, wherein the anti-displacement mechanism (910-1) has a hook-shaped structure, and the anti-displacement mechanism (910-1) comprises at least one positioning hook (910-11).

58. The solid tumor tracking apparatus according to claim 55, wherein the optical tracking carrier (2) is a light guide optical fiber (22), a proximal end of the light guide optical fiber (22) is connected to a light guide joint (26), a distal end of the light guide optical fiber (22) is connected and fastened to the anti-displacement mechanism (910-1), and light outlets (22-2) of the light guide optical fiber (22) are provided in the anti-displacement mechanism (910-1), to track the anti-displacement mechanism (910-1).

59. The solid tumor tracking apparatus according to claim 58, wherein the light guide optical fiber (22) has a non-smooth surface (22-1), the light outlets (22-2) are provided in a side surface of the light guide optical fiber (22), and the light guide optical fiber (22) performs overall tracking on the anti-displacement mechanism (910-1).

60. The solid tumor tracking apparatus according to claim 59, wherein the light guide optical fiber (22) is made of a flexible medical material, and is connected and fastened to the anti-displacement mechanism (910-1), and when the anti-displacement mechanism (910-1) deforms, the light guide optical fiber (22) deforms along with the anti-displacement mechanism (910-1).

61. The solid tumor tracking apparatus according to claim 53, wherein the solid tumor tracking apparatus (910) comprises an anti-displacement mechanism (910-1), a delivery mechanism (910-2), and the medical optical tracking system (500); and the optical tracking carrier (2) of the medical optical tracking system (500) is disposed on the anti-displacement mechanism (910-1), to track the anti-displacement mechanism (910-1).

62. The solid tumor tracking apparatus according to claim 61, wherein the delivery mechanism (910-2) comprises a delivery sheath (910-21) and a pushing mechanism (910-22), the anti-displacement mechanism (910-1) is disposed in the delivery sheath (910-21), and the pushing mechanism (910-22) can push the anti-displacement mechanism (910-1) out of the delivery sheath (910-21).

63. The solid tumor tracking apparatus according to claim 53, wherein the light source (1) of the medical optical tracking system (500) is an LED light source (11), the optical tracking carrier (2) is made of a transparent material, and light-emitting ends (11-1) of the LED light source (11) are disposed on the optical tracking carrier (2), and are disposed together with the optical tracking carrier (2) on the anti-displacement mechanism (910-1), to track the anti-displacement mechanism (910-1).

64. The solid tumor tracking apparatus according to claim 63, wherein the LED light source (11) comprises the light-emitting ends (11-1), a circuit system (11-2), a drive board (11-3), and a power supply (11-4); the light-emitting ends (11-1) are connected to the drive board (11-3) and the power supply (11-4) by the circuit system (11-2), and under the control of the drive board (11-3), the power supply (11-4) supplies power to the light-emitting ends (11-1) through the circuit system (11-2), and the light-emitting ends (11-1) emit light; and the drive board (11-3) and the power supply (11-4) are disposed in vitro, and the light-emitting ends (11-1) are disposed in vivo, to track the anti-displacement mechanism (910-1).

65. The solid tumor tracking apparatus according to claim 64, wherein the circuit system (11-2) is a flexible circuit board (11-21), and the light-emitting ends (11-1) of the LED light source (11) are dispersedly disposed on the flexible circuit board (11-21) and packaged in the optical tracking carrier (2), to form an LED lamp strip, or an LED lamp net, or an LED lamp bulb, disposed on the anti-displacement mechanism (910-1), to track the anti-displacement mechanism (910-1).

66. The solid tumor tracking apparatus according to claim 64, wherein the light-emitting ends (11-1) of the LED light source (11) are disposed on the anti-displacement mechanism (910-1), and are wrapped with the optical tracking carrier (2) made of the transparent material outside, and light emitted by the light-emitting ends (11-1) tracks the anti-displacement mechanism (910-1).

67. The solid tumor tracking apparatus according to claim 66, wherein the light-emitting ends (11-1) are dispersedly disposed on the anti-displacement mechanism (910-1), to perform overall tracking on the anti-displacement mechanism (910-1).

68. The solid tumor tracking apparatus according to claim 64, wherein the circuit system (11-2) is a flexible circuit board (11-21), the optical tracking carrier (2) is made of a soft transparent material, the light-emitting ends (11-1), the flexible circuit board (11-21), and a development mechanism (4) are disposed together in the optical tracking carrier (2), and are disposed on the anti-displacement mechanism (910-1), and when the anti-displacement mechanism (910-1) deforms, the light-emitting ends (11-1), the flexible circuit board (11-21), the development mechanism (4), and the optical tracking carrier (2) deform together along with the anti-displacement mechanism (910-1).

69. A blood vessel tracking apparatus, wherein the blood vessel tracking apparatus (920) comprises the medical optical tracking system (500) according to claim 1.

70. The blood vessel tracking apparatus according to claim 69, wherein the blood vessel tracking apparatus (920) comprises a development mechanism (4).

71. The blood vessel tracking apparatus according to claim 69, wherein the blood vessel tracking apparatus (920) comprises a coating (3), and the coating (3) is an anticoagulant coating.

72. The blood vessel tracking apparatus according to claim 69, wherein the blood vessel tracking apparatus (920) comprises a delivery end (920-1), a guide wire (920-2), a protection sleeve (5), and the medical optical tracking system (500); the medical optical tracking system (500) and the guide wire (920-2) are disposed in the protection sleeve (5), a coating (3) is provided outside the protection sleeve (5), and the coating (3) is an anticoagulant coating; the guide wire (920-2) is made of a metal material, and can perform development under X-rays, to form a development mechanism (4), and also has a heat conduction function; and the delivery end (920-1) can deliver the blood vessel tracking apparatus (920) to a position requiring blood vessel tracking.

73. The blood vessel tracking apparatus according to claim 72, wherein the guide wire (920-2) is made of a shape-memory alloy, and a distal end of the blood vessel tracking apparatus (920) is shaped, to form a shaping mechanism (25).

74. The blood vessel tracking apparatus according to claim 69, wherein the medical optical tracking system (500) is provided with a channel (24), and the channel (24) forms a guide wire operation hole (920-3).

75. The blood vessel tracking apparatus according to claim 74, wherein a side wall of the optical tracking carrier (2) is made of a transparent material, a light guide optical fiber (22) is provided in the side wall, the light guide optical fiber (22) has a non-smooth surface (22-1), light outlets (22-2) are provided in a side surface in a length direction, a coating (3) is provided on an outer surface of the optical tracking carrier (2), and the coating (3) is an anticoagulant coating; a proximal end of the light guide optical fiber (22) is connected to a light guide joint (26), and under the action of the light emitted by the light source (1), the light guide optical fiber (22) may overall emit light, to track a blood vessel.

76. The blood vessel tracking apparatus according to claim 69, wherein the light source (1) is a medical cold light source (12).

77. The blood vessel tracking apparatus according to claim 69, wherein a side wall of the optical tracking carrier (2) is made of a transparent material, the light source (1) is an LED light source (11), and light-emitting ends (11-1) of the LED light source (11) are dispersedly disposed in the side wall of the optical tracking carrier (2), to form an LED lamp strip or an LED lamp net, to track a blood vessel.
